# EUROPEAN PATENT APPLICATION

(11) **EP 4 621 486 A2**
(43) Date of publication of application: **24.09.2025**
(21) Application number: 25162618.0
(22) Date of filing: 10.03.2025
(51) Int. Cl.: G03F 7/004, G03F 7/075, G03F 7/09, C07C 211/00, C07D 235/18, C07D 251/04, C07D 487/18

(54) **POLYVALENT AMMONIUM SALT COMPOUND, COMPOSITION FOR FORMING SILICON-CONTAINING RESIST UNDERLAYER FILM, AND PATTERNING PROCESS**

(30) Priority: 12.03.2024 JP 2024038401
(71) Applicant: SHIN-ETSU CHEMICAL CO., LTD., Tokyo 1000005 (JP)
(72) Inventor: SAWAMURA, Takashi, Niigata (JP); MITSUI, Ryo, Niigata (JP)
(74) Representative: Röthinger, Rainer

(57) **Abstract**

The present invention is a polyvalent ammonium salt compound having two or more ammonium ions per molecule, the polyvalent ammonium salt compound being represented by the following general formula (A-1). This can provide: a composition for forming a silicon-containing resist underlayer film with which it is possible to form a silicon-containing resist underlayer film that has an appropriate etching rate and can improve LWR and CDU in an ultra-fine pattern in a multilayer resist method; a polyvalent ammonium salt compound to be contained in the composition; and a patterning process using the composition.

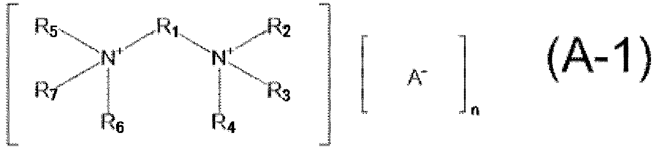

## Description

### TECHNICAL FIELD

The present invention relates to: a polyvalent ammonium salt compound; a composition for forming a silicon-containing resist underlayer film containing the compound; and a patterning process using the composition.

### BACKGROUND ART

Along with high integration and high processing speed of Large-Scale Integrated circuits (LSIs), miniaturization of pattern size is rapidly advancing. Along with the miniaturization, lithography technology has achieved a fine patterning by shortening wavelength of a light source and selecting an appropriate resist composition accordingly. The composition mainly used is a positive photoresist composition for monolayer. The monolayer positive photoresist composition not only allows a resist resin to have a skeleton having etching resistance against dry etching with chlorine- or fluorine-based gas plasma, but also provides a switching mechanism that makes an exposed part soluble, thereby dissolving the exposed part to form a pattern and processing a substrate to be processed by dry etching while using the remaining resist pattern as an etching mask.

However, when the pattern becomes finer, that is, the pattern width is reduced without changing the thickness of the photoresist film to be used, resolution performance of the photoresist film is lowered. In addition, pattern development of the photoresist film with a developer excessively increases a so-called aspect ratio of the pattern, resulting in pattern collapse. Therefore, the photoresist film has been thinned along with the miniaturization of the pattern.

On the other hand, a substrate to be processed has been generally processed by dry etching while using a photoresist film having a formed pattern as an etching mask. In practice, however, there is no dry etching method capable of providing an absolute etching selectivity between the photoresist film and the substrate to be processed. The resist film is thus also damaged and collapses during processing of the substrate, and the resist pattern cannot be accurately transferred to the substrate to be processed. Accordingly, higher dry etching resistance has been required in a photoresist composition along with the miniaturization of the pattern. However, on the other hand, a resin used for the photoresist composition needs to have low light absorption at exposure wavelength in order to improve the resolution. For this reason, the resin used for the photoresist composition has shifted to a novolak resin, polyhydroxystyrene, and a resin having an aliphatic polycyclic skeleton as the exposure light shifted from i-line to KrF and ArF, which have shorter wavelength. However, this shift has actually accelerated an etching rate under dry etching conditions for processing the substrate, and recent photoresist compositions having high resolution rather tend to have low etching resistance.

As a result, the substrate to be processed has to be dry-etched with a thinner photoresist film having lower etching resistance. Therefore, a demand for finding a composition used in this processing and the process therefor has become urgent.

A multilayer resist method is one of the solutions for the above problems. This method is as follows: a resist underlayer film having a different etching selectivity from that of a photoresist film (i.e., a resist upper layer film) is provided between the resist upper layer film and a substrate to be processed; a pattern is formed in the resist upper layer film; the pattern is transferred to the resist underlayer film by dry etching while using the resist upper layer film pattern as a dry etching mask; and the pattern is further transferred to the substrate to be processed by dry etching while using the resist underlayer film as a dry etching mask.

One of the multilayer resist methods is a three-layer resist method, which can be performed with a typical resist composition used in the monolayer resist method. For example, this three-layer resist method includes the following steps: an organic film containing a novolak resin or the like is formed on a substrate to be processed; a silicon-containing resist underlayer film is formed thereon; and a usual organic photoresist film is formed thereon as a resist upper layer film. Since the organic resist upper layer film ensures an excellent etching selectivity ratio relative to the silicon-containing resist underlayer film when dry etching is performed with fluorine-based gas plasma, the resist upper layer film pattern can be transferred to the silicon-containing resist underlayer film by dry etching with fluorine-based gas plasma. This method allows the pattern to be transferred to the silicon-containing resist underlayer film even when using a resist composition with which it is difficult to form a pattern having a sufficient film thickness for directly processing the substrate to be processed or a resist composition that has insufficient dry etching resistance for processing the substrate. Then, by subsequently performing transfer of the pattern by virtue of dry etching using oxygen gas plasma or hydrogen gas plasma, the pattern can be transferred to the organic film containing a novolak resin or the like, which has a sufficient dry etching resistance for processing the substrate. After this organic film pattern is formed, the remaining silicon-containing resist underlayer film is generally removed by dry etching with fluorine-based gas plasma or wet etching with, for example, an alkali- or fluorine-based etching solution to eliminate the defect-causing residue. If the etching rate is insufficient, this increases a possibility that the residue derived from the silicon-containing resist underlayer film stays and causes defects, or that longer etching treatment is required, bringing about problems such as damaging the substrate to be processed. As described above, for precise patterning and smooth removal, the silicon-containing resist underlayer film needs to be etched at an appropriate speed.

Meanwhile, the recent advents of ArF immersion lithography, EUV lithography, and so forth start to realize finer pattern formations, and in association with this, thinning of photoresist films has progressed further. Such thinning of photoresist films brings about degradation in edge roughness (LWR) and degradation in the critical dimension uniformity (CDU) of hole patterns in ultra-fine patterns, and therefore, improvement in LWR and CDU is a serious problem.

Accordingly, there have been proposed compositions, each containing a curing catalyst, for forming a silicon-containing resist underlayer film for ArF or EUV lithography (Patent Documents 1 and 2). The curing catalyst has a suitable structure for catalyzing a silanol condensation reaction and promoting the formation of siloxane bonds that serve as the main skeleton of a silicon-containing resist underlayer film. In addition, it is about to be revealed that the selection of a curing catalyst has various influences on properties of a silicon-containing resist underlayer film. Examples of the properties include acidity/basicity, hydrophilicity/hydrophobicity, hardness, film density, etching rate, etc. Such a curing catalyst has a structure similar to that of a sensitivity modifier in an upper layer resist. Therefore, there is a problem that, if a curing catalyst diffuses to an upper layer resist due to heating or exposure, there is a great influence on the pattern-formability of a photoresist. Since there is an influence particularly on LWR and CDU performance, there are demands for the development of a curing catalyst that is prevented from diffusing to an upper layer resist.

Meanwhile, for the improvement of LWR and CDU, a photosensitive upper layer resist material containing a compound having a cation and an anion in a molecule is known (Patent Document 3). However, in recent ultra-fine patterns, LWR and CDU of higher precision are desired, and improvement in the performance of resist underlayer films is also needed.

### CITATION LIST

### PATENT LITERATURE

Patent Document 1: JP2007-302873A
Patent Document 2: WO2013/161372A1
Patent Document 3: JP2011-016746A

### SUMMARY OF INVENTION

### TECHNICAL PROBLEM

The present invention has been made to solve the above-described problems. An object of the present invention is to provide: a composition for forming a silicon-containing resist underlayer film with which it is possible to form a silicon-containing resist underlayer film that has an appropriate etching rate and can improve LWR and CDU in an ultra-fine pattern in a multilayer resist method; a polyvalent ammonium salt compound to be contained in the composition; and a patterning process using the composition.

### SOLUTION TO PROBLEM

The present invention has been made to achieve the object, and provides a polyvalent ammonium salt compound having two or more ammonium ions per molecule, the polyvalent ammonium salt compound being represented by the following general formula (A-1), 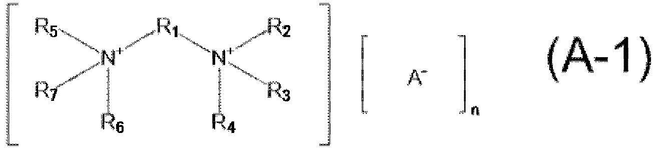 wherein R₁ represents a hydrocarbon group having 1 to 20 carbon atoms and optionally having a substituent or an aromatic group having 6 to 20 carbon atoms and optionally having a substituent, and optionally contains one or more nitrogen atoms; R₂, R₃, R₄, R₅, R₆, and R₇ each independently represent a hydrogen atom, a hydrocarbon group having 1 to 20 carbon atoms and optionally having a substituent, or an aromatic group having 6 to 20 carbon atoms and optionally having a substituent, and optionally contain one or more nitrogen atoms; any two or more selected from R₁, R₂, R₃, R₄, R₅, R₆, and R₇ are optionally bonded to each other to form a ring together with the nitrogen atom in the formula; A⁻ represents an organic or inorganic anion to be a counter ion of an ammonium cation, and excludes bistrifluoromethanesulfonylimide; and "n" represents an integer of 2 to 10.

When such a polyvalent ammonium salt compound is contained in a composition for forming a silicon-containing resist underlayer film, the polyvalent ammonium salt compound functions as a curing catalyst and can promote the thermosetting of the composition. In addition, the inventive compound is polyvalent, and therefore, exhibits higher reactivity than monovalent ammonium salt compounds when contained in a composition for forming a silicon-containing resist underlayer film. Thus, substance diffusion to a resist upper layer film can be suppressed, and it is possible to form a strong silicon-containing resist underlayer film having excellent LWR and CDU properties. Furthermore, a composition for forming a silicon-containing resist underlayer film containing the inventive polyvalent ammonium salt compound makes it possible to form a silicon-containing resist underlayer film having an excellent etching rate by virtue of the advantageous effects of silicon atoms.

In the present invention, the A⁻ is preferably formate, acetate, propionate, butyrate, hexanoate, benzoate, t-butylbenzoate, trichloroacetate, trifluoroacetate, 2-hydroxy-2,2-bis(trifluoromethyl)acetate, trimethylacetate, pentafluoropropionate, methanesulfonate, butanesulfonate, benzenesulfonate, toluenesulfonate, trifluoromethanesulfonate, methyl sulfate ion, chloride ion, bromide ion, iodide ion, fluoride ion, cyanide ion, nitrate ion, nitrite ion, or hydroxide ion.

When the A⁻ is an anion specified above, the advantageous effects of the present invention can be exhibited more sufficiently.

In the present invention, the polyvalent ammonium salt compound is preferably represented by the following general formula (B-1), the following general formula (B-2), the following general formula (B-3), or the following general formula (B-4), 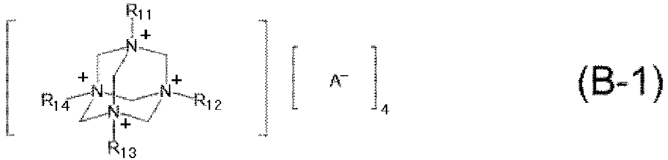 wherein R₁₁, R₁₂, R₁₃, and R₁₄ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 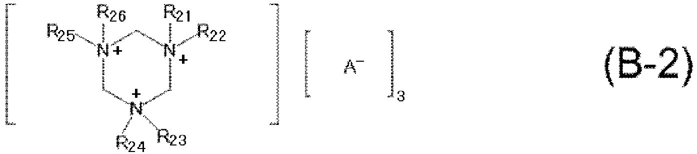 wherein R₂₁, R₂₂, R₂₃, R₂₄, R₂₅, and R₂₆ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 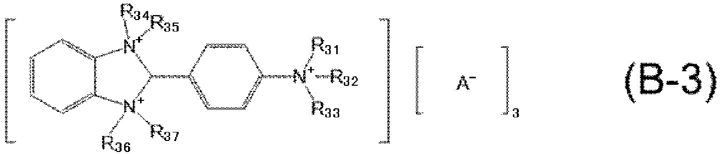 wherein R₃₁, R₃₂, R₃₃, R₃₄, R₃₅, R₃₆, and R₃₇ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 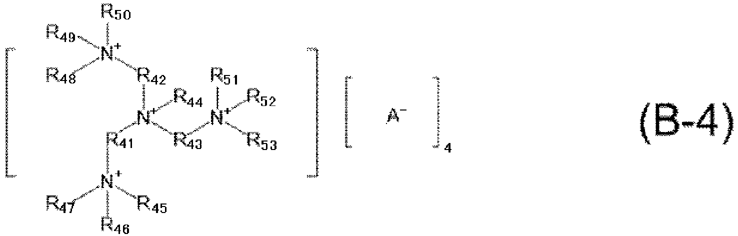 wherein R₄₁, R₄₂, and R₄₃ each independently represent a hydrocarbon group having 1 to 20 carbon atoms and optionally having a substituent or an aromatic group having 6 to 20 carbon atoms and optionally having a **substituent;** R₄₄, R₄₅, R₄₆, R₄₇, R₄₈, R₄₉, R₅₀, R₅₁, R₅₂, and R₅₃ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above.

Such a polyvalent ammonium salt compound can exhibit the advantageous effects of the present invention more effectively.

The present invention also provides a composition for forming a silicon-containing resist underlayer film, comprising: the above-described polyvalent ammonium salt compound; and a thermally crosslinkable polysiloxane.

Such a composition for forming a silicon-containing resist underlayer film makes it possible to form a silicon-containing resist underlayer film that has an appropriate etching rate and can improve LWR and CDU in an ultra-fine pattern in a multilayer resist method.

In the present invention, the thermally crosslinkable polysiloxane preferably contains any one or more of a repeating unit represented by the following general formula (Sx-1), a repeating unit represented by the following general formula (Sx-2), and a partial structure represented by the following general formula (Sx-3), 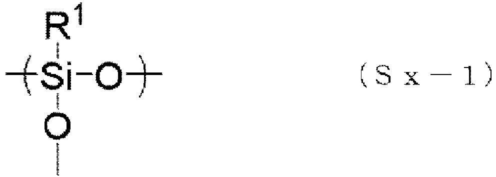 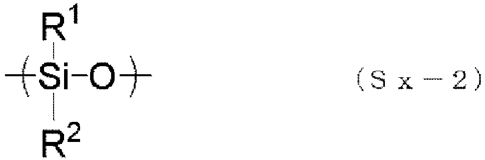 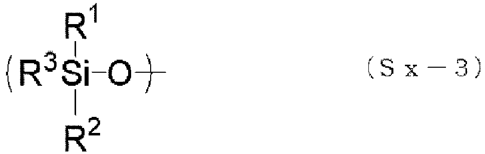 wherein R¹, R², and R³ are each identical to or different from each other and represent a monovalent organic group having 1 to 30 carbon atoms.

When the thermally crosslinkable polysiloxane contains a structure specified above, the advantageous effects of the present invention can be exhibited more sufficiently.

The inventive composition for forming a silicon-containing resist underlayer film preferably further comprises an acid generator.

By an acid generator being contained as necessary, it is possible to make fine adjustments to the pattern profile, exposure sensitivity, etc.

In this case, the acid generator is preferably a photo-acid generator, which generates an acid by an action of a high-energy beam.

Thus, it is possible to adjust appropriately the pattern profile, exposure sensitivity, etc. of a resist upper layer film while minimizing the degradation of other properties, and in addition, a photo-acid generator is also effective for reducing residues derived from a resist upper layer film in some cases.

The present invention also provides a patterning process for forming a pattern in a body to be processed, comprising the steps of:
forming an organic film on a body to be processed by using a coating-type organic film material;
forming a silicon-containing resist underlayer film on the organic film by using the above-described composition for forming a silicon-containing resist underlayer film;
forming a resist upper layer film on the silicon-containing resist underlayer film by using a resist upper layer film composition comprising a photoresist composition;
forming a circuit pattern in the resist upper layer film;
transferring the pattern to the silicon-containing resist underlayer film by etching while using the resist upper layer film having the formed circuit pattern as a mask;
transferring the pattern to the organic film by etching while using the silicon-containing resist underlayer film having the transferred pattern as a mask; and
transferring the pattern to the body to be processed by etching while using the organic film having the transferred pattern as a mask.

The present invention also provides a patterning process for forming a pattern in a body to be processed, comprising the steps of:
forming a hard mask on a body to be processed by a CVD method;
forming a silicon-containing resist underlayer film on the hard mask by using the above-described composition for forming a silicon-containing resist underlayer film;
forming a resist upper layer film on the silicon-containing resist underlayer film by using a resist upper layer film composition comprising a photoresist composition;
forming a circuit pattern in the resist upper layer film;
transferring the pattern to the silicon-containing resist underlayer film by etching while using the resist upper layer film having the formed circuit pattern as a mask;
transferring the pattern to the hard mask by dry etching while using the silicon-containing resist underlayer film having the transferred pattern as a mask; and
transferring the pattern to the body to be processed by dry etching while using the hard mask having the transferred pattern as a mask.

According to the above-described patterning processes, pattern collapse of the resist upper layer film is suppressed, excellent pattern transfer to the silicon-containing resist underlayer film by dry etching can be achieved, and the silicon-containing resist underlayer film that remains after the patterning has been completed can be removed easily, so that defects due to residues hardly occur. Therefore the patterning processes are particularly practical for the formation of a fine pattern.

In the present invention, it is preferable that the circuit pattern is formed in the resist upper layer film by a photolithography with a wavelength of 10 nm or more and 300 nm or less, a direct drawing by electron beam, a nanoimprinting, or a combination thereof.

When the circuit pattern is formed in the resist upper layer film by a method specified above, the advantageous effects of the present invention can be exhibited more sufficiently.

In the present invention, the body to be processed can be a semiconductor device substrate or the semiconductor device substrate coated with any of a metal film, a metal carbide film, a metal oxide film, a metal nitride film, a metal oxycarbide film, and a metal oxynitride film.

When a material specified above is used as the body to be processed, the advantageous effects of the present invention can be exhibited more sufficiently.

Furthermore, in the present invention, the metal is preferably silicon, gallium, titanium, tungsten, hafnium, zirconium, chromium, germanium, copper, silver, gold, indium, arsenic, palladium, tantalum, iridium, aluminum, iron, molybdenum, cobalt, or an alloy thereof.

When the metal constituting the body to be processed is a metal specified above, the advantageous effects of the present invention can be exhibited more sufficiently.

### ADVANTAGEOUS EFFECTS OF INVENTION

As described above, when the inventive polyvalent ammonium salt compound is contained in a composition for forming a silicon-containing resist underlayer film containing a thermally crosslinkable polysiloxane as a curing catalyst, it is not only possible to form an ultra-fine upper layer resist pattern having excellent LWR and CDU, excellent dry etching selectivity relative to the resist upper layer film and the organic film or the hard mask can be achieved, and therefore, a pattern for a semiconductor device can be formed in a substrate with a good yield. In addition, a formed silicon-containing resist underlayer film can achieve high etching selectivity relative to organic materials, and therefore, a formed upper layer resist pattern can be transferred successively to the silicon-containing resist underlayer film and an organic film or a hard mask by using a dry etching process. Furthermore, the formed silicon-containing resist underlayer film has a sufficient etching rate, and therefore, the silicon-containing resist underlayer film that remains after the patterning has been completed can be removed easily, so that defects due to residues hardly occur. Therefore, the compound is particularly useful for the formation of a fine pattern.

### DESCRIPTION OF EMBODIMENTS

Hereinafter, the present invention will be described in more detail. As stated above, there have been demands for the development of: a composition for forming a silicon-containing resist underlayer film with which it is possible to form a silicon-containing resist underlayer film that has an appropriate etching rate and can improve LWR and CDU in an ultra-fine pattern in a multilayer resist method; a polyvalent ammonium salt compound to be contained in the composition; and a patterning process using the composition.

To achieve the above-described object, the present inventors have earnestly studied and found out that, when a polyvalent ammonium salt compound having a particular structure is contained in a composition for forming a silicon-containing resist underlayer film, it is possible to give a silicon-containing resist underlayer film that can improve the LWR and CDU of an ultra-fine pattern and has an etching rate suitable for processing in a multilayer resist method. Thus, the present invention has been completed.

That is, the present invention is a polyvalent ammonium salt compound having two or more ammonium ions per molecule, the polyvalent ammonium salt compound being represented by the following general formula (A-1), 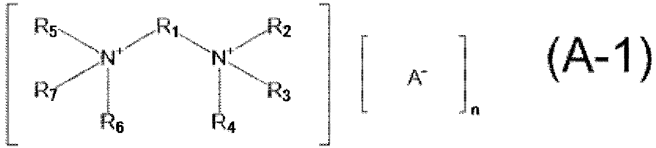 wherein R₁ represents a hydrocarbon group having 1 to 20 carbon atoms and optionally having a substituent or an aromatic group having 6 to 20 carbon atoms and optionally having a substituent, and optionally contains one or more nitrogen atoms; R₂, R₃, R₄, R₅, R₆, and R₇ each independently represent a hydrogen atom, a hydrocarbon group having 1 to 20 carbon atoms and optionally having a substituent, or an aromatic group having 6 to 20 carbon atoms and optionally having a substituent, and optionally contain one or more nitrogen atoms; any two or more selected from R₁, R₂, R₃, R₄, R₅, R₆, and R₇ are optionally bonded to each other to form a ring together with the nitrogen atom in the formula; A⁻ represents an organic or inorganic anion to be a counter ion of an ammonium cation, and excludes bistrifluoromethanesulfonylimide; and "n" represents an integer of 2 to 10.

Hereinafter, the present invention will be described in detail, but the present invention is not limited thereto.

### <Composition for Forming Silicon-Containing Resist Underlayer Film>

The inventive composition for forming a silicon-containing resist underlayer film contains: a polyvalent ammonium salt compound represented by the following general formula (A-1); and a thermally crosslinkable polysiloxane. Besides these components, the composition can also contain optional components.

In the following, each component contained in the inventive composition for forming a silicon-containing resist underlayer film will be described in detail.

### <Polyvalent Ammonium Salt Compound>

The inventive polyvalent ammonium salt compound is represented by the following general formula (A-1), and can be used as a curing catalyst. 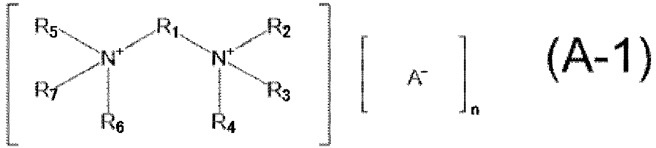 In the formula, R₁ represents a hydrocarbon group having 1 to 20 carbon atoms and optionally having a substituent or an aromatic group having 6 to 20 carbon atoms and optionally having a substituent, and optionally contains one or more nitrogen atoms; R₂, R₃, R₄, R₅, R₆, and R₇ each independently represent a hydrogen atom, a hydrocarbon group having 1 to 20 carbon atoms and optionally having a substituent, or an aromatic group having 6 to 20 carbon atoms and optionally having a substituent, and optionally contain one or more nitrogen atoms; any two or more selected from R₁, R₂, R₃, R₄, R₅, R₆, and R₇ are optionally bonded to each other to form a ring together with the nitrogen atom in the formula; A⁻ represents an organic or inorganic anion to be a counter ion of an ammonium cation, and excludes bistrifluoromethanesulfonylimide; and "n" represents an integer of 2 to 10.

### [Polyvalent Ammonium Salt Compound Represented By General Formula (A-1)]

The inventive polyvalent ammonium salt compound functions as a curing catalyst and promotes thermosetting when contained in a composition for forming a silicon-containing resist underlayer film. At the time of curing, the compound has higher reactivity than monovalent ammonium salt compounds, and therefore, is prevented from diffusing to an upper layer resist. Thus, the compound provides a strong composition for forming a silicon-containing resist underlayer film that contributes to the improvement of LWR and CDU. Furthermore, a formed silicon-containing resist underlayer film has an appropriate constitution and structure, and therefore, is assumed to have a sufficient etching rate for processing.

Note that, in the present description, films formed below the resist upper layer film are referred to as underlayer films, and include those called resist underlayer films and silicon-containing resist middle layer films.

In the general formula (A-1), R₁ represents a hydrocarbon group having 1 to 20 carbon atoms and optionally having a substituent or an aromatic group having 6 to 20 carbon atoms and optionally having a substituent, and optionally contains one or more nitrogen atoms; and R₂, R₃, R₄, R₅, R₆, and R₇ each independently represent a hydrogen atom, a hydrocarbon group having 1 to 20 carbon atoms and optionally having a substituent, or an aromatic group having 6 to 20 carbon atoms and optionally having a substituent, and optionally contain one or more nitrogen atoms. Specific examples of the aromatic group having 6 to 20 carbon atoms include a phenyl group, a naphthyl group, an anthracenyl group, a phenanthrenyl group, and a pyrenyl group. Examples of the substituent that is optionally contained include linear, branched, or cyclic, saturated or unsaturated hydrocarbon groups having 1 to 12 carbon atoms, aromatic hydrocarbon groups, heteroaromatic groups, alkoxy groups, a hydroxy group, an ester group, a carbonyl group, an amino group, a halogen group, a sulfide group, a carboxy group, a sulfo group, an amide group, an imide group, a cyano group, an aldehyde group, an imino group, a urea group, a carbamate group, a carbonate group, a nitro group, a sulfonyl group, and groups derived from a combination of these groups. Furthermore, any two or more selected from R₁, R₂, R₃, R₄, R₅, R₆, and R₇ are optionally bonded to each other to form a ring together with the nitrogen atom in the formula.

To achieve the advantageous effects of the present invention more effectively, the polyvalent ammonium salt compound can be a compound represented by the following general formula (B-1), the following general formula (B-2), the following general formula (B-3), or the following general formula (B-4).

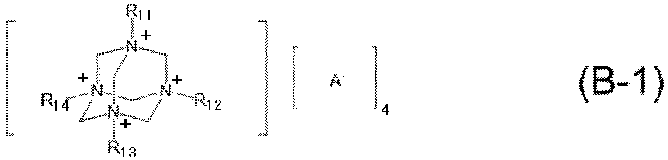

In the formula, R₁₁, R₁₂, R₁₃, and R₁₄ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above.

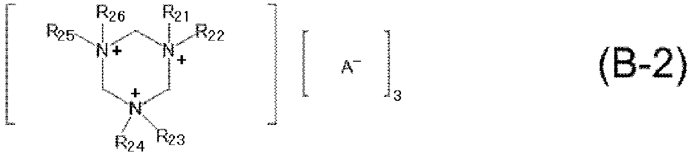

In the formula, R₂₁, R₂₂, R₂₃, R₂₄, R₂₅, and R₂₆ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above.

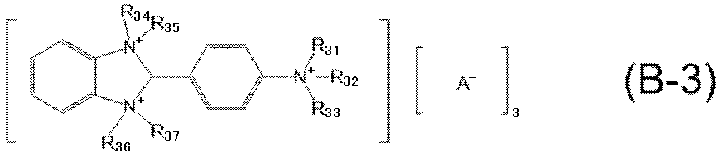

In the formula, R₃₁, R₃₂, R₃₃, R₃₄, R₃₅, R₃₆, and R₃₇ each independently represent a hydrogen atom, a hydrocarbon group having 1 to 20 carbon atoms and optionally having a substituent, or an aromatic group having 6 to 20 carbon atoms and optionally having a substituent, and are identical to or different from each other; and A⁻ is as defined above.

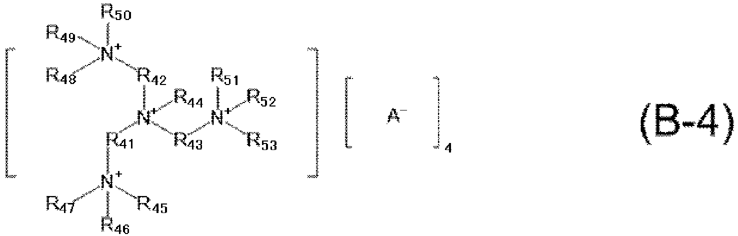

In the formula, R₄₁, R₄₂, and R₄₃ each independently represent a hydrocarbon group having 1 to 20 carbon atoms and optionally having a substituent or an aromatic group having 6 to 20 carbon atoms and optionally having a substituent; R₄₄, R₄₅, R₄₆, R₄₇, R₄₈, R₄₉, R₅₀, R₅₁, R₅₂, and R₅₃ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above.

In the general formula (B-1), R₁₁, R₁₂, R₁₃, and R₁₄ each independently represent a hydrogen atom, a hydrocarbon group having 1 to 20 carbon atoms and optionally having a substituent, or an aromatic group having 6 to 20 carbon atoms and optionally having a substituent. Specific examples of the aromatic group having 6 to 20 carbon atoms include a phenyl group, a naphthyl group, an anthracenyl group, a phenanthrenyl group, and a pyrenyl group. A⁻ represents an organic or inorganic anion to be a counter ion of an ammonium cation. Examples of the substituent that is optionally contained include linear, branched, or cyclic, saturated or unsaturated hydrocarbon groups having 1 to 12 carbon atoms, aromatic hydrocarbon groups, heteroaromatic groups, alkoxy groups, a hydroxy group, an ester group, a carbonyl group, an amino group, halogen groups, a sulfide group, a carboxy group, a sulfo group, an amide group, an imide group, a cyano group, an aldehyde group, an imino group, a urea group, a carbamate group, a carbonate group, a nitro group, a sulfonyl group, and groups derived from a combination of these groups. For achieving the advantageous effects of the present invention more effectively, a phenyl group, a naphthyl group, a furanyl group, a thiophenyl group, a pyrrolyl group, and a pyridinyl group are preferable, and a phenyl group and a naphthyl group are more preferable.

In the general formula (B-2), R₂₁, R₂₂, R₂₃, R₂₄, R₂₅, and R₂₆ each independently represent a hydrogen atom, a hydrocarbon group having 1 to 20 carbon atoms and optionally having a substituent, or an aromatic group having 6 to 20 carbon atoms and optionally having a substituent. Specific examples of the aromatic group having 6 to 20 carbon atoms include a phenyl group, a naphthyl group, an anthracenyl group, a phenanthrenyl group, and a pyrenyl group. A⁻ represents an organic or inorganic anion to be a counter ion of an ammonium cation. Examples of the substituent that is optionally contained include linear, branched, or cyclic, saturated or unsaturated hydrocarbon groups having 1 to 12 carbon atoms, aromatic hydrocarbon groups, heteroaromatic groups, alkoxy groups, a hydroxy group, an ester group, a carbonyl group, an amino group, halogen groups, a sulfide group, a carboxy group, a sulfo group, an amide group, an imide group, a cyano group, an aldehyde group, an imino group, a urea group, a carbamate group, a carbonate group, a nitro group, a sulfonyl group, and groups derived from a combination of these groups. For achieving the advantageous effects of the present invention more effectively, a phenyl group, a naphthyl group, a furanyl group, a thiophenyl group, a pyrrolyl group, and a pyridinyl group are preferable, and a phenyl group and a naphthyl group are more preferable.

In the general formula (B-3), R₃₁, R₃₂, R₃₃, R₃₄, R₃₅, R₃₆, and R₃₇ each independently represent a hydrogen atom, a hydrocarbon group having 1 to 20 carbon atoms and optionally having a substituent, or an aromatic group having 6 to 20 carbon atoms and optionally having a substituent. Specific examples of the aromatic group having 6 to 20 carbon atoms include a phenyl group, a naphthyl group, an anthracenyl group, a phenanthrenyl group, and a pyrenyl group. A⁻ represents an organic or inorganic anion to be a counter ion of an ammonium cation. Examples of the substituent that is optionally contained include linear, branched, or cyclic, saturated or unsaturated hydrocarbon groups having 1 to 12 carbon atoms, aromatic hydrocarbon groups, heteroaromatic groups, alkoxy groups, a hydroxy group, an ester group, a carbonyl group, an amino group, halogen groups, a sulfide group, a carboxy group, a sulfo group, an amide group, an imide group, a cyano group, an aldehyde group, an imino group, a urea group, a carbamate group, a carbonate group, a nitro group, a sulfonyl group, and groups derived from a combination of these groups. For achieving the advantageous effects of the present invention more effectively, a phenyl group, a naphthyl group, a furanyl group, a thiophenyl group, a pyrrolyl group, and a pyridinyl group are preferable, and a phenyl group and a naphthyl group are more preferable.

In the general formula (B-4), R₄₁, R₄₂, and R₄₃ each independently represent a hydrocarbon group having 1 to 20 carbon atoms and optionally having a substituent or an aromatic group having 6 to 20 carbon atoms and optionally having a substituent; and R₄₄, R₄₅, R₄₆, R₄₇, R₄₈, R₄₉, R₅₀, R₅₁, R₅₂, and R₅₃ each independently represent a hydrogen atom, a hydrocarbon group having 1 to 20 carbon atoms and optionally having a substituent, or an aromatic group having 6 to 20 carbon atoms and optionally having a substituent. Specific examples of the aromatic group having 6 to 20 carbon atoms include a phenyl group, a naphthyl group, an anthracenyl group, a phenanthrenyl group, and a pyrenyl group. A⁻ represents an organic or inorganic anion to be a counter ion of an ammonium cation. Examples of the substituent that is optionally contained include linear, branched, or cyclic, saturated or unsaturated hydrocarbon groups having 1 to 12 carbon atoms, aromatic hydrocarbon groups, heteroaromatic groups, alkoxy groups, a hydroxy group, an ester group, a carbonyl group, an amino group, halogen groups, a sulfide group, a carboxy group, a sulfo group, an amide group, an imide group, a cyano group, an aldehyde group, an imino group, a urea group, a carbamate group, a carbonate group, a nitro group, a sulfonyl group, and groups derived from a combination of these groups. For achieving the advantageous effects of the present invention more effectively, a phenyl group, a naphthyl group, a furanyl group, a thiophenyl group, a pyrrolyl group, and a pyridinyl group are preferable, and a phenyl group and a naphthyl group are more preferable.

In the general formulae (B-1), (B-2), (B-3), and (B-4), A⁻ represents an organic or inorganic anion to be a counter ion of an ammonium cation. Specific examples of the organic anion include formate, acetate, propionate, butyrate, hexanoate, benzoate, t-butylbenzoate, trichloroacetate, trifluoroacetate, 2-hydroxy-2,2-bis (trifluoromethyl)acetate, trimethylacetate, pentafluoropropionate, methanesulfonate, butanesulfonate, benzenesulfonate, toluenesulfonate, trifluoromethanesulfonate, and methyl sulfate ion. Specific examples of the inorganic anion include chloride ion, bromide ion, iodide ion, fluoride ion, cyanide ion, nitrate ion, nitrite ion, and hydroxide ion. Among these anions, chloride ion, iodide ion, fluoride ion, nitrate ion, nitrite ion, and hydroxide ion are preferable, and chloride ion and nitrate ion are more preferable.

In the present invention, one kind of the polyvalent ammonium salt compound represented by the general formula (A-1) may be used, or two or more kinds thereof may be used in mixture. The polyvalent ammonium salt compound represented by the general formula (A-1) is preferably contained in an amount of 0.01 to 50 parts by mass, more preferably 0.1 to 40 parts by mass, based on 100 parts by mass of the thermally crosslinkable polysiloxane (Sx) described later.

More specific examples of the polyvalent ammonium salt compound represented by the general formula (A-1) include the following compounds, but are not limited thereto.

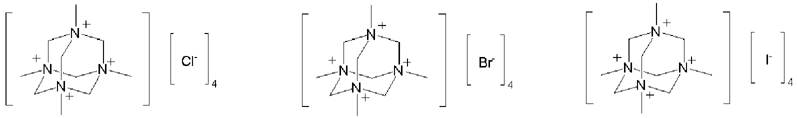

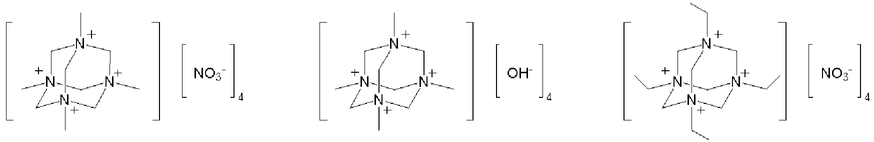

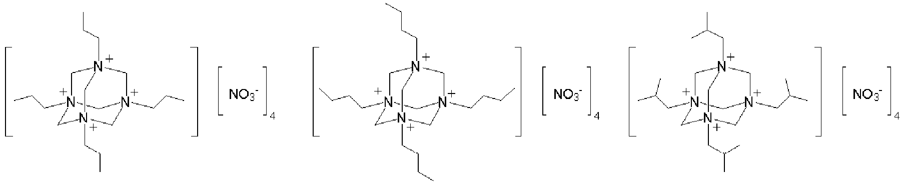

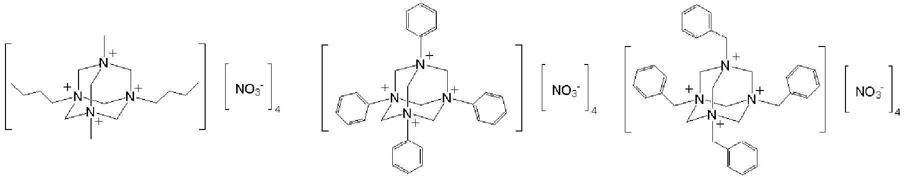

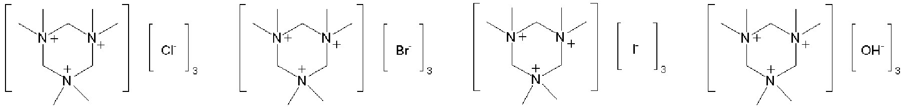

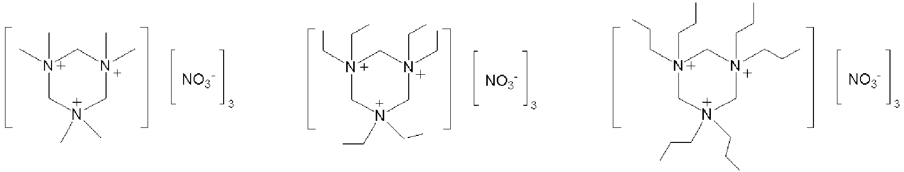

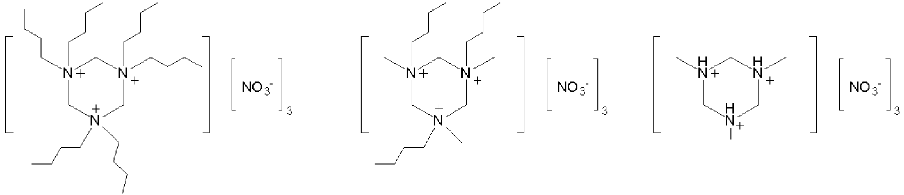

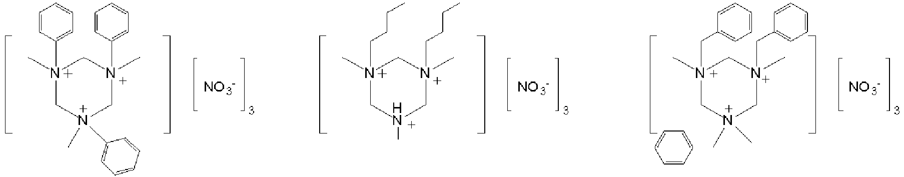

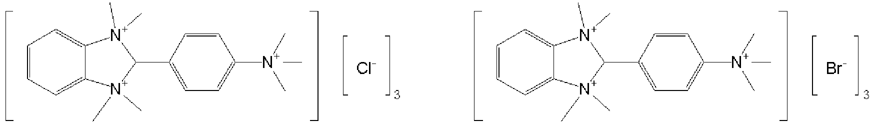

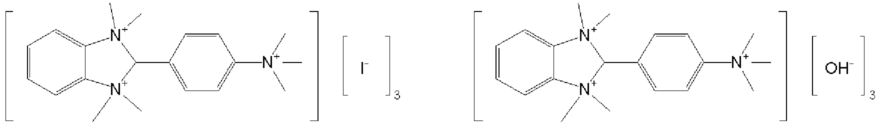

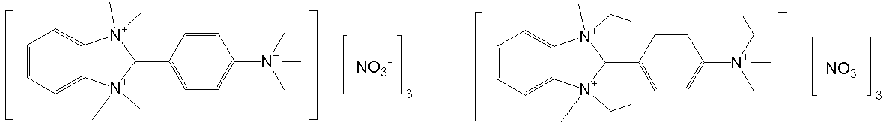

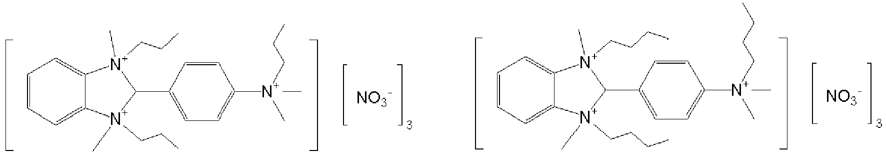

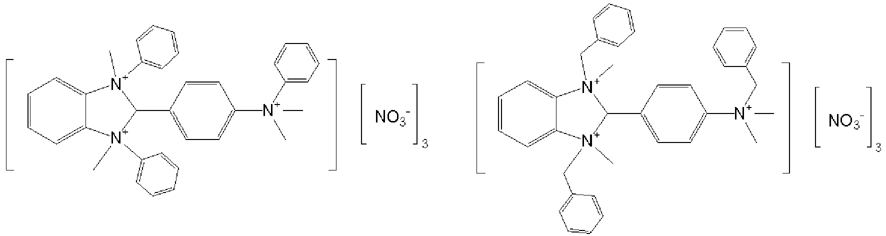

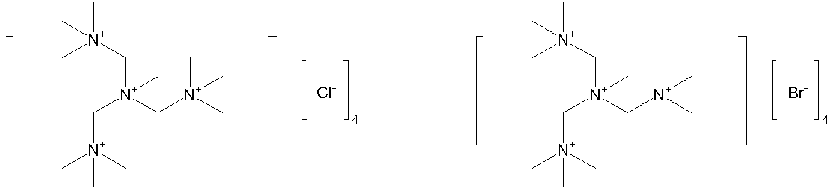

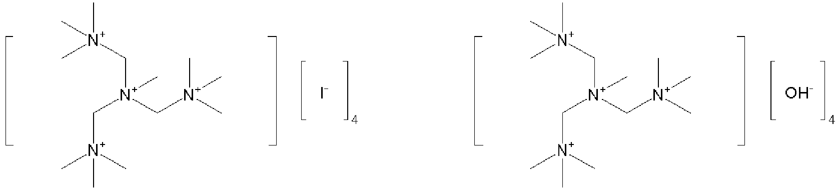

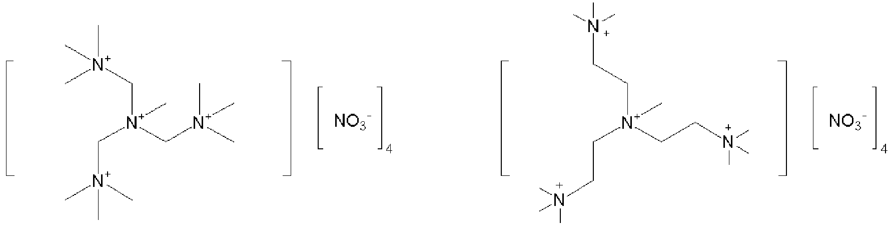

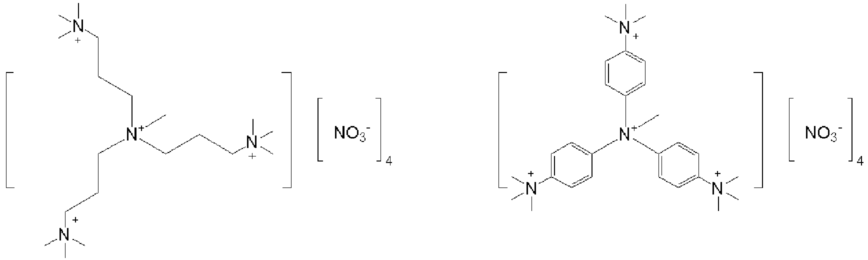

### [Thermally Crosslinkable Polysiloxane]

The inventive composition for forming a silicon-containing resist underlayer film contains a thermally crosslinkable polysiloxane in addition to one or more kinds of the polyvalent ammonium salt compound represented by the general formula (A-1).

The thermally crosslinkable polysiloxane (Sx) used in the present invention preferably contains any one or more of a repeating unit represented by the following general formula (Sx-1), a repeating unit represented by the following general formula (Sx-2), and a partial structure represented by the following general formula (Sx-3).

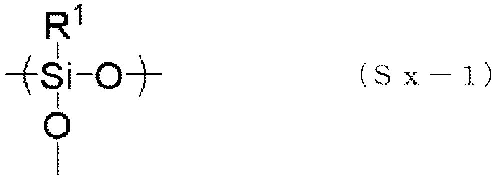

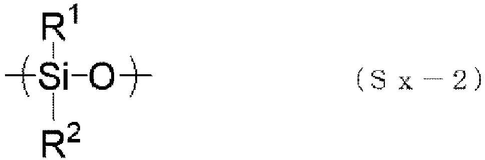

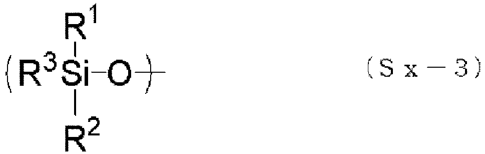

In the formulae, R¹, R², and R³ are each identical to or different from each other and represent a monovalent organic group having 1 to 30 carbon atoms.

The thermally crosslinkable polysiloxane (Sx) can be manufactured, for example, by the hydrolysis condensation of a hydrolysable monomer (Sm) described below.

Specific examples of the hydrolysable monomer (Sm) include tetramethoxysilane, tetraethoxysilane, tetrapropoxysilane, tetraisopropoxysilane, trimethoxysilane, triethoxysilane, tripropoxysilane, triisopropoxysilane, methyltrimethoxysilane, methyltriethoxysilane, methyltripropoxysilane, methyltriisopropoxysilane, ethyltrimethoxysilane, ethyltriethoxysilane, ethyltripropoxysilane, ethyltriisopropoxysilane, vinyltrimethoxysilane, vinyltriethoxysilane, vinyltripropoxysilane, vinyltriisopropoxysilane, propyltrimethoxysilane, propyltriethoxysilane, propyltripropoxysilane, propyltriisopropoxysilane, isopropyltrimethoxysilane, isopropyltriethoxysilane, isopropyltripropoxysilane, isopropyltriisopropoxysilane, butyltrimethoxysilane, butyltriethoxysilane, butyltripropoxysilane, butyltriisopropoxysilane, isobutyltrimethoxysilane, isobutyltriethoxysilane, isobutyltripropoxysilane, isobutyltriisopropoxysilane, sec-butyltrimethoxysilane, sec-butyltriethoxysilane, sec-butyltripropoxysilane, sec-butyltriisopropoxysilane, t-butyltrimethoxysilane, t-butyltriethoxysilane, t-butyltripropoxysilane, t-butyltriisopropoxysilane, allyltrimethoxysilane, allyltriethoxysilane, allyltripropoxysilane, allyltriisopropoxysilane, cyclopropyltrimethoxysilane, cyclopropyltriethoxysilane, cyclopropyltripropoxysilane, cyclopropyltriisopropoxysilane, cyclobutyltrimethoxysilane, cyclobutyltriethoxysilane, cyclobutyltripropoxysilane, cyclobutyltriisopropoxysilane, cyclopentyltrimethoxysilane, cyclopentyltriethoxysilane, cyclopentyltripropoxysilane, cyclopentyltriisopropoxysilane, cyclohexyltrimethoxysilane, cyclohexyltriethoxysilane, cyclohexyltripropoxysilane, cyclohexyltriisopropoxysilane, cyclohexenyltrimethoxysilane, cyclohexenyltriethoxysilane, cyclohexenyltripropoxysilane, cyclohexenyltriisopropoxysilane, cyclohexenylethyltrimethoxysilane, cyclohexenylethyltriethoxysilane, cyclohexenylethyltripropoxysilane, cyclohexenylethyltriisopropoxysilane, cyclooctyltrimethoxysilane, cyclooctyltriethoxysilane, cyclooctyltripropoxysilane, cyclooctyltriisopropoxysilane, cyclopentadienylpropyltrimethoxysilane, cyclopentadienylpropyltriethoxysilane, cyclopentadienylpropyltripropoxysilane, cyclopentadienylpropyltriisopropoxysilane, bicycloheptenyltrimethoxysilane, bicycloheptenyltriethoxysilane, bicycloheptenyltripropoxysilane, bicycloheptenyltriisopropoxysilane, bicycloheptyltrimethoxysilane, bicycloheptyltriethoxysilane, bicycloheptyltripropoxysilane, bicycloheptyltriisopropoxysilane, adamantyltrimethoxysilane, adamantyltriethoxysilane, adamantyltripropoxysilane, adamantyltriisopropoxysilane, phenyltrimethoxysilane, phenyltriethoxysilane, phenyltripropoxysilane, phenyltriisopropoxysilane, benzyltrimethoxysilane, benzyltriethoxysilane, benzyltripropoxysilane, benzyltriisopropoxysilane, anisyltrimethoxysilane, anisyltriethoxysilane, anisyltripropoxysilane, anisyltriisopropoxysilane, tolyltrimethoxysilane, tolyltriethoxysilane, tolyltripropoxysilane, tolyltriisopropoxysilane, phenethyltrimethoxysilane, phenethyltriethoxysilane, phenethyltripropoxysilane, phenethyltriisopropoxysilane, naphthyltrimethoxysilane, naphthyltriethoxysilane, naphthyltripropoxysilane, naphthyltriisopropoxysilane, dimethyldimethoxysilane, dimethyldiethoxysilane, methylethyldimethoxysilane, methylethyldiethoxysilane, dimethyldipropoxysilane, dimethyldiisopropoxysilane, diethyldimethoxysilane, diethyldiethoxysilane, diethyldipropoxysilane, diethyldiisopropoxysilane, dipropyldimethoxysilane, dipropyldiethoxysilane, dipropyldipropoxysilane, dipropyldiisopropoxysilane, diisopropyldimethoxysilane, diisopropyldiethoxysilane, diisopropyldipropoxysilane, diisopropyldiisopropoxysilane, dibutyldimethoxysilane, dibutyldiethoxysilane, dibutyldipropoxysilane, dibutyldiisopropoxysilane, di-sec-butyldimethoxysilane, di-sec-butyldiethoxysilane, di-sec-butyldipropoxysilane, di-sec-butyldiisopropoxysilane, di-t-butyldimethoxysilane, di-t-butyldiethoxysilane, di-t-butyldipropoxysilane, di-t-butyldiisopropoxysilane, dicyclopropyldimethoxysilane, dicyclopropyldiethoxysilane, dicyclopropyldipropoxysilane, dicyclopropyldiisopropoxysilane, dicyclobutyldimethoxysilane, dicyclobutyldiethoxysilane, dicyclobutyldipropoxysilane, dicyclobutyldiisopropoxysilane, dicyclopentyldimethoxysilane, dicyclopentyldiethoxysilane, dicyclopentyldipropoxysilane, dicyclopentyldiisopropoxysilane, dicyclohexyldimethoxysilane, dicyclohexyldiethoxysilane, dicyclohexyldipropoxysilane, dicyclohexyldiisopropoxysilane, dicyclohexenyldimethoxysilane, dicyclohexenyldiethoxysilane, dicyclohexenyldipropoxysilane, dicyclohexenyldiisopropoxysilane, dicyclohexenylethyldimethoxysilane, dicyclohexenylethyldiethoxysilane, dicyclohexenylethyldipropoxysilane, dicyclohexenylethyldiisopropoxysilane, dicyclooctyldimethoxysilane, dicyclooctyldiethoxysilane, dicyclooctyldipropoxysilane, dicyclooctyldiisopropoxysilane, dicyclopentadienylpropyldimethoxysilane, dicyclopentadienylpropyldiethoxysilane, dicyclopentadienylpropyldipropoxysilane, dicyclopentadienylpropyldiisopropoxysilane, bis(bicycloheptenyl)dimethoxysilane, bis(bicycloheptenyl)diethoxysilane, bis(bicycloheptenyl)dipropoxysilane, bis(bicycloheptenyl)diisopropoxysilane, bis(bicycloheptyl)dimethoxysilane, bis(bicycloheptyl)diethoxysilane, bis(bicycloheptyl)dipropoxysilane, bis(bicycloheptyl)diisopropoxysilane, diadamantyldimethoxysilane, diadamantyldiethoxysilane, diadamantyldipropoxysilane, diadamantyldiisopropoxysilane, diphenyldimethoxysilane, diphenyldiethoxysilane, methylphenyldimethoxysilane, methylphenyldiethoxysilane, diphenyldipropoxysilane, diphenyldiisopropoxysilane, trimethylmethoxysilane, trimethylethoxysilane, dimethylethylmethoxysilane, dimethylethylethoxysilane, dimethylphenylmethoxysilane, dimethylphenylethoxysilane, dimethylbenzylmethoxysilane, dimethylbenzylethoxysilane, dimethylphenethylmethoxysilane, dimethylphenethylethoxysilane, and the like.

Preferable examples of the compounds include tetramethoxysilane, tetraethoxysilane, methyltrimethoxysilane, methyltriethoxysilane, ethyltrimethoxysilane, ethyltriethoxysilane, vinyltrimethoxysilane, vinyltriethoxysilane, propyltrimethoxysilane, propyltriethoxysilane, isopropyltrimethoxysilane, isopropyltriethoxysilane, butyltrimethoxysilane, butyltriethoxysilane, isobutyltrimethoxysilane, isobutyltriethoxysilane, allyltrimethoxysilane, allyltriethoxysilane, cyclopentyltrimethoxysilane, cyclopentyltriethoxysilane, cyclohexyltrimethoxysilane, cyclohexyltriethoxysilane, cyclohexenyltrimethoxysilane, cyclohexenyltriethoxysilane, phenyltrimethoxysilane, phenyltriethoxysilane, benzyltrimethoxysilane, benzyltriethoxysilane, phenethyltrimethoxysilane, phenethyltriethoxysilane, dimethyldimethoxysilane, dimethyldiethoxysilane, diethyldimethoxysilane, diethyldiethoxysilane, methylethyldimethoxysilane, methylethyldiethoxysilane, dipropyldimethoxysilane, dibutyldimethoxysilane, methylphenyldimethoxysilane, methylphenyldiethoxysilane, trimethylmethoxysilane, dimethylethylmethoxysilane, dimethylphenylmethoxysilane, dimethylbenzylmethoxysilane, dimethylphenethylmethoxysilane, and the like.

Other examples of the organic groups represented by R¹, R², and R³ include organic groups having one or more carbon-oxygen single bonds or carbon-oxygen double bonds. Specific examples include organic groups having one or more groups selected from the group consisting of an ether bond, an ester bond, alkoxy groups, a hydroxy group, etc. Examples of the organic groups include ones shown by the following general formula (Sm-R).

(P-Q₁-(S₁)ᵥ₁-Q₂-)ᵤ-(T)ᵥ₂-Q₃-(S₂)ᵥ₃-Q₄- (Sm-R)

In the general formula (Sm-R), P represents a hydrogen atom, a cyclic ether group, a hydroxy group, an alkoxy group having 1 to 4 carbon atoms, an alkylcarbonyloxy group having 2 to 6 carbon atoms, or an alkylcarbonyl group having 2 to 6 carbon atoms; Q₁, Q₂, Q₃, and Q₄ each independently represent -C_{q}H_{(2q-p)}Pₚ-, where P is as defined above, "p" represents an integer of 0 to 3, and "q" represents an integer of 0 to 10, provided that q=0 means a single bond; "u" represents an integer of 0 to 3; S₁ and S₂ each independently represent -O-, -CO-, -OCO-, -COO-, or -OCOO-. "v1", "v2", and "v3" each independently represent 0 or 1. T represents a divalent atom other than carbon, or a divalent group including an alicyclic, aromatic, or heterocyclic ring.

As T, examples of the alicyclic, aromatic, or heterocyclic ring optionally containing a hetero atom such as an oxygen atom are shown below. In T, positions bonded to Q₂ and Q₃ are not particularly limited, and can be selected appropriately in consideration of reactivity dependent on steric factors, availability of commercial reagents used in the reaction, and so on.

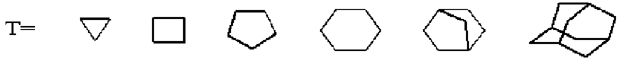

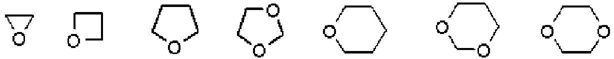

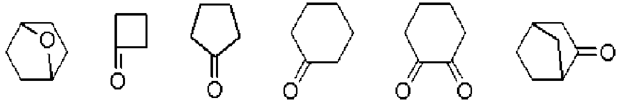

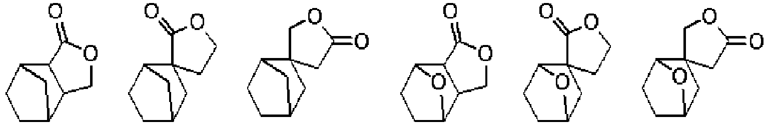

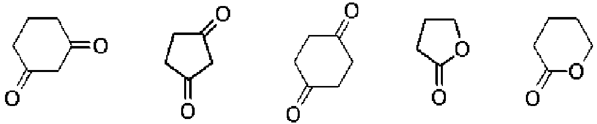

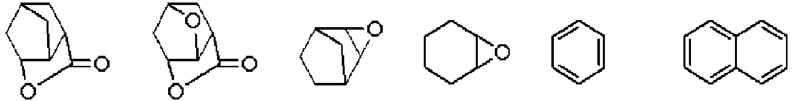

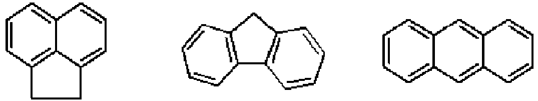

Preferable examples of the organic groups having one or more carbon-oxygen single bonds or carbon-oxygen double bonds in the general formula (Sm-R) include the following. Note that, in the following formulae, (Si) is depicted to show a bonding site to Si.

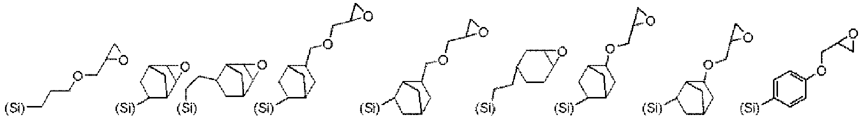

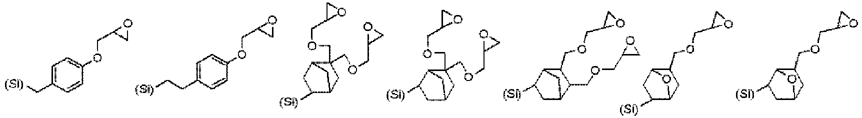

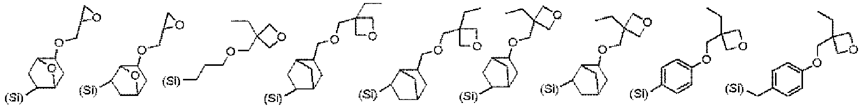

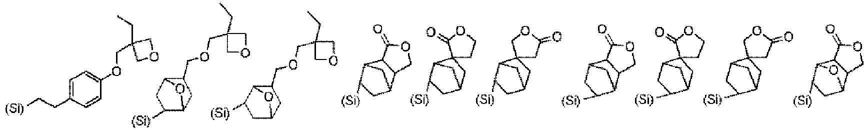

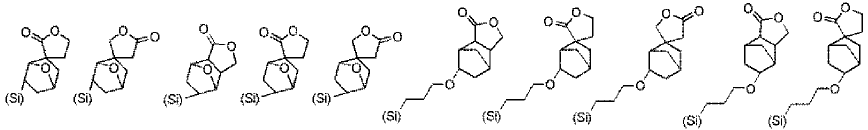

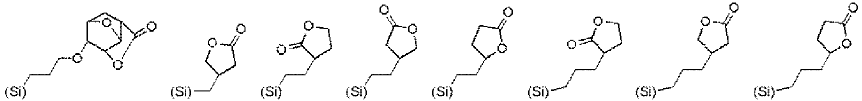

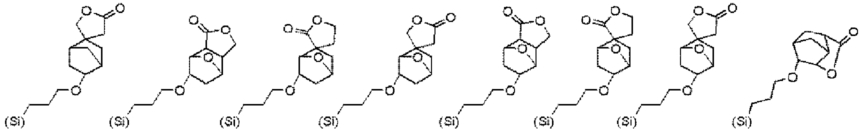

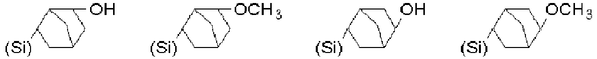

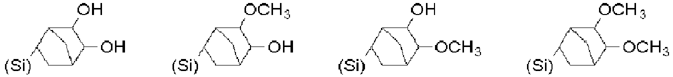

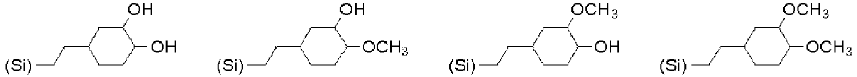

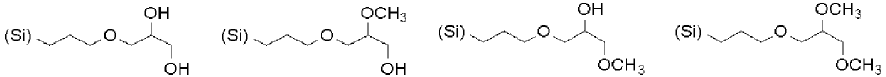

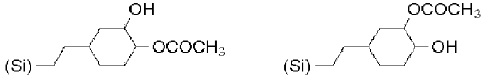

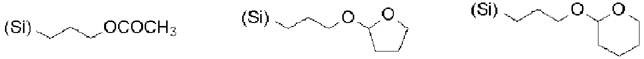

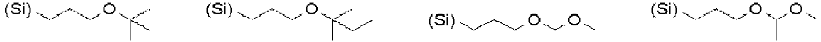

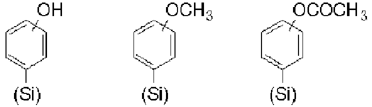

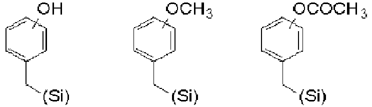

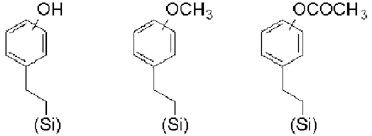

Furthermore, as examples of the organic groups of R¹, R², and R³, an organic group containing a silicon-silicon bond can also be used. Specific examples thereof include the following.

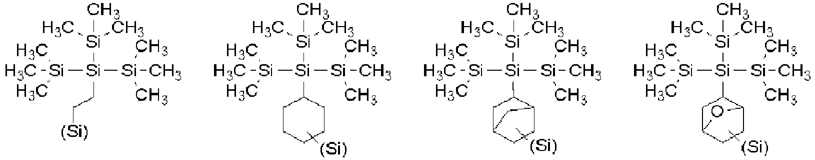

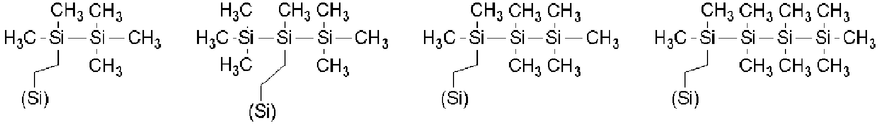

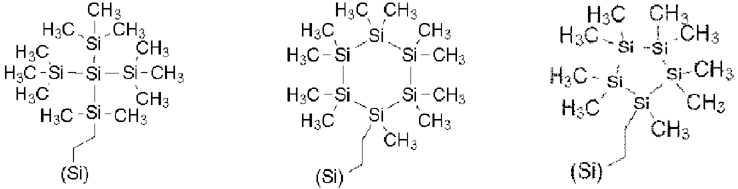

Further, as examples of the organic groups of R¹, R², and R³, organic groups having a protective group that is decomposed with an acid can also be used. Specific examples thereof include organic groups shown in paragraphs (0043) to (0048) of JP2013-167669A and organic groups obtained from silicon compounds shown in paragraph (0056) of JP2013-224279A.

Furthermore, as examples of the organic groups of R¹, R², and R³, an organic group having a fluorine atom can also be used. Specific examples thereof include organic groups obtained from silicon compounds shown in paragraphs (0059) to (0065) of JP2012-53253A.

To the hydrolysable monomer (Sm), 1, 2, or 3 chlorine atoms, bromine atoms, iodine atoms, acetoxy groups, methoxy groups, ethoxy groups, propoxy groups, butoxy groups, or the like are bonded as hydrolysable groups on the silicon atom shown as (Si) in the partial structures shown above.

### [Method for Synthesizing Thermally Crosslinkable Polysiloxane (Sx)]

### (Synthesis Method 1: Acid Catalyst)

The thermally crosslinkable polysiloxane (Sx) used in the present invention can be produced by the hydrolysis condensation of one kind of the hydrolysable monomer (Sm) or a mixture of two or more kinds thereof in the presence of an acid catalyst.

Examples of the acid catalyst used in this event include organic acids, such as formic acid, acetic acid, oxalic acid, maleic acid, methanesulfonic acid, benzenesulfonic acid, and toluenesulfonic acid; and hydrofluoric acid, hydrochloric acid, hydrobromic acid, sulfuric acid, nitric acid, perchloric acid, and phosphoric acid. The catalyst is preferably used in an amount of 1×10⁻⁶ to 10 mol, more preferably 1×10⁻⁵ to 5 mol, and further preferably 1×10⁻⁴ to 1 mol, relative to 1 mol of the monomer.

When the thermally crosslinkable polysiloxane (Sx) is obtained from these monomers by the hydrolysis condensation, water is preferably added in an amount of 0.01 to 100 mol, more preferably 0.05 to 50 mol, further preferably 0.1 to 30 mol, per mol of the hydrolysable substituent bonded to the monomer. When the amount is 100 mol or less, a reaction device can be made small and economical. When the amount is 0.01 mol or more, the reaction progresses sufficiently.

As the operation method, the monomer is added to an aqueous solution of a catalyst to initiate the hydrolysis condensation reaction. In this event, an organic solvent may be added to the aqueous solution of the catalyst, or the monomer may be diluted with an organic solvent, or both of these operations may be performed. The reaction temperature is preferably 0 to 100°C, more preferably 5 to 80°C. As a preferable method, when the monomer is added dropwise, the temperature is maintained at 5 to 80°C, and then the mixture is aged at 20 to 80°C.

The organic solvent which can be added to the aqueous solution of the catalyst or with which the monomer can be diluted is preferably methanol, ethanol, 1-propanol, 2-propanol, 1-butanol, 2-butanol, 2-methyl-1-propanol, ethylene glycol, propylene glycol, acetone, acetonitrile, tetrahydrofuran, toluene, hexane, ethyl acetate, methyl ethyl ketone, methyl isobutyl ketone, cyclohexanone, methyl amyl ketone, butanediol monomethyl ether, propylene glycol monomethyl ether, ethylene glycol monomethyl ether, butanediol monoethyl ether, propylene glycol monoethyl ether, ethylene glycol monoethyl ether, ethylene glycol monopropyl ether, propylene glycol dimethyl ether, diethylene glycol dimethyl ether, propylene glycol monomethyl ether acetate, propylene glycol monoethyl ether acetate, ethyl pyruvate, butyl acetate, methyl 3-methoxypropionate, ethyl 3-ethoxypropionate, tert-butyl acetate, t-butyl propionate, propylene glycol mono-t-butyl ether acetate, γ-butyrolactone, mixtures thereof, and the like.

Among these organic solvents, water-soluble solvents are preferable. Examples thereof include alcohols, such as methanol, ethanol, 1-propanol, and 2-propanol; polyhydric alcohols, such as ethylene glycol and propylene glycol; polyhydric alcohol condensate derivatives, such as butanediol monomethyl ether, propylene glycol monomethyl ether, ethylene glycol monomethyl ether, butanediol monoethyl ether, propylene glycol monoethyl ether, ethylene glycol monoethyl ether, butanediol monopropyl ether, propylene glycol monopropyl ether, and ethylene glycol monopropyl ether; acetone, acetonitrile, tetrahydrofuran, and the like. Among these, particularly preferable is one having a boiling point of 100°C or lower.

Note that the organic solvent is preferably used in an amount of 0 to 1,000 ml, particularly preferably 0 to 500 ml, relative to 1 mol of the monomer. When the organic solvent is used in a small amount, only a small reaction vessel is required, and this is economical.

Then, if necessary, neutralization reaction of the catalyst is carried out to obtain an aqueous solution of a reaction mixture. In this event, the amount of an alkaline substance usable for the neutralization is preferably 0.1 to 2 equivalents relative to the acid used as the catalyst. This alkaline substance may be any substance as long as it shows alkalinity in water.

Subsequently, by-products such as alcohol produced by the hydrolysis condensation reaction are preferably removed from the aqueous solution of the reaction mixture by a procedure such as removal under reduced pressure. In this event, the aqueous solution of the reaction mixture is heated at a temperature of preferably 0 to 100°C, more preferably 10 to 90°C, further preferably 15 to 80°C, although the temperature depends on the kinds of the added organic solvent, the alcohol produced in the reaction, and so forth. Additionally, in this event, the degree of vacuum is preferably atmospheric pressure or less, more preferably 80 kPa or less in absolute pressure, further preferably 50 kPa or less in absolute pressure, although the degree of vacuum varies depending on the kinds of the organic solvent, alcohol, etc. to be removed, as well as exhausting equipment, condensation equipment, and heating temperature. In this case, it is difficult to accurately know the amount of alcohol to be removed, but it is desirable to remove about 80 mass% or more of the produced alcohol, etc.

Next, the acid catalyst used in the hydrolysis condensation may be removed from the aqueous solution of the reaction mixture. As a method for removing the acid catalyst, the thermally crosslinkable polysiloxane solution is mixed with water, and the thermally crosslinkable polysiloxane is extracted with an organic solvent. Preferably, the organic solvent used in this event is capable of dissolving the thermally crosslinkable polysiloxane, and achieves two-layer separation when mixed with water. Examples include methanol, ethanol, 1-propanol, 2-propanol, 1-butanol, 2-butanol, 2-methyl-1-propanol, acetone, tetrahydrofuran, toluene, hexane, ethyl acetate, cyclohexanone, methyl amyl ketone, butanediol monomethyl ether, propylene glycol monomethyl ether, ethylene glycol monomethyl ether, butanediol monoethyl ether, propylene glycol monoethyl ether, ethylene glycol monoethyl ether, butanediol monopropyl ether, propylene glycol monopropyl ether, ethylene glycol monopropyl ether, propylene glycol dimethyl ether, diethylene glycol dimethyl ether, propylene glycol monomethyl ether acetate, propylene glycol monoethyl ether acetate, ethyl pyruvate, butyl acetate, methyl 3-methoxypropionate, ethyl 3-ethoxypropionate, t-butyl acetate, t-butyl propionate, propylene glycol mono-t-butyl ether acetate, γ-butyrolactone, methyl isobutyl ketone, cyclopentyl methyl ether, mixtures thereof, and the like.

Further, it is also possible to use a mixture of a water-soluble organic solvent and a slightly-water-soluble organic solvent. Preferable examples of the mixture include the mixture of methanol and ethyl acetate, the mixture of ethanol and ethyl acetate, the mixture of 1-propanol and ethyl acetate, the mixture of 2-propanol and ethyl acetate, the mixture of butanediol monomethyl ether and ethyl acetate mixture, the mixture of propylene glycol monomethyl ether and ethyl acetate, the mixture of ethylene glycol monomethyl ether and ethyl acetate, the mixture of butanediol monoethyl ether and ethyl acetate, the mixture of propylene glycol monoethyl ether and ethyl acetate, the mixture of ethylene glycol monoethyl ether and ethyl acetate, the mixture of butanediol monopropyl ether and ethyl acetate, the mixture of propylene glycol monopropyl ether and ethyl acetate, the mixture of ethylene glycol monopropyl ether and ethyl acetate, the mixture of methanol and methyl isobutyl ketone, the mixture of ethanol and methyl isobutyl ketone, the mixture of 1-propanol and methyl isobutyl ketone, the mixture of 2-propanol and methyl isobutyl ketone, the mixture of propylene glycol monomethyl ether and methyl isobutyl ketone, the mixture of ethylene glycol monomethyl ether and methyl isobutyl ketone, the mixture of propylene glycol monoethyl ether and methyl isobutyl ketone, the mixture of ethylene glycol monoethyl ether and methyl isobutyl ketone, the mixture of propylene glycol monopropyl ether and methyl isobutyl ketone, the mixture of ethylene glycol monopropyl ether and methyl isobutyl ketone, the mixture of methanol and cyclopentyl methyl ether, the mixture of ethanol and cyclopentyl methyl ether, the mixture of 1-propanol and cyclopentyl methyl ether, the mixture of 2-propanol and cyclopentyl methyl ether, the mixture of propylene glycol monomethyl ether and cyclopentyl methyl ether, the mixture of ethylene glycol monomethyl ether and cyclopentyl methyl ether, the mixture of propylene glycol monoethyl ether and cyclopentyl methyl ether, the mixture of ethylene glycol monoethyl ether and cyclopentyl methyl ether, the mixture of propylene glycol monopropyl ether and cyclopentyl methyl ether, the mixture of ethylene glycol monopropyl ether and cyclopentyl methyl ether, the mixture of methanol and propylene glycol methyl ether acetate, the mixture of ethanol and propylene glycol methyl ether acetate, the mixture of 1-propanol and propylene glycol methyl ether acetate, the mixture of 2-propanol and propylene glycol methyl ether acetate, the mixture of propylene glycol monomethyl ether and propylene glycol methyl ether acetate, the mixture of ethylene glycol monomethyl ether and propylene glycol methyl ether acetate, the mixture of propylene glycol monoethyl ether and propylene glycol methyl ether acetate, the mixture of ethylene glycol monoethyl ether and propylene glycol methyl ether acetate, the mixture of propylene glycol monopropyl ether and propylene glycol methyl ether acetate, the mixture of ethylene glycol monopropyl ether and propylene glycol methyl ether acetate, and the like. However, the combination is not limited thereto.

Although the mixing ratio of the water-soluble organic solvent and the slightly-water-soluble organic solvent is appropriately selected, the amount of the water-soluble organic solvent is preferably 0.1 to 1,000 parts by mass, more preferably 1 to 500 parts by mass, further preferably 2 to 100 parts by mass, based on 100 parts by mass of the slightly-water-soluble organic solvent.

Subsequently, the thermally crosslinkable polysiloxane solution may be washed with neutral water. As the water, what is commonly called deionized water or ultrapure water may be used. The amount of the water is preferably 0.01 to 100 L, more preferably 0.05 to 50 L, and further preferably 0.1 to 5 L, relative to 1 L of the thermally crosslinkable polysiloxane solution. This washing procedure may be performed by putting both the thermally crosslinkable polysiloxane solution and neutral water into the same container, followed by stirring and then leaving to stand to separate the aqueous layer. The washing may be performed once or more, preferably once to approximately five times, since washing ten times or more does not always produce the full washing effects thereof.

Other methods for removing the acid catalyst include a method using an ion-exchange resin, and a method in which the acid catalyst is removed after neutralization with an epoxy compound such as ethylene oxide and propylene oxide. These methods can be appropriately selected in accordance with the acid catalyst used in the reaction.

In this water-washing operation, part of the thermally crosslinkable polysiloxane escapes into the aqueous layer, so that substantially the same effect as fractionation operation is obtained in some cases. Hence, the number of water-washing operations and the amount of washing water may be appropriately selected in view of the catalyst removal effect and the fractionation effect.

To either of a solution of the thermally crosslinkable polysiloxane with the acid catalyst still remaining or a solution of the thermally crosslinkable polysiloxane with the acid catalyst having been removed, a final solvent may be added for solvent exchange under reduced pressure to obtain a desired thermally crosslinkable polysiloxane solution. In this event, the temperature during the solvent exchange is preferably 0 to 100°C, more preferably 10 to 90°C, further preferably 15 to 80°C, depending on the kinds of the reaction solvent and the extraction solvent to be removed. Moreover, the degree of vacuum in this event is preferably atmospheric pressure or less, more preferably 80 kPa or less in absolute pressure, further preferably 50 kPa or less in absolute pressure, although the degree of vacuum varies depending on the kinds of the extraction solvent to be removed, exhausting equipment, condensation equipment, and heating temperature.

In this event, the thermally crosslinkable polysiloxane may become unstable due to the solvent exchange. This occurs due to incompatibility of the thermally crosslinkable polysiloxane with the final solvent. Thus, in order to prevent this phenomenon, a monohydric, dihydric, or polyhydric alcohol having cyclic ether as a substituent as shown in paragraphs (0181) and (0182) of JP2009-126940A may be added as a stabilizer. The alcohol is preferably added in an amount of 0 to 25 parts by mass, more preferably 0 to 15 parts by mass, further preferably 0 to 5 parts by mass, based on 100 parts by mass of the thermally crosslinkable polysiloxane in the solution before the solvent exchange. When the alcohol is added, the amount is preferably 0.5 parts by mass or more. If necessary, the monohydric, dihydric, or polyhydric alcohol having cyclic ether as a substituent may be added to the solution before the solvent exchange, and then the solvent exchange operation may be performed.

It is preferable to maintain the thermally crosslinkable polysiloxane in a solution state with a proper concentration. The concentration in this state is preferably 0.1 to 20 mass%. When the concentration is as described, the condensation reaction does not progress further, and therefore, the polysiloxane does not become no longer soluble in an organic solvent. Moreover, the amount of the solvent is small, and therefore, is economical and preferable.

The final solvent added to the thermally crosslinkable polysiloxane solution is preferably an alcohol-based solvent. Particularly preferable examples include monoalkyl ether derivatives of ethylene glycol, diethylene glycol, triethylene glycol, propylene glycol, dipropylene glycol, butanediol, or the like. Specifically, preferable examples include butanediol monomethyl ether, propylene glycol monomethyl ether, ethylene glycol monomethyl ether, butanediol monoethyl ether, propylene glycol monoethyl ether, ethylene glycol monoethyl ether, butanediol monopropyl ether, propylene glycol monopropyl ether, ethylene glycol monopropyl ether, diacetone alcohol, and the like.

When these solvents are used as the main component, a non-alcohol-based solvent can also be added as an adjuvant solvent. Examples of the adjuvant solvent include acetone, tetrahydrofuran, toluene, hexane, ethyl acetate, cyclohexanone, methyl amyl ketone, propylene glycol dimethyl ether, diethylene glycol dimethyl ether, propylene glycol monomethyl ether acetate, propylene glycol monoethyl ether acetate, ethyl pyruvate, butyl acetate, methyl 3-methoxypropionate, ethyl 3-ethoxypropionate, t-butyl acetate, t-butyl propionate, propylene glycol mono-t-butyl ether acetate, γ-butyrolactone, methyl isobutyl ketone, cyclopentyl methyl ether, and the like.

As an alternative reaction operation using an acid catalyst, water or a water-containing organic solvent may be added to the monomer or an organic solution of the monomer to initiate the hydrolysis reaction. In this event, the catalyst may be added to the monomer or the organic solution of the monomer, or may be added to the water or the water-containing organic solvent. The reaction temperature is preferably 0 to 100°C, more preferably 10 to 80°C. As a preferable method, when the water is added dropwise, the mixture is heated to 10 to 50°C, and then further heated to 20 to 80°C for aging.

When the organic solvent is used, a water-soluble solvent is preferable. Examples thereof include methanol, ethanol, 1-propanol, 2-propanol, 1-butanol, 2-butanol, 2-methyl-1-propanol, acetone, tetrahydrofuran, and acetonitrile; polyhydric alcohol condensate derivatives, such as butanediol monomethyl ether, propylene glycol monomethyl ether, ethylene glycol monomethyl ether, butanediol monoethyl ether, propylene glycol monoethyl ether, ethylene glycol monoethyl ether, butanediol monopropyl ether, propylene glycol monopropyl ether, ethylene glycol monopropyl ether, propylene glycol dimethyl ether, diethylene glycol dimethyl ether, propylene glycol monomethyl ether acetate, propylene glycol monoethyl ether acetate, and propylene glycol monopropyl ether acetate; mixtures thereof, and the like.

The organic solvent is preferably used in an amount of 0 to 1,000 ml, particularly preferably 0 to 500 ml, relative to 1 mol of the monomer. When the organic solvent is used in a small amount, only a small reaction vessel is required, and this is economical. The obtained aqueous solution of the reaction mixture may be subjected to post-treatment by the same procedure as described above to obtain a thermally crosslinkable polysiloxane.

### (Synthesis Method 2: Alkali Catalyst)

Alternatively, the thermally crosslinkable polysiloxane (Sx) can also be produced by the hydrolysis condensation of one kind of the hydrolysable monomer (Sm) or a mixture of two or more kinds thereof in the presence of an alkali catalyst. Examples of the alkali catalyst used in this event include methylamine, ethylamine, propylamine, butylamine, ethylenediamine, hexamethylenediamine, dimethylamine, diethylamine, ethylmethylamine, trimethylamine, triethylamine, tripropylamine, tributylamine, cyclohexylamine, dicyclohexylamine, monoethanolamine, diethanolamine, dimethyl monoethanolamine, monomethyl diethanolamine, triethanolamine, diazabicyclooctane, diazabicyclocyclononene, diazabicycloundecene, hexamethylenetetramine, aniline, N,N-dimethylaniline, pyridine, N,N-dimethylaminopyridine, pyrrole, piperazine, pyrrolidine, piperidine, picoline, tetramethylammonium hydroxide, choline hydroxide, tetrapropylammonium hydroxide, tetrabutylammonium hydroxide, ammonia, lithium hydroxide, sodium hydroxide, potassium hydroxide, barium hydroxide, calcium hydroxide, and the like. The catalyst is preferably used in an amount of 1×10⁻⁶ mol to 10 mol, more preferably 1×10⁻⁵ mol to 5 mol, and further preferably 1×10⁻⁴ mol to 1 mol, relative to 1 mol of the monomer.

When the thermally crosslinkable polysiloxane is obtained from the monomer by the hydrolysis condensation, water is preferably added in an amount of 0.1 to 50 mol per mol of the hydrolysable substituent bonded to the monomer. When the amount is 50 mol or less, a device used for the reaction can be made small and economical. When the amount is 0.1 mol or more, the reaction progresses sufficiently.

As the operation method, the monomer is added to an aqueous solution of a catalyst to initiate the hydrolysis condensation reaction. In this event, an organic solvent may be added to the aqueous solution of the catalyst, or the monomer may be diluted with an organic solvent, or both of these operations may be performed. The reaction temperature is preferably 0 to 100°C, more preferably 5 to 80°C. As a preferable method, when the monomer is added dropwise, the temperature is maintained at 5 to 80°C, and then the mixture is aged at 20 to 80°C.

As the organic solvent which can be added to an aqueous solution of the alkali catalyst or with which the monomer can be diluted, the same organic solvents as those exemplified as the organic solvents which can be added to the aqueous solution of the acid catalyst are preferably used. Note that the organic solvent is used in an amount of preferably 0 to 1,000 ml relative to 1 mol of the monomer because the reaction can be performed economically.

Then, if necessary, neutralization reaction of the catalyst is carried out to obtain an aqueous solution of the reaction mixture. In this event, the amount of an acidic substance usable for the neutralization is preferably 0.1 to 2 equivalents relative to the alkaline substance used as the catalyst. This acidic substance may be any substance as long as it exhibits acidity in water.

Subsequently, by-products such as alcohol produced by the hydrolysis condensation reaction are preferably removed from the aqueous solution of the reaction mixture by a procedure such as removal under reduced pressure. In this event, the aqueous solution of the reaction mixture is heated at a temperature of preferably 0 to 100°C, more preferably 10 to 90°C, further preferably 15 to 80°C, although the temperature depends on the kinds of the added organic solvent and alcohol produced in the reaction. Moreover, the degree of vacuum in this event is preferably atmospheric pressure or less, more preferably 80 kPa or less in absolute pressure, further preferably 50 kPa or less in absolute pressure, although the degree of vacuum varies depending on the kinds of the organic solvent and alcohol to be removed, as well as exhausting equipment, condensation equipment, and heating temperature. In this case, it is difficult to accurately know the amount of alcohol to be removed, but it is desirable to remove about 80 mass% or more of the produced alcohol.

Next, to remove the catalyst used in the hydrolysis condensation, the thermally crosslinkable polysiloxane is extracted with an organic solvent. Preferably, the organic solvent used in this event is capable of dissolving the thermally crosslinkable polysiloxane, and achieves two-layer separation when mixed with water. Examples of the organic solvent include methanol, ethanol, 1-propanol, 2-propanol, 1-butanol, 2-butanol, 2-methyl-1-propanol, acetone, tetrahydrofuran, toluene, hexane, ethyl acetate, cyclohexanone, methyl amyl ketone, propylene glycol monomethyl ether, ethylene glycol monomethyl ether, propylene glycol monoethyl ether, ethylene glycol monoethyl ether, propylene glycol monopropyl ether, ethylene glycol monopropyl ether, propylene glycol dimethyl ether, diethylene glycol dimethyl ether, propylene glycol monomethyl ether acetate, propylene glycol monoethyl ether acetate, ethyl pyruvate, butyl acetate, methyl 3-methoxypropionate, ethyl 3-ethoxypropionate, t-butyl acetate, t-butyl propionate, propylene glycol mono-t-butyl ether acetate, γ-butyrolactone, methyl isobutyl ketone, cyclopentyl methyl ether, mixtures thereof, and the like.

Further, it is also possible to use a mixture of a water-soluble organic solvent and a slightly-water-soluble organic solvent.

As concrete examples of the organic solvent used for removing the alkali catalyst, it is possible to use the same organic solvents or the same mixture of the water-soluble organic solvent and the slightly-water-soluble organic solvent as those specifically exemplified for the acid catalyst removal described above.

Although the mixing ratio of the water-soluble organic solvent and the slightly-water-soluble organic solvent is appropriately selected, the amount of the water-soluble organic solvent is preferably 0.1 to 1,000 parts by mass, more preferably 1 to 500 parts by mass, and further preferably 2 to 100 parts by mass, based on 100 parts by mass of the slightly-water-soluble organic solvent.

Subsequently, the thermally crosslinkable polysiloxane solution may be washed with neutral water. As the water, what is commonly called deionized water or ultrapure water may be used. The amount of the water is preferably 0.01 to 100 L, more preferably 0.05 to 50 L, and further preferably 0.1 to 5 L, relative to 1 L of the thermally crosslinkable polysiloxane solution. This washing procedure may be performed by putting both the thermally crosslinkable polysiloxane solution and neutral water into the same container, followed by stirring and then leaving to stand to separate the aqueous layer. The washing may be performed once or more, preferably once to approximately five times, since washing ten times or more does not always produce the full washing effects thereof.

To the washed thermally crosslinkable polysiloxane solution, a final solvent may be added for solvent exchange under reduced pressure to obtain a desired thermally crosslinkable polysiloxane solution. In this event, the temperature during the solvent exchange is preferably 0 to 100°C, more preferably 10 to 90°C, further preferably 15 to 80°C, depending on the kinds of the extraction solvent to be removed. Moreover, the degree of vacuum in this event is preferably atmospheric pressure or less, more preferably 80 kPa or less in absolute pressure, further preferably 50 kPa or less in absolute pressure, although the degree of vacuum varies depending on the kinds of the extraction solvent to be removed, exhausting equipment, condensation equipment, and heating temperature.

The final solvent added to the thermally crosslinkable polysiloxane solution is preferably an alcohol-based solvent. Particularly preferable alcohol-based solvents include monoalkyl ether derivatives of ethylene glycol, diethylene glycol, triethylene glycol, propylene glycol, dipropylene glycol, and the like. Specifically, preferable examples include propylene glycol monomethyl ether, ethylene glycol monomethyl ether, propylene glycol monoethyl ether, ethylene glycol monoethyl ether, propylene glycol monopropyl ether, ethylene glycol monopropyl ether, diacetone alcohol, and the like.

As an alternative reaction operation using an alkali catalyst, water or a water-containing organic solvent may be added to the monomer or an organic solution of the monomer to initiate the hydrolysis reaction. In this event, the catalyst may be added to the monomer or the organic solution of the monomer, or may be added to the water or the water-containing organic solvent. The reaction temperature is preferably 0 to 100°C, more preferably 10 to 80°C. As a preferable method, when the water is added dropwise, the mixture is heated to 10 to 50°C, and then further heated to 20 to 80°C for aging.

The organic solvent usable for the organic solution of the monomer or the water-containing organic solvent is preferably a water-soluble solvent. Examples thereof include methanol, ethanol, 1-propanol, 2-propanol, 1-butanol, 2-butanol, and 2-methyl-1-propanol, acetone, tetrahydrofuran, acetonitrile; polyhydric alcohol condensate derivatives, such as propylene glycol monomethyl ether, ethylene glycol monomethyl ether, propylene glycol monoethyl ether, ethylene glycol monoethyl ether, propylene glycol monopropyl ether, ethylene glycol monopropyl ether, propylene glycol dimethyl ether, diethylene glycol dimethyl ether, propylene glycol monomethyl ether acetate, propylene glycol monoethyl ether acetate, and propylene glycol monopropyl ether acetate; mixtures thereof, and the like.

The molecular weight of the thermally crosslinkable polysiloxane obtained by the above synthesis method 1 or 2 can be adjusted not only through the selection of the monomers, but also through the control of the reaction condition during the polymerization. When the weight-average molecular weight is 100,000 or less, the generation of foreign matters or coating spots does not occur. Therefore, the polysiloxane preferably has a weight-average molecular weight of 100,000 or less, more preferably 200 to 50,000, further preferably 300 to 30,000. Regarding data on the weight-average molecular weight, the molecular weight is expressed in terms of polystyrene which is obtained by gel permeation chromatography (GPC) using a refractive index (RI) detector, tetrahydrofuran as an eluent, and polystyrene as a reference substance.

The physical properties of the thermally crosslinkable polysiloxane used in the present invention vary depending on the kind of acid or alkali catalyst and reaction conditions adopted in the hydrolysis condensation. Therefore, the physical properties of the thermally crosslinkable polysiloxane can be selected appropriately in accordance with the target performance of the resist underlayer film.

Furthermore, it is possible to use, as a component of a composition for forming a resist underlayer film, a polysiloxane derivative produced from a mixture of one or more kinds of the hydrolysable monomer (Sm) and a hydrolysable metal compound represented by the following general formula (Mm) under conditions using the acid or alkali catalyst.

U(OR⁷)ₘ₇(OR⁸)ₘ₈ (Mm)

In the general formula (Mm), R⁷ and R⁸ each independently represent an organic group having 1 to 30 carbon atoms; m7 + m8 is the same number as a valence determined by the kind of U; "m7" and "m8" each represent an integer of 0 or more; and U represents an element belonging to the group III, IV, or V in the periodic table, other than carbon and silicon.

Examples of the hydrolysable metal compound represented by the general formula (Mm) used in this event include the following. When U is boron, examples of the hydrolysable metal compound shown by the general formula (Mm) include boron methoxide, boron ethoxide, boron propoxide, boron butoxide, boron amyloxide, boron hexyloxide, boron cyclopentoxide, boron cyclohexyloxide, boron allyloxide, boron phenoxide, boron methoxyethoxide, boric acid, boron oxide, and the like.

When U is aluminum, examples of the hydrolysable metal compound shown by the general formula (Mm) include aluminum methoxide, aluminum ethoxide, aluminum propoxide, aluminum butoxide, aluminum amyloxide, aluminum hexyloxide, aluminum cyclopentoxide, aluminum cyclohexyloxide, aluminum allyloxide, aluminum phenoxide, aluminum methoxyethoxide, aluminum ethoxyethoxide, aluminum dipropoxy(ethyl acetoacetate), aluminum dibutoxy(ethyl acetoacetate), aluminum propoxy bis(ethyl acetoacetate), aluminum butoxy bis(ethyl acetoacetate), aluminum 2,4-pentanedionate, aluminum 2,2,6,6-tetramethyl-3,5-heptanedionate, and the like.

When U is gallium, examples of the hydrolysable metal compound shown by the general formula (Mm) include gallium methoxide, gallium ethoxide, gallium propoxide, gallium butoxide, gallium amyloxide, gallium hexyloxide, gallium cyclopentoxide, gallium cyclohexyloxide, gallium allyloxide, gallium phenoxide, gallium methoxyethoxide, gallium ethoxyethoxide, gallium dipropoxy(ethyl acetoacetate), gallium dibutoxy(ethyl acetoacetate), gallium propoxy bis(ethyl acetoacetate), gallium butoxy bis(ethyl acetoacetate), gallium 2,4-pentanedionate, gallium 2,2,6,6-tetramethyl-3,5-heptanedionate, and the like.

When U is yttrium, examples of the hydrolysable metal compound shown by the general formula (Mm) include yttrium methoxide, yttrium ethoxide, yttrium propoxide, yttrium butoxide, yttrium amyloxide, yttrium hexyloxide, yttrium cyclopentoxide, yttrium cyclohexyloxide, yttrium allyloxide, yttrium phenoxide, yttrium methoxyethoxide, yttrium ethoxyethoxide, yttrium dipropoxy(ethyl acetoacetate), yttrium dibutoxy(ethyl acetoacetate), yttrium propoxy bis(ethyl acetoacetate), yttrium butoxy bis(ethyl acetoacetate), yttrium 2,4-pentanedionate, yttrium 2,2,6,6-tetramethyl-3,5-heptanedionate, and the like.

When U is germanium, examples of the hydrolysable metal compound shown by the general formula (Mm) include germanium methoxide, germanium ethoxide, germanium propoxide, germanium butoxide, germanium amyloxide, germanium hexyloxide, germanium cyclopentoxide, germanium cyclohexyloxide, germanium allyloxide, germanium phenoxide, germanium methoxyethoxide, germanium ethoxyethoxide, and the like.

When U is titanium, examples of the hydrolysable metal compound shown by the general formula (Mm) include titanium methoxide, titanium ethoxide, titanium propoxide, titanium butoxide, titanium amyloxide, titanium hexyloxide, titanium cyclopentoxide, titanium cyclohexyloxide, titanium allyloxide, titanium phenoxide, titanium methoxyethoxide, titanium ethoxyethoxide, titanium dipropoxy bis(ethyl acetoacetate), titanium dibutoxy bis(ethyl acetoacetate), titanium dipropoxy bis(2,4-pentanedionate), titanium dibutoxy bis(2,4-pentanedionate), and the like.

When U is hafnium, examples of the hydrolysable metal compound shown by the general formula (Mm) include hafnium methoxide, hafnium ethoxide, hafnium propoxide, hafnium butoxide, hafnium amyloxide, hafnium hexyloxide, hafnium cyclopentoxide, hafnium cyclohexyloxide, hafnium allyloxide, hafnium phenoxide, hafnium methoxyethoxide, hafnium ethoxyethoxide, hafnium dipropoxy bis(ethyl acetoacetate), hafnium dibutoxy bis(ethyl acetoacetate), hafnium dipropoxy bis(2,4-pentanedionate), hafnium dibutoxy bis(2,4-pentanedionate), and the like.

When U is tin, examples of the hydrolysable metal compound shown by the general formula (Mm) include methoxy tin, ethoxy tin, propoxy tin, butoxy tin, phenoxy tin, methoxyethoxy tin, ethoxyethoxy tin, tin 2,4-pentanedionate, tin 2,2,6,6-tetramethyl-3,5-heptanedionate, and the like.

When U is arsenic, examples of the hydrolysable metal compound shown by the general formula (Mm) include methoxy arsenic, ethoxy arsenic, propoxy arsenic, butoxy arsenic, phenoxy arsenic, and the like.

When U is antimony, examples of the hydrolysable metal compound shown by the general formula (Mm) include methoxy antimony, ethoxy antimony, propoxy antimony, butoxy antimony, phenoxy antimony, antimony acetate, antimony propionate, and the like.

When U is niobium, examples of the hydrolysable metal compound shown by the general formula (Mm) include methoxy niobium, ethoxy niobium, propoxy niobium, butoxy niobium, phenoxy niobium, and the like.

When U is tantalum, examples of the hydrolysable metal compound shown by the general formula (Mm) include methoxy tantalum, ethoxy tantalum, propoxy tantalum, butoxy tantalum, phenoxy tantalum, and the like.

When U is bismuth, examples of the hydrolysable metal compound shown by the general formula (Mm) include methoxy bismuth, ethoxy bismuth, propoxy bismuth, butoxy bismuth, phenoxy bismuth, and the like.

When U is phosphorus, examples of the hydrolysable metal compound shown by the general formula (Mm) include trimethyl phosphate, triethyl phosphate, tripropyl phosphate, trimethyl phosphite, triethyl phosphite, tripropyl phosphite, diphosphorous pentaoxide, and the like.

When U is vanadium, examples of the hydrolysable metal compound shown by the general formula (Mm) include vanadium oxide bis(2,4-pentanedionate), vanadium 2,4-pentanedionate, vanadium tributoxide oxide, vanadium tripropoxide oxide, and the like.

When U is zirconium, examples of the hydrolysable metal compound shown by the general formula (Mm) include methoxy zirconium, ethoxy zirconium, propoxy zirconium, butoxy zirconium, phenoxy zirconium, zirconium dibutoxide bis(2,4-pentanedionate), zirconium dipropoxide bis(2,2,6,6-tetramethyl-3,5-heptanedionate), and the like.

In the inventive composition for forming a silicon-containing resist underlayer film, the thermally crosslinkable polysiloxane (Sx) is preferably contained in an amount of, for example, 0.1 to 10 mass% relative to the solvent.

### [Acid Generator]

The inventive composition for forming a silicon-containing resist underlayer film preferably further contains an acid generator. One or more kinds of the acid generator can be contained. As the acid generator, any substance may be used, for example, a thermal acid generator, a photo-acid generator, or an acid amplifier, as long as the substance functions as an acid precursor. In the present invention, it is preferable that the acid generator is a photo-acid generator, which generates an acid by the action of a high-energy beam, and more preferable that the acid generator to be contained is a sulfonium salt and is a photo-acid generator, which generates an acid by the action of a high-energy beam. Further specific examples include materials shown in paragraphs [0160] to [0179] of JP2009-126940A, but are not limited thereto. The acid generator is preferably contained in an amount of 0 to 10 parts by mass, more preferably 0.1 to 5 parts by mass based on 100 parts by mass of the thermally crosslinkable polysiloxane (Sx).

### [Other Components]

### (Crosslinking Catalyst)

The inventive composition for forming a silicon-containing resist underlayer film may contain another crosslinking catalyst (Xc) in addition to the polyvalent ammonium salt compound represented by the general formula (A-1). Examples of crosslinking catalysts (Xc) that can be added include compounds represented by the following general formula (Xc0).

LₐH_{b}A (Xc0)

In the general formula (Xc0), L represents lithium, sodium, potassium, rubidium, cesium, sulfonium, iodonium, phosphonium, or ammonium; A represents a non-nucleophilic counter ion; "a" represents an integer of 1 or more; "b" represents 0 or an integer of 1 or more; and a + b is a valence of the non-nucleophilic counter ion.

As examples of crosslinking catalysts (Xc) that may be used in the present invention, specific examples of the compounds represented by the general formula (Xc0) include a sulfonium salt of the following general formula (Xc-1), an iodonium salt of the following general formula (Xc-2), a phosphonium salt of the following general formula (Xc-3), an ammonium salt of the following general formula (Xc-4), an alkaline metal salt, and the like.

Examples of the sulfonium salt (Xc-1), the iodonium salt (Xc-2), the phosphonium salt (Xc-3), and the ammonium salt (Xc-4) include the following.

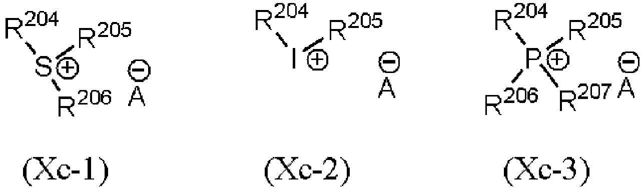

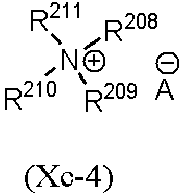

In the formulae, R²⁰⁴, R²⁰⁵, R²⁰⁶, and R²⁰⁷ each represent a linear, branched, or cyclic alkyl group, alkenyl group, oxoalkyl group, or oxoalkenyl group having 1 to 12 carbon atoms, a substituted or unsubstituted aryl group having 6 to 20 carbon atoms, or an aralkyl group or aryloxoalkyl group having 7 to 12 carbon atoms; part or all of the hydrogen atoms of these groups are optionally substituted with an alkoxy group or the like. Additionally, R²⁰⁵ and R²⁰⁶ may form a ring; when a ring is formed, R²⁰⁵ and R²⁰⁶ each represent an alkylene group having 1 to 6 carbon atoms. A⁻ represents a non-nucleophilic counter ion. R²⁰⁸, R²⁰⁹, R²¹⁰, and R²¹¹ are the same as R²⁰⁴, R²⁰⁵, R²⁰⁶, and R²⁰⁷, or may each be a hydrogen atom. R²⁰⁸ and R²⁰⁹, or R²⁰⁸, R²⁰⁹ and R²¹⁰ may form a ring; when a ring is formed, R²⁰⁸ and R²⁰⁹, or R²⁰⁸, R²⁰⁹ and R²¹⁰, represent an alkylene group having 3 to 10 carbon atoms.

R²⁰⁴, R²⁰⁵, R²⁰⁶, R²⁰⁷, R²⁰⁸, R²⁰⁹, R²¹⁰, and R²¹¹ may be identical to or different from one another. Specifically, examples of the alkyl group include a methyl group, an ethyl group, a propyl group, an isopropyl group, an n-butyl group, a sec-butyl group, a tert-butyl group, a pentyl group, a hexyl group, a heptyl group, an octyl group, a cyclopentyl group, a cyclohexyl group, a cycloheptyl group, a cyclopropylmethyl group, a 4-methylcyclohexyl group, a cyclohexylmethyl group, a norbornyl group, an adamantyl group, and the like. Examples of the alkenyl group include a vinyl group, an allyl group, a propenyl group, a butenyl group, a hexenyl group, a cyclohexenyl group, and the like. Examples of the oxoalkyl group include a 2-oxocyclopentyl group, a 2-oxocyclohexyl group, a 2-oxopropyl group, a 2-cyclopentyl-2-oxoethyl group, a 2-cyclohexyl-2-oxoethyl group, a 2-(4-methylcyclohexyl)-2-oxoethyl group, and the like. Examples of the oxoalkenyl group include a 2-oxopropenyl group, a 2-oxobutenyl group, a 2-oxohexenyl group, a 2-oxocyclopentenyl group, a 2-oxocyclohexenyl group, and the like. Examples of the aryl group include a phenyl group, a naphthyl group, and the like; alkoxyphenyl groups, such as a p-methoxyphenyl group, an m-methoxyphenyl group, an o-methoxyphenyl group, an ethoxyphenyl group, a p-tert-butoxyphenyl group, and an m-tert-butoxyphenyl group; alkylphenyl groups, such as a 2-methylphenyl group, a 3-methylphenyl group, a 4-methylphenyl group, an ethylphenyl group, a 4-tert-butylphenyl group, a 4-butylphenyl group, and a dimethylphenyl group; alkylnaphthyl groups, such as a methylnaphthyl group and an ethylnaphthyl group; alkoxynaphthyl groups, such as a methoxynaphthyl group and an ethoxynaphthyl group; dialkylnaphthyl groups, such as a dimethylnaphthyl group and a diethylnaphthyl group; dialkoxynaphthyl groups, such as a dimethoxynaphthyl group and a diethoxynaphthyl group; and the like. Examples of the aralkyl group include a benzyl group, a phenylethyl group, a phenethyl group, and the like. Examples of the aryloxoalkyl group include 2-aryl-2-oxoethyl groups such as a 2-phenyl-2-oxoethyl group, a 2-(1-naphthyl)-2-oxoethyl group, and a 2-(2-naphthyl)-2-oxoethyl group; and the like.

Examples of the non-nucleophilic counter ion A⁻ in (Xc-1) to (Xc-4) include monovalent ions, such as hydroxide ion, formate ion, acetate ion, propionate ion, butanoate ion, pentanoate ion, hexanoate ion, heptanoate ion, octanoate ion, nonanoate ion, decanoate ion, oleate ion, stearate ion, linoleate ion, linolenate ion, benzoate ion, phthalate ion, isophthalate ion, terephthalate ion, salicylate ion, trifluoroacetate ion, monochloroacetate ion, dichloroacetate ion, trichloroacetate ion, fluoride ion, chloride ion, bromide ion, iodide ion, nitrate ion, nitrite ion, chlorate ion, bromate ion, methanesulfonate ion, paratoluenesulfonate ion, and monomethylsulfate ion; monovalent or divalent ions such as oxalate ion, malonate ion, methylmalonate ion, ethylmalonate ion, propylmalonate ion, butylmalonate ion, dimethylmalonate ion, diethylmalonate ion, succinate ion, methylsuccinate ion, glutarate ion, adipate ion, itaconate ion, maleate ion, fumarate ion, citraconate ion, citrate ion, carbonate ion, sulfate ion, and the like.

Examples of the alkaline metal salt include salts of lithium, sodium, potassium, cesium, magnesium, and calcium; monovalent salts, such as hydroxide, formate, acetate, propionate, butanoate, pentanoate, hexanoate, heptanoate, octanoate, nonanoate, decanoate, oleate, stearate, linoleate, linolenate, benzoate, phthalate, isophthalate, terephthalate, salicylate, trifluoroacetate, monochloroacetate, dichloroacetate, and trichloroacetate; monovalent or divalent salts, such as oxalate, malonate, methylmalonate, ethylmalonate, propylmalonate, butylmalonate, dimethylmalonate, diethylmalonate, succinate, methylsuccinate, glutarate, adipate, itaconate, maleate, fumarate, citraconate, citrate, carbonate, and the like.

Specific examples of the sulfonium salt (Xc-1) include triphenylsulfonium formate, triphenylsulfonium acetate, triphenylsulfonium propionate, triphenylsulfonium butanoate, triphenylsulfonium benzoate, triphenylsulfonium phthalate, triphenylsulfonium isophthalate, triphenylsulfonium terephthalate, triphenylsulfonium salicylate, triphenylsulfonium trifluoromethanesulfonate, triphenylsulfonium trifluoroacetate, triphenylsulfonium monochloroacetate, triphenylsulfonium dichloroacetate, triphenylsulfonium trichloroacetate, triphenylsulfonium hydroxide, triphenylsulfonium nitrate, triphenylsulfonium chloride, triphenylsulfonium bromide, triphenylsulfonium oxalate, triphenylsulfonium malonate, triphenylsulfonium methylmalonate, triphenylsulfonium ethylmalonate, triphenylsulfonium propylmalonate, triphenylsulfonium butylmalonate, triphenylsulfonium dimethylmalonate, triphenylsulfonium diethylmalonate, triphenylsulfonium succinate, triphenylsulfonium methylsuccinate, triphenylsulfonium glutarate, triphenylsulfonium adipate, triphenylsulfonium itaconate, triphenylsulfonium maleate, triphenylsulfonium fumarate, triphenylsulfonium citraconate, triphenylsulfonium citrate, triphenylsulfonium carbonate, bistriphenylsulfonium oxalate, bistriphenylsulfonium maleate, bistriphenylsulfonium fumarate, bistriphenylsulfonium citraconate, bistriphenylsulfonium citrate, bistriphenylsulfonium carbonate, and the like.

Specific examples of the iodonium salt (Xc-2) include diphenyliodonium formate, diphenyliodonium acetate, diphenyliodonium propionate, diphenyliodonium butanoate, diphenyliodonium benzoate, diphenyliodonium phthalate, diphenyliodonium isophthalate, diphenyliodonium terephthalate, diphenyliodonium salicylate, diphenyliodonium trifluoromethanesulfonate, diphenyliodonium trifluoroacetate, diphenyliodonium monochloroacetate, diphenyliodonium dichloroacetate, diphenyliodonium trichloroacetate, diphenyliodonium hydroxide, diphenyliodonium nitrate, diphenyliodonium chloride, diphenyliodonium bromide, diphenyliodonium iodide, diphenyliodonium oxalate, diphenyliodonium maleate, diphenyliodonium fumarate, diphenyliodonium citraconate, diphenyliodonium citrate, diphenyliodonium carbonate, bisdiphenyliodonium oxalate, bisdiphenyliodonium maleate, bisdiphenyliodonium fumarate, bisdiphenyliodonium citraconate, bisdiphenyliodonium citrate, bisdiphenyliodonium carbonate, and the like.

Specific examples of the phosphonium salt (Xc-3) include tetraethylphosphonium formate, tetraethylphosphonium acetate, tetraethylphosphonium propionate, tetraethylphosphonium butanoate, tetraethylphosphonium benzoate, tetraethylphosphonium phthalate, tetraethylphosphonium isophthalate, tetraethylphosphonium terephthalate, tetraethylphosphonium salicylate, tetraethylphosphonium trifluoromethanesulfonate, tetraethylphosphonium trifluoroacetate, tetraethylphosphonium monochloroacetate, tetraethylphosphonium dichloroacetate, tetraethylphosphonium trichloroacetate, tetraethylphosphonium hydroxide, tetraethylphosphonium nitrate, tetraethylphosphonium chloride, tetraethylphosphonium bromide, tetraethylphosphonium iodide, tetraethylphosphonium oxalate, tetraethylphosphonium maleate, tetraethylphosphonium fumarate, tetraethylphosphonium citraconate, tetraethylphosphonium citrate, tetraethylphosphonium carbonate, bistetraethylphosphonium oxalate, bistetraethylphosphonium maleate, bistetraethylphosphonium fumarate, bistetraethylphosphonium citraconate, bistetraethylphosphonium citrate, bistetraethylphosphonium carbonate, tetraphenylphosphonium formate, tetraphenylphosphonium acetate, tetraphenylphosphonium propionate, tetraphenylphosphonium butanoate, tetraphenylphosphonium benzoate, tetraphenylphosphonium phthalate, tetraphenylphosphonium isophthalate, tetraphenylphosphonium terephthalate, tetraphenylphosphonium salicylate, tetraphenylphosphonium trifluoromethanesulfonate, tetraphenylphosphonium trifluoroacetate, tetraphenylphosphonium monochloroacetate, tetraphenylphosphonium dichloroacetate, tetraphenylphosphonium trichloroacetate, tetraphenylphosphonium hydroxide, tetraphenylphosphonium nitrate, tetraphenylphosphonium chloride, tetraphenylphosphonium bromide, tetraphenylphosphonium iodide, tetraphenylphosphonium oxalate, tetraphenylphosphonium maleate, tetraphenylphosphonium fumarate, tetraphenylphosphonium citraconate, tetraphenylphosphonium citrate, tetraphenylphosphonium carbonate, bistetraphenylphosphonium oxalate, bistetraphenylphosphonium maleate, bistetraphenylphosphonium fumarate, bistetraphenylphosphonium citraconate, bistetraphenylphosphonium citrate, bistetraphenylphosphonium carbonate, and the like.

Specific examples of the ammonium salt (Xc-4) include tetramethylammonium formate, tetramethylammonium acetate, tetramethylammonium propionate, tetramethylammonium butanoate, tetramethylammonium benzoate, tetramethylammonium phthalate, tetramethylammonium isophthalate, tetramethylammonium terephthalate, tetramethylammonium salicylate, tetramethylammonium trifluoromethanesulfonate, tetramethylammonium trifluoroacetate, tetramethylammonium monochloroacetate, tetramethylammonium dichloroacetate, tetramethylammonium trichloroacetate, tetramethylammonium hydroxide, tetramethylammonium nitrate, tetramethylammonium chloride, tetramethylammonium bromide, tetramethylammonium iodide, tetramethylammonium monomethylsulfate, tetramethylammonium oxalate, tetramethylammonium malonate, tetramethylammonium maleate, tetramethylammonium fumarate, tetramethylammonium citraconate, tetramethylammonium citrate, tetramethylammonium carbonate, bistetramethylammonium oxalate, bistetramethylammonium malonate, bistetramethylammonium maleate, bistetramethylammonium fumarate, bistetramethylammonium citraconate, bistetramethylammonium citrate, bistetramethylammonium carbonate, tetraethylammonium formate, tetraethylammonium acetate, tetraethylammonium propionate, tetraethylammonium butanoate, tetraethylammonium benzoate, tetraethylammonium phthalate, tetraethylammonium isophthalate, tetraethylammonium terephthalate, tetraethylammonium salicylate, tetraethylammonium trifluoromethanesulfonate, tetraethylammonium trifluoroacetate, tetraethylammonium monochloroacetate, tetraethylammonium dichloroacetate, tetraethylammonium trichloroacetate, tetraethylammonium hydroxide, tetraethylammonium nitrate, tetraethylammonium chloride, tetraethylammonium bromide, tetraethylammonium iodide, tetraethylammonium monomethylsulfate, tetraethylammonium oxalate, tetraethylammonium malonate, tetraethylammonium maleate, tetraethylammonium fumarate, tetraethylammonium citraconate, tetraethylammonium citrate, tetraethylammonium carbonate, bistetraethylammonium oxalate, bistetraethylammonium malonate, bistetraethylammonium maleate, bistetraethylammonium fumarate, bistetraethylammonium citraconate, bistetraethylammonium citrate, bistetraethylammonium carbonate, tetrapropylammonium formate, tetrapropylammonium acetate, tetrapropylammonium propionate, tetrapropylammonium butanoate, tetrapropylammonium benzoate, tetrapropylammonium phthalate, tetrapropylammonium isophthalate, tetrapropylammonium terephthalate, tetrapropylammonium salicylate, tetrapropylammonium trifluoromethanesulfonate, tetrapropylammonium trifluoroacetate, tetrapropylammonium monochloroacetate, tetrapropylammonium dichloroacetate, tetrapropylammonium trichloroacetate, tetrapropylammonium hydroxide, tetrapropylammonium nitrate, tetrapropylammonium chloride, tetrapropylammonium bromide, tetrapropylammonium iodide, tetrapropylammonium monomethylsulfate, tetrapropylammonium oxalate, tetrapropylammonium malonate, tetrapropylammonium maleate, tetrapropylammonium fumarate, tetrapropylammonium citraconate, tetrapropylammonium citrate, tetrapropylammonium carbonate, bistetrapropylammonium oxalate, bistetrapropylammonium malonate, bistetrapropylammonium maleate, bistetrapropylammonium fumarate, bistetrapropylammonium citraconate, bistetrapropylammonium citrate, bistetrapropylammonium carbonate, tetrabutylammonium formate, tetrabutylammonium acetate, tetrabutylammonium propionate, tetrabutylammonium butanoate, tetrabutylammonium benzoate, tetrabutylammonium phthalate, tetrabutylammonium isophthalate, tetrabutylammonium terephthalate, tetrabutylammonium salicylate, tetrabutylammonium trifluoromethanesulfonate, tetrabutylammonium trifluoroacetate, tetrabutylammonium monochloroacetate, tetrabutylammonium dichloroacetate, tetrabutylammonium trichloroacetate, tetrabutylammonium hydroxide, tetrabutylammonium nitrate, tetrabutylammonium chloride, tetrabutylammonium bromide, tetrabutylammonium iodide, tetrabutylammonium methanesulfonate, tetrabutylammonium monomethylsulfate, tetrabutylammonium oxalate, tetrabutylammonium malonate, tetrabutylammonium maleate, tetrabutylammonium fumarate, tetrabutylammonium citraconate, tetrabutylammonium citrate, tetrabutylammonium carbonate, bistetrabutylammonium oxalate, bistetrabutylammonium malonate, bistetrabutylammonium maleate, bistetrabutylammonium fumarate, bistetrabutylammonium citraconate, bistetrabutylammonium citrate, bistetrabutylammonium carbonate, and the like.

Examples of the alkaline metal salt include lithium formate, lithium acetate, lithium propionate, lithium butanoate, lithium benzoate, lithium phthalate, lithium isophthalate, lithium terephthalate, lithium salicylate, lithium trifluoromethanesulfonate, lithium trifluoroacetate, lithium monochloroacetate, lithium dichloroacetate, lithium trichloroacetate, lithium hydroxide, lithium nitrate, lithium chloride, lithium bromide, lithium iodide, lithium methanesulfonate, lithium hydrogen oxalate, lithium hydrogen malonate, lithium hydrogen maleate, lithium hydrogen fumarate, lithium hydrogen citraconate, lithium hydrogen citrate, lithium hydrogen carbonate, lithium oxalate, lithium malonate, lithium maleate, lithium fumarate, lithium citraconate, lithium citrate, lithium carbonate, sodium formate, sodium acetate, sodium propionate, sodium butanoate, sodium benzoate, sodium phthalate, sodium isophthalate, sodium terephthalate, sodium salicylate, sodium trifluoromethanesulfonate, sodium trifluoroacetate, sodium monochloroacetate, sodium dichloroacetate, sodium trichloroacetate, sodium hydroxide, sodium nitrate, sodium chloride, sodium bromide, sodium iodide, sodium methanesulfonate, sodium hydrogen oxalate, sodium hydrogen malonate, sodium hydrogen maleate, sodium hydrogen fumarate, sodium hydrogen citraconate, sodium hydrogen citrate, sodium hydrogen carbonate, sodium oxalate, sodium malonate, sodium maleate, sodium fumarate, sodium citraconate, sodium citrate, sodium carbonate, potassium formate, potassium acetate, potassium propionate, potassium butanoate, potassium benzoate, potassium phthalate, potassium isophthalate, potassium terephthalate, potassium salicylate, potassium trifluoromethanesulfonate, potassium trifluoroacetate, potassium monochloroacetate, potassium dichloroacetate, potassium trichloroacetate, potassium hydroxide, potassium nitrate, potassium chloride, potassium bromide, potassium iodide, potassium methanesulfonate, potassium hydrogen oxalate, potassium hydrogen malonate, potassium hydrogen maleate, potassium hydrogen fumarate, potassium hydrogen citraconate, potassium hydrogen citrate, potassium hydrogen carbonate, potassium oxalate, potassium malonate, potassium maleate, potassium fumarate, potassium citraconate, potassium citrate, potassium carbonate, and the like.

In the present invention, as the crosslinking catalyst (Xc), the composition for forming a resist underlayer film may contain a polysiloxane (Xc-10) having an ammonium salt, a sulfonium salt, a phosphonium salt, or an iodonium salt as part of the structure.

As a raw material for producing (Xc-10) used here, it is possible to employ a compound represented by the following general formula (Xm):

R^{1A}_{A1}R^{2A}_{A2}R^{3A}_{A3}Si(OR^{0A})_{(4-A1-A2-A3)} (Xm)

In the general formula (Xm), R^{0A} represents a hydrocarbon group having 1 to 6 carbon atoms; at least one of R^{1A}, R^{2A}, and R^{3A} represents an organic group having the ammonium salt, the sulfonium salt, the phosphonium salt, or the iodonium salt; the other(s) of R^{1A}, R^{2A}, and R^{3A} represent a hydrogen atom or a monovalent organic group having 1 to 30 carbon atoms; and A1, A2, and A3 each represent 0 or 1, given that 1 ≤ A1 + A2 + A3 ≤ 3.

Here, examples of R^{0A} include a methyl group, an ethyl group, an n-propyl group, an iso-propyl group, an n-butyl group, an iso-butyl group, a sec-butyl group, a tert-butyl group, an n-pentyl group, a cyclopentyl group, an n-hexyl group, a cyclohexyl group, and a phenyl group.

Examples of Xm include a compound represented by the following general formula (Xm-1), being a hydrolysable silicon compound having a structure partially containing a sulfonium salt.

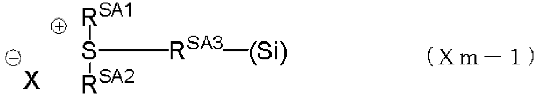

In the general formula (Xm-1), R^{SA1} and R^{SA2} each represent a linear, branched, or cyclic alkyl group, alkenyl group, oxoalkyl group, or oxoalkenyl group having 1 to 20 carbon atoms, a substituted or unsubstituted aryl group having 6 to 20 carbon atoms, or an aralkyl group or aryloxyalkyl group having 7 to 20 carbon atoms; and some or all of the hydrogen atoms of these groups are optionally substituted with an alkoxy group, an amino group, an alkylamino group, a halogen atom, or the like. Moreover, R^{SA1} and R^{SA2} may form a ring together with a sulfur atom bonded to R^{SA1} and R^{SA2}; and when a ring is formed, R^{SA1} and R^{SA2} each represent an alkylene group having 1 to 6 carbon atoms. R^{SA3} represents a linear, branched, or cyclic alkylene group or alkenylene group having 1 to 20 carbon atoms, or a substituted or unsubstituted arylene group having 6 to 20 carbon atoms; and some or all of the hydrogen atoms of these groups are optionally substituted with an alkoxy group, an amino group, an alkylamino group, or the like.

In the general formula (Xm-1), (Si) shows an attachment point to Si.

Examples of X⁻ include hydroxide ion, fluoride ion, chloride ion, bromide ion, iodide ion, formate ion, acetate ion, propionate ion, butanoate ion, pentanoate ion, hexanoate ion, heptanoate ion, octanoate ion, nonanoate ion, decanoate ion, oleate ion, stearate ion, linoleate ion, linolenate ion, benzoate ion, p-methylbenzoate ion, p-t-butylbenzoate ion, phthalate ion, isophthalate ion, terephthalate ion, salicylate ion, trifluoroacetate ion, monochloroacetate ion, dichloroacetate ion, trichloroacetate ion, nitrate ion, chlorate ion, perchlorate ion, bromate ion, iodate ion, methanesulfonate ion, benzenesulfonate ion, toluenesulfonate ion, monomethylsulfate ion, hydrogen sulfate ion, oxalate ion, malonate ion, methylmalonate ion, ethylmalonate ion, propylmalonate ion, butylmalonate ion, dimethylmalonate ion, diethylmalonate ion, succinate ion, methylsuccinate ion, glutarate ion, adipate ion, itaconate ion, maleate ion, fumarate ion, citraconate ion, citrate ion, carbonate ion, and the like.

Specific examples of the cation moiety of the compound represented by the general formula (Xm-1) include the following ions.

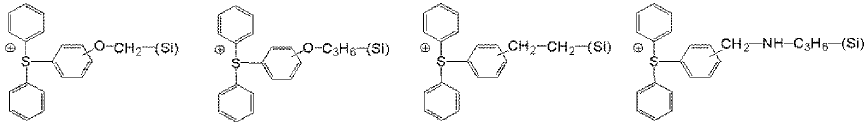

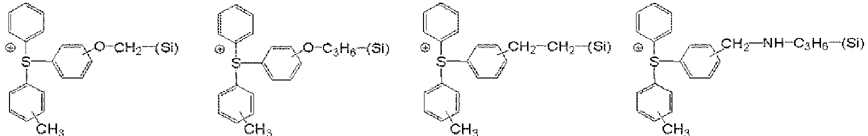

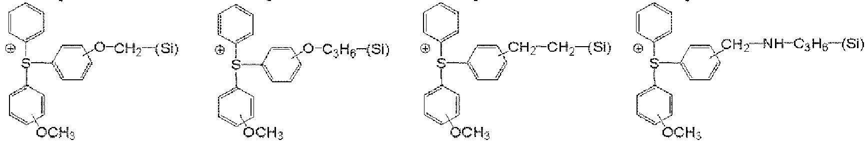

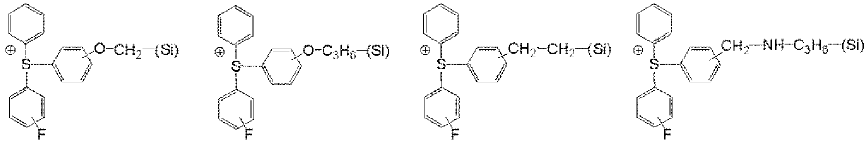

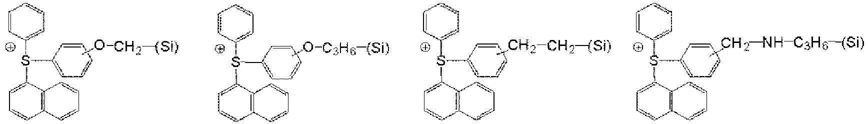

Examples of Xm include a compound represented by the following general formula (Xm-2), being a hydrolysable silicon compound having a structure partially containing an iodonium salt.

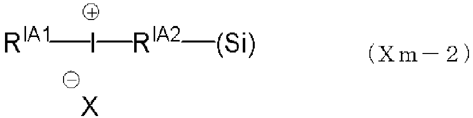

In the general formula (Xm-2), R^{IA1} represents a linear, branched, or cyclic alkyl group, alkenyl group, oxoalkyl group, or oxoalkenyl group having 1 to 20 carbon atoms, a substituted or unsubstituted aryl group having 6 to 20 carbon atoms, or an aralkyl group or an aryloxoalkyl group having 7 to 20 carbon atoms; some or all of the hydrogen atoms of these groups are optionally substituted with an alkoxy group, an amino group, an alkylamino group, a halogen atom, or the like. R^{IA2} represents a linear, branched, or cyclic alkylene group or alkenylene group having 1 to 20 carbon atoms, or a substituted or unsubstituted arylene group having 6 to 20 carbon atoms; and some or all of the hydrogen atoms of these groups are optionally substituted with an alkoxy group, an amino group, an alkylamino group, or the like.

Note that in the general formula (Xm-2), (Si) shows an attachment point to Si. X⁻ is as defined above.

Specific examples of the cation moiety of the compound represented by the general formula (Xm-2) include the following ions.

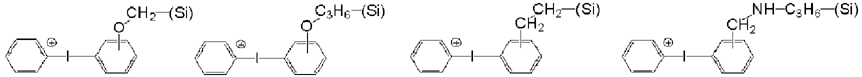

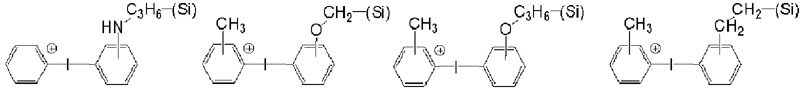

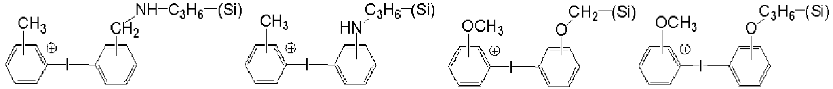

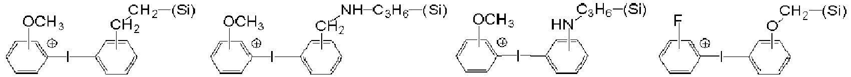

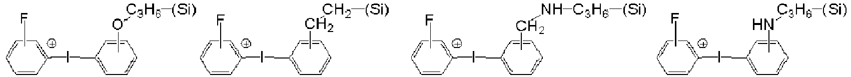

Examples of Xm include a compound represented by the following general formula (Xm-3), being a hydrolysable silicon compound having a structure partially containing a phosphonium salt.

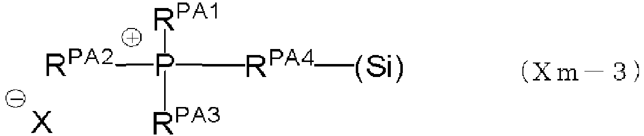

In the general formula (Xm-3), R^{PA1}, R^{PA2}, and R^{PA3} each represent a linear, branched, or cyclic alkyl group, alkenyl group, oxoalkyl group, or oxoalkenyl group having 1 to 20 carbon atoms, a substituted or unsubstituted aryl group having 6 to 20 carbon atoms, or an aralkyl group or an aryloxoalkyl group having 7 to 20 carbon atoms; and part or all of the hydrogen atoms of these groups are optionally substituted with an alkoxy group, an amino group, an alkylamino group, a halogen atom, or the like. Moreover, R^{PA1} and R^{PA2} may form a ring together with a phosphorus atom bonded to R^{PA1} and R^{PA2}; and when a ring is formed, R^{PA1} and R^{PA2} each represent an alkylene group having 1 to 6 carbon atoms. R^{PA4} represents a linear, branched, or cyclic alkylene group or alkenylene group having 1 to 20 carbon atoms, or a substituted or unsubstituted arylene group having 6 to 20 carbon atoms; and part or all of the hydrogen atoms of these groups are optionally substituted with an alkoxy group, an amino group, an alkylamino group, or the like.

Note that in the general formula (Xm-3), (Si) shows an attachment point to Si. X⁻ is as defined above.

Specific examples of the cation moiety of the compound represented by the general formula (Xm-3) include the following ions.

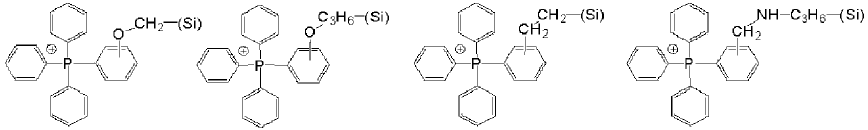

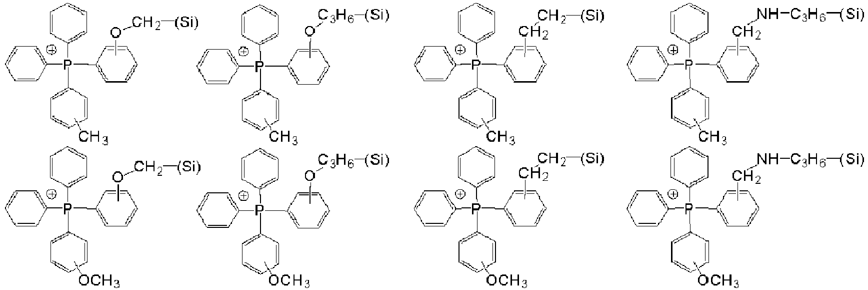

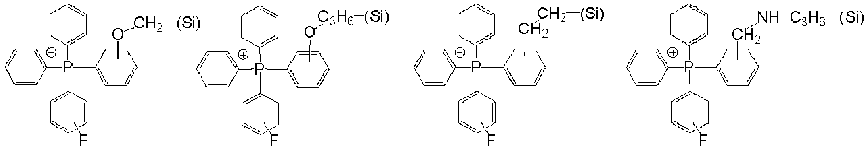

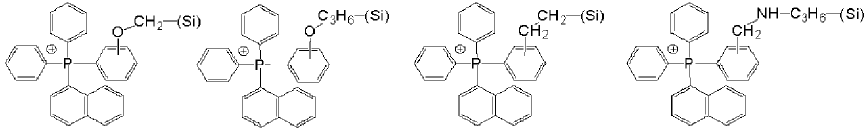

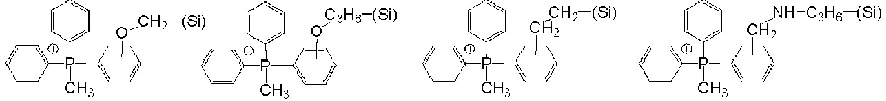

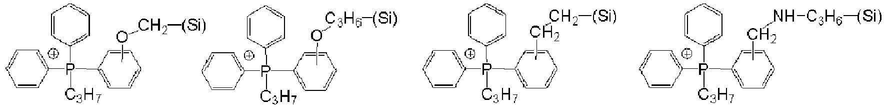

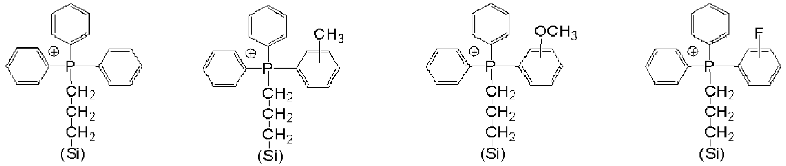

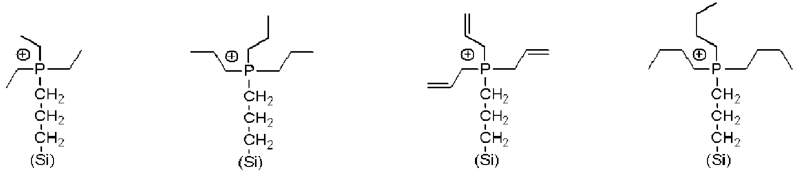

Examples of Xm include a compound represented by the following general formula (Xm-4), being a hydrolysable silicon compound having a structure partially containing an ammonium salt.

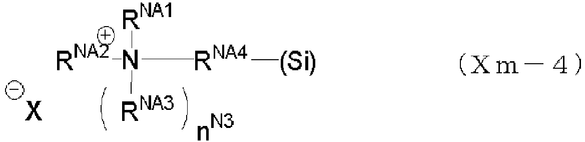

In the general formula (Xm-4), R^{NA1}, R^{NA2}, and R^{NA3} each represent a hydrogen atom, a linear, branched, or cyclic alkyl group, alkenyl group, oxoalkyl group, or oxoalkenyl group having 1 to 20 carbon atoms, a substituted or unsubstituted aryl group having 6 to 20 carbon atoms, or an aralkyl group or aryloxyalkyl group having 7 to 20 carbon atoms; and some or all of the hydrogen atoms of these groups are optionally substituted with an alkoxy group, an amino group, an alkylamino group, or the like. Moreover, R^{NA1} and R^{NA2} may form a ring together with a nitrogen atom bonded to R^{NA1} and R^{NA2}; and when a ring is formed, R^{NA1} and R^{NA2} each represent an alkylene group having 1 to 6 carbon atoms or a cyclic heterocyclic ring or heteroaromatic ring containing nitrogen. R^{NA4} represents a linear, branched, or cyclic alkylene group or alkenylene group having 1 to 20 carbon atoms, or a substituted or unsubstituted arylene group having 6 to 20 carbon atoms; and some or all of the hydrogen atoms of these groups are optionally substituted with an alkoxy group, an amino group, an alkylamino group, or the like. In the case where R^{NA1} and R^{NA2}, or R^{NA1} and R^{NA4}, form a cyclic structure which further contains unsaturated nitrogen, n^{N3}=0; in the other cases, n^{N3}=1.

Note that in the general formula (Xm-4), (Si) shows an attachment point to Si. X⁻ is as defined above.

Specific examples of the cation moiety of the compound represented by the general formula (Xm-4) include the following ions.

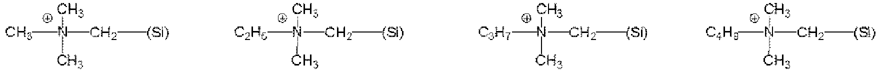

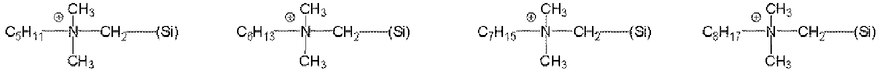

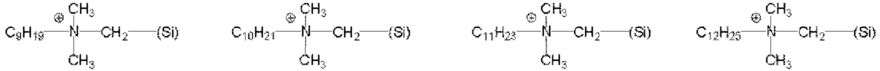

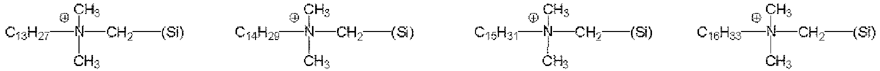

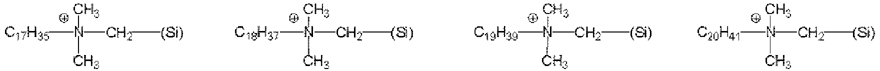

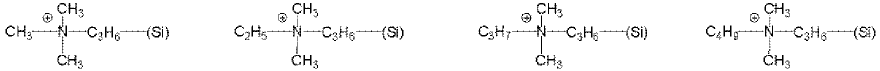

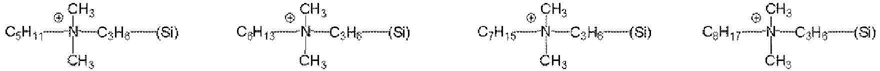

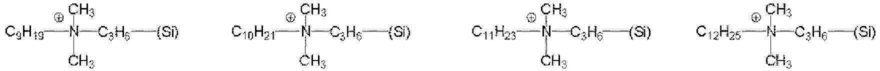

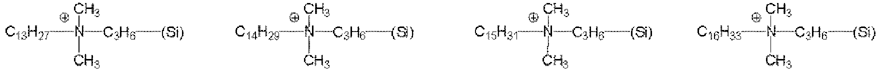

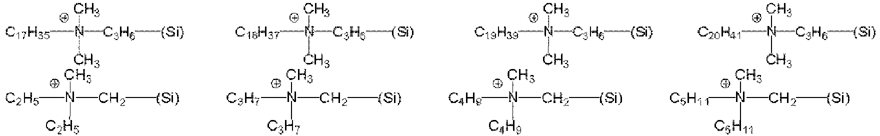

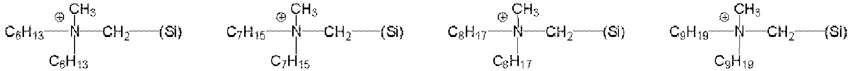

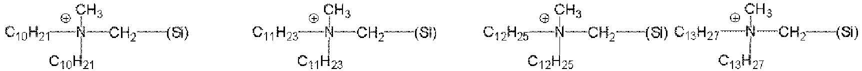

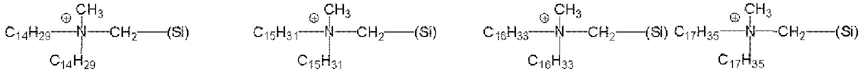

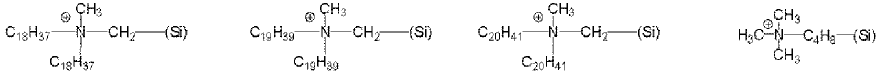

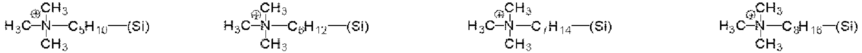

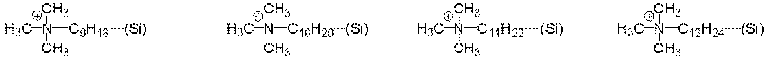

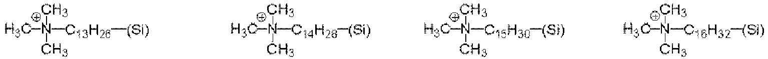

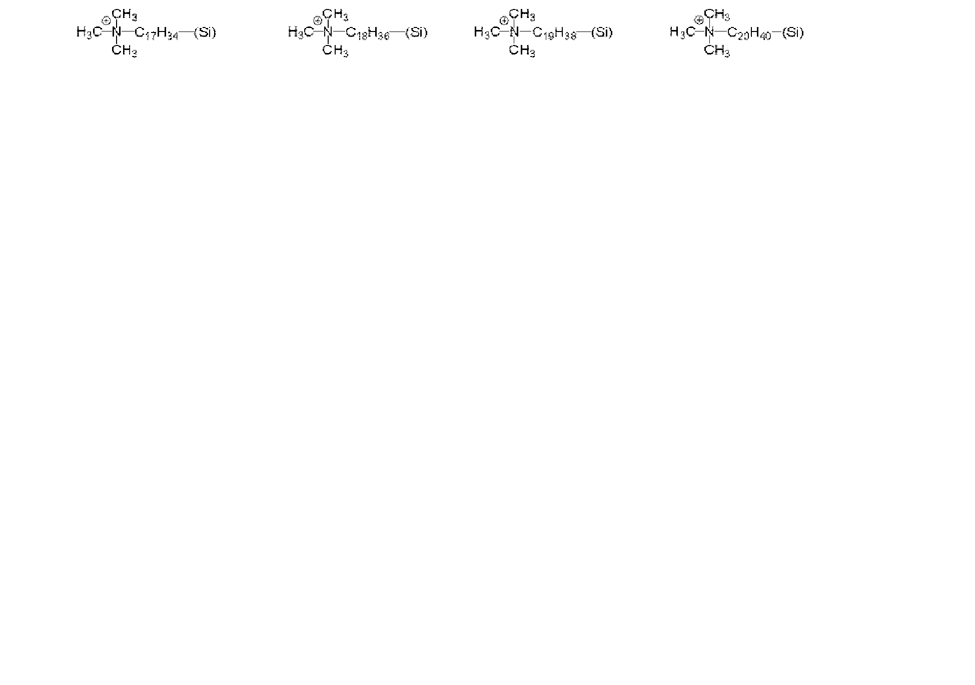

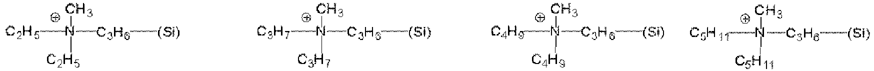

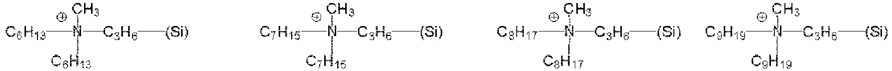

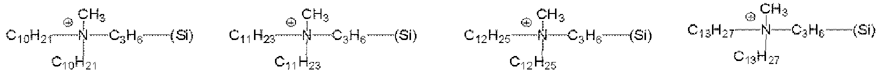

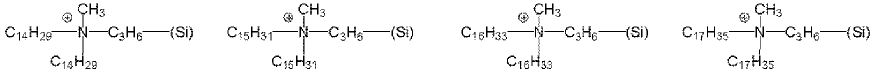

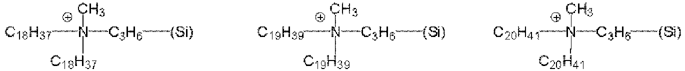

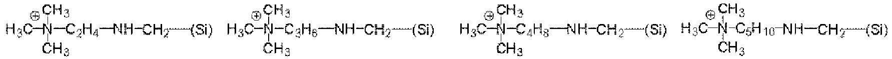

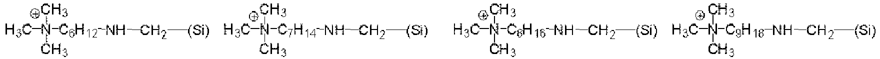

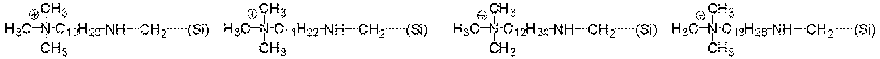

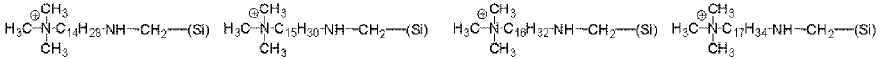

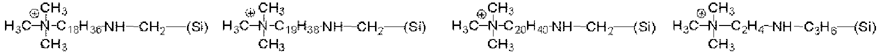

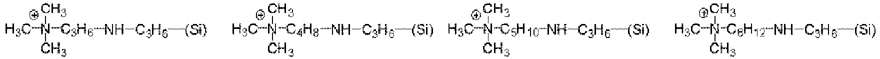

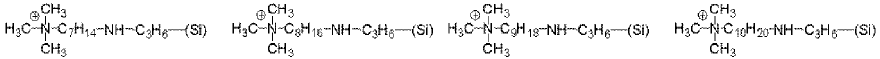

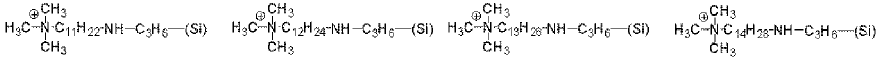

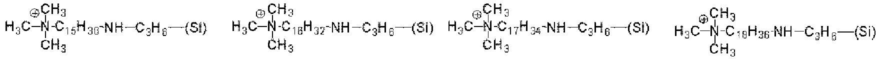

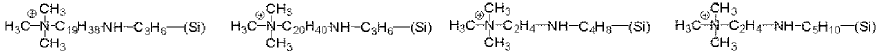

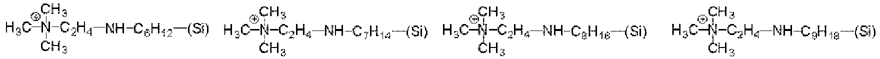

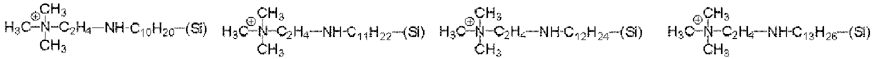

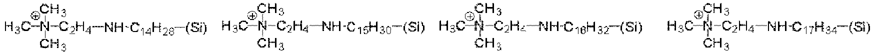

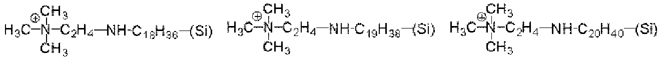

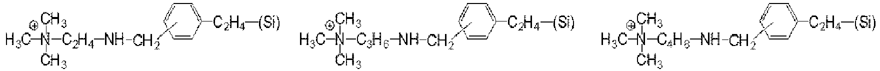

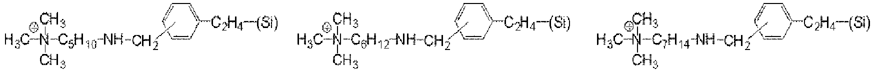

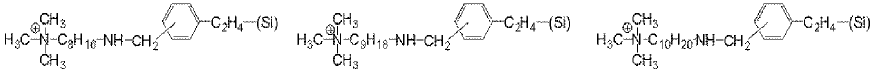

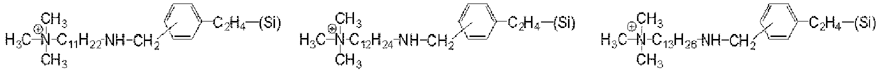

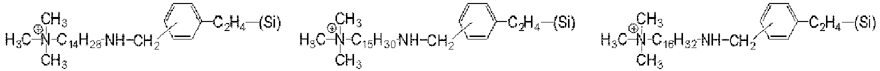

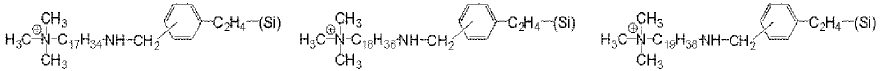

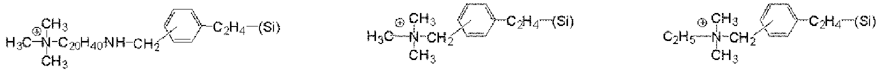

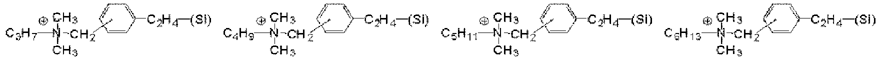

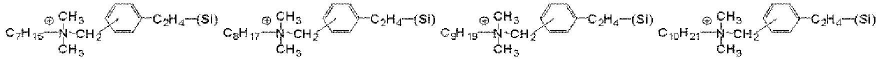

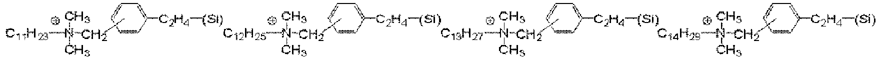

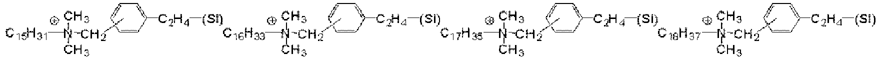

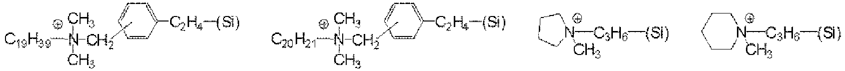

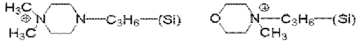

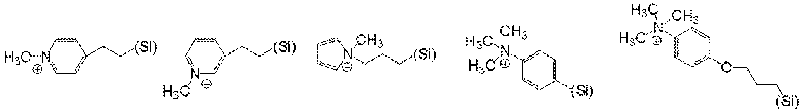

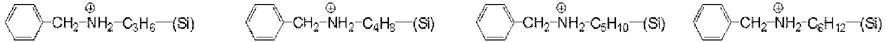

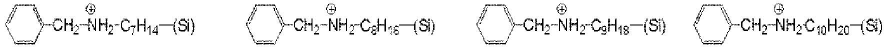

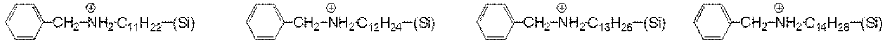

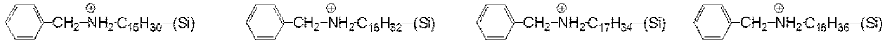

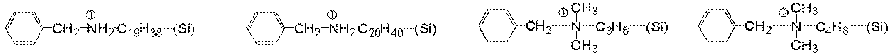

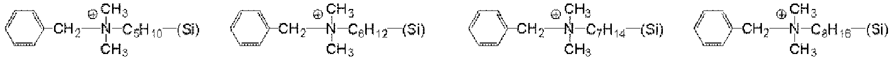

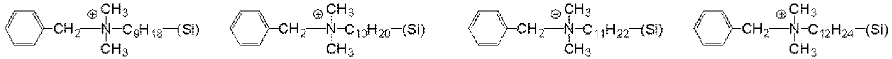

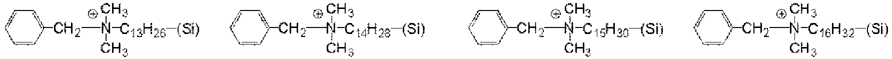

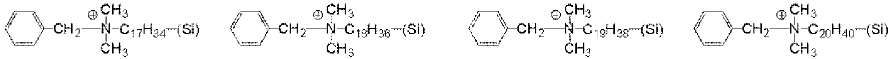

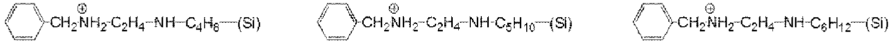

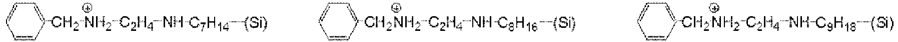

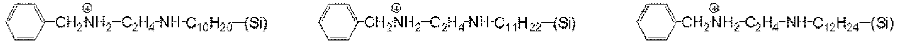

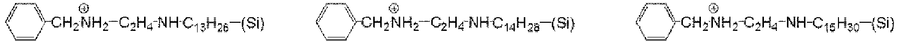

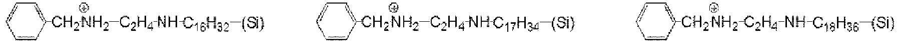

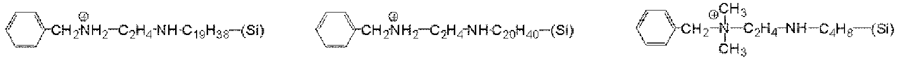

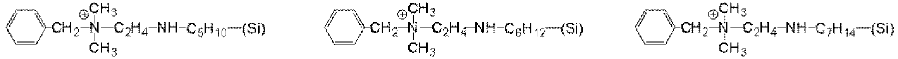

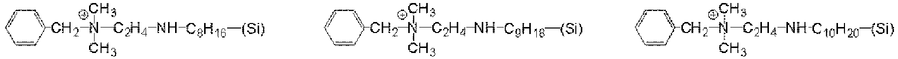

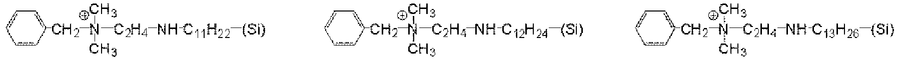

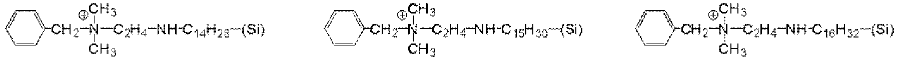

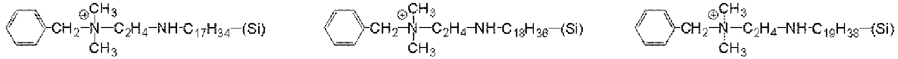

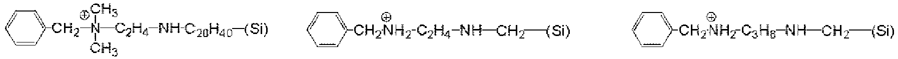

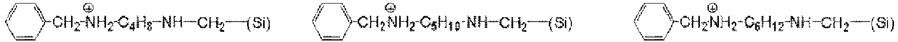

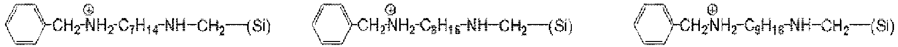

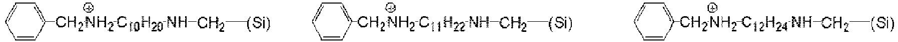

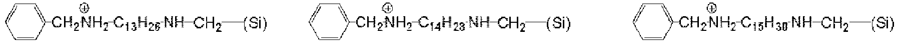

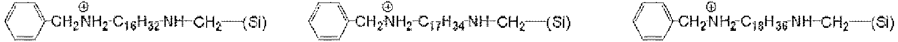

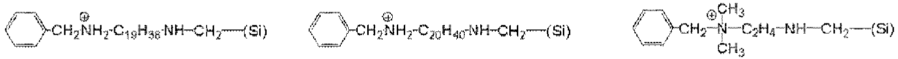

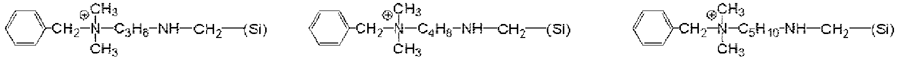

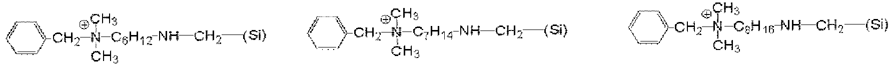

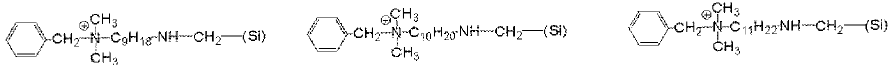

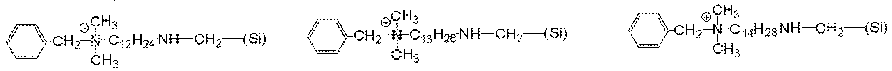

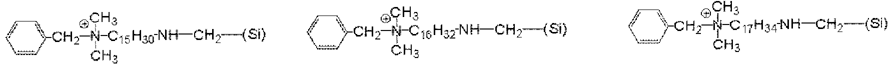

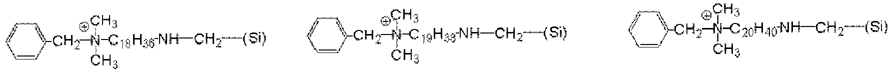

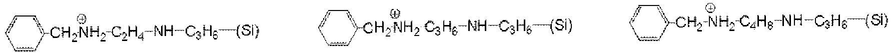

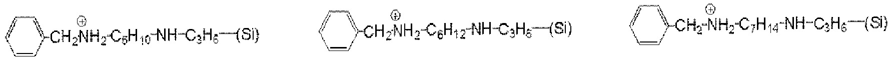

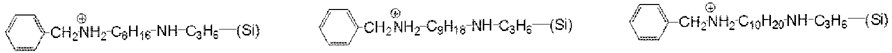

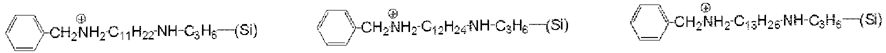

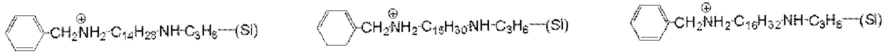

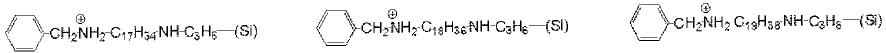

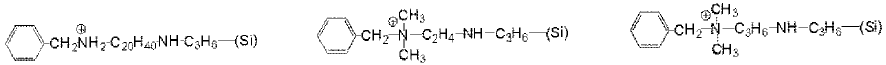

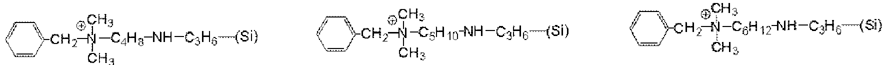

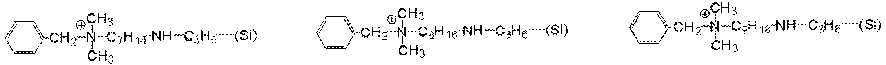

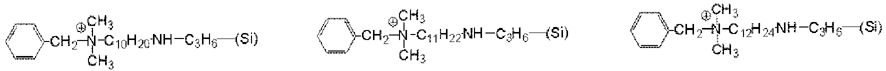

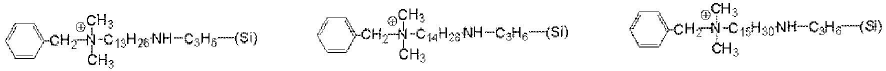

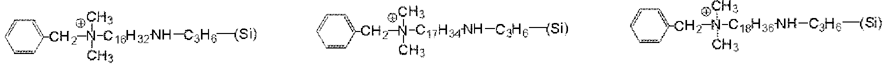

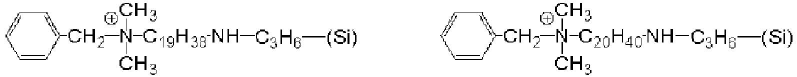

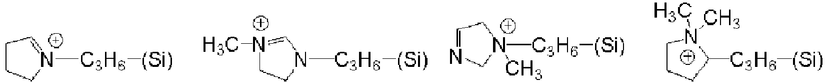

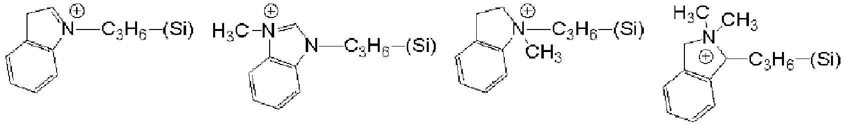

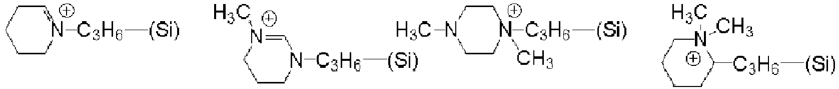

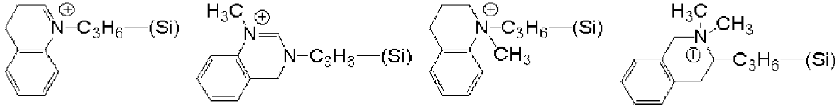

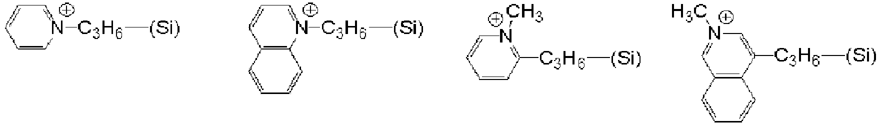

Examples of hydrolysable silicon compounds to be used with the above-described (Xm-1), (Xm-2), (Xm-3), and (Xm-4) for producing (Xc-10) include the hydrolysable monomer (Sm). Furthermore, the hydrolysable metal compound (Mm) represented by the general formula (Mm) may be added.

One or more kinds of the monomers (Xm-1), (Xm-2), (Xm-3), and (Xm-4) as shown above, one or more kinds of (Sm), and furthermore, if necessary, one or more kinds of (Mm) may be selected and mixed before the reaction or during the reaction to achieve the starting material for reaction for forming (Xc-10). The reaction conditions can be the same as the conditions for the method for synthesizing the thermally crosslinkable polysiloxane (Sx).

The molecular weight of the crosslinking catalyst (Xc-10) obtained can be adjusted not only through the selection of the monomers, but also through the control of the reaction condition during the polymerization. When the weight-average molecular weight is 100,000 or less, the generation of foreign matters or coating spots does not occur. Therefore, the crosslinking catalyst preferably has a weight-average molecular weight of 100,000 or less, more preferably 200 to 50,000, further preferably 300 to 30,000. Regarding data on the weight-average molecular weight, the molecular weight is expressed in terms of polystyrene which is obtained by gel permeation chromatography (GPC) using a refractive index (RI) detector, tetrahydrofuran as an eluent, and polystyrene as a reference substance.

Incidentally, one kind of the crosslinking catalysts (Xc-1), (Xc-2), (Xc-3), (Xc-4), and (Xc-10) may be used, or two or more kinds thereof may be used in combination. The amount of the crosslinking catalyst to be contained is preferably 0.01 to 50 parts by mass, more preferably 0.1 to 40 parts by mass based on 100 parts by mass of the base polymer (the thermally crosslinkable polysiloxane (Sx) obtained by a method described above).

### (Organic Acid)

To improve the stability of the inventive composition for forming a silicon-containing resist underlayer film, a monovalent, divalent, or polyvalent organic acid having 1 to 30 carbon atoms is preferably contained. Examples of the acid contained in this event include formic acid, acetic acid, propionic acid, butanoic acid, pentanoic acid, hexanoic acid, heptanoic acid, octanoic acid, nonanoic acid, decanoic acid, oleic acid, stearic acid, linoleic acid, linolenic acid, benzoic acid, phthalic acid, isophthalic acid, terephthalic acid, salicylic acid, trifluoroacetic acid, monochloroacetic acid, dichloroacetic acid, trichloroacetic acid, oxalic acid, malonic acid, methylmalonic acid, ethylmalonic acid, propylmalonic acid, butylmalonic acid, dimethylmalonic acid, diethylmalonic acid, succinic acid, methylsuccinic acid, glutaric acid, adipic acid, itaconic acid, maleic acid, fumaric acid, citraconic acid, citric acid, and the like. Particularly, oxalic acid, maleic acid, formic acid, acetic acid, propionic acid, citric acid, and the like are preferable. Moreover, a mixture of two or more acids may be used to keep the stability. The amount of the organic acid to be contained is preferably 0.001 to 25 parts by mass, more preferably 0.01 to 15 parts by mass, and further preferably 0.1 to 5 parts by mass, based on 100 parts by mass of the silicon contained in the inventive composition for forming a silicon-containing resist underlayer film.

Otherwise, the organic acid(s) may be contained based on the pH of the inventive composition for forming a silicon-containing resist underlayer film so as to satisfy preferably 0 ≤ pH ≤ 7, more preferably 0.3 ≤ pH ≤ 6.5, further preferably 0.5 ≤ pH ≤ 6.

### (Water)

In the present invention, water may be contained in the composition for forming a silicon-containing resist underlayer film. When water is contained, the polysiloxane compound in the inventive composition for forming a silicon-containing resist underlayer film is hydrated, so that the lithography performance is improved. The water content in the solvent component of the inventive composition for forming a silicon-containing resist underlayer film is preferably more than 0 mass% and less than 50 mass%, particularly preferably 0.3 to 30 mass%, further preferably 0.5 to 20 mass%. When the amount of water contained is less than 50 mass%, the uniformity of the silicon-containing resist underlayer film is excellent, and there is no risk of repellence occurring. Meanwhile, when the amount of water contained is more than 0 mass%, excellent lithography performance can be achieved.

The solvent including water is preferably used in a total amount of 100 to 100,000 parts by mass, particularly preferably 200 to 50,000 parts by mass, based on 100 parts by mass of the thermally crosslinkable polysiloxane, which is the base polymer.

### (Stabilizer)

Further, in the present invention, a stabilizer can be contained in the composition for forming a silicon-containing resist underlayer film. As the stabilizer, a monohydric, dihydric, or polyhydric alcohol having a cyclic ether substituent can be contained. Particularly, adding stabilizers shown in paragraphs [0181] and [0182] of JP2009-126940A enables stability improvement of the composition for forming a silicon-containing resist underlayer film. The amount of the stabilizer to be contained is preferably 0 to 50 parts by mass, more preferably 0 to 40 parts by mass, based on 100 parts by mass of the thermally crosslinkable polysiloxane (Sx), which is the base polymer.

### (Surfactant)

Further, in the present invention, a surfactant can be contained in the composition for forming a silicon-containing resist underlayer film as necessary. Specifically, the materials described in paragraph [0185] of JP2009-126940A can be contained as the surfactant. The amount of the surfactant to be contained is preferably 0 to 10 parts by mass, more preferably 0 to 5 parts by mass, based on 100 parts by mass of the thermally crosslinkable polysiloxane (Sx), which is the base polymer.

### (High-Boiling-Point Solvent)

Further, in the present invention, a high-boiling-point solvent having a boiling point of 180°C or higher can also be contained in the composition as necessary. Examples of the high-boiling-point solvent include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, gamma-butyrolactone, tripropylene glycol monomethyl ether, diacetone alcohol, n-nonyl acetate, ethylene glycol monoethyl ether acetate, 1,2-diacetoxyethane, 1-acetoxy-2-methoxyethane, 1,2-diacetoxypropane, diethylene glycol monomethyl ether acetate, diethylene glycol monoethyl ether acetate, diethylene glycol mono-n-butyl ether acetate, propylene glycol monomethyl ether acetate, propylene glycol monopropyl ether acetate, propylene glycol monobutyl ether acetate, dipropylene glycol monomethyl ether acetate, dipropylene glycol monoethyl ether acetate, and the like. The amount of the high-boiling-point solvent to be contained is preferably 0 to 20 mass%, more preferably 0 to 10 mass% of the solvent component.

### [Patterning Process]

A patterning process of the present invention is a patterning process (so-called "multilayer resist method") for forming a pattern in a body to be processed, including the steps of:
forming an organic film on a body to be processed by using a coating-type organic film material;
forming a silicon-containing resist underlayer film on the organic film by using the above-described composition for forming a silicon-containing resist underlayer film;
forming a resist upper layer film on the silicon-containing resist underlayer film by using a resist upper layer film composition containing a photoresist composition;
forming a circuit pattern in the resist upper layer film;
transferring the pattern to the silicon-containing resist underlayer film by etching while using the resist upper layer film having the formed circuit pattern as a mask;
transferring the pattern to the organic film by etching while using the silicon-containing resist underlayer film having the transferred pattern as a mask; and
transferring the pattern to the body to be processed by etching while using the organic film having the transferred pattern as a mask.

A patterning process of the present invention is a patterning process for forming a pattern in a body to be processed, including the steps of:
forming a hard mask on a body to be processed by a CVD method;
forming a silicon-containing resist underlayer film on the hard mask by using the above-described composition for forming a silicon-containing resist underlayer film;
forming a resist upper layer film on the silicon-containing resist underlayer film by using a resist upper layer film composition containing a photoresist composition;
forming a circuit pattern in the resist upper layer film;
transferring the pattern to the silicon-containing resist underlayer film by etching while using the resist upper layer film having the formed circuit pattern as a mask;
transferring the pattern to the hard mask by dry etching while using the silicon-containing resist underlayer film having the transferred pattern as a mask; and
transferring the pattern to the body to be processed by dry etching while using the hard mask having the transferred pattern as a mask.

A patterning process of the present invention is a patterning process for forming a pattern in a body to be processed, including the steps of:
forming a silicon-containing resist underlayer film on a body to be processed by using the above-described composition for forming a silicon-containing resist underlayer film;
forming a resist upper layer film on the silicon-containing resist underlayer film by using a resist upper layer film composition containing a photoresist composition;
forming a circuit pattern in the resist upper layer film;
transferring the pattern to the silicon-containing resist underlayer film by etching while using the resist upper layer film having the formed circuit pattern as a mask; and
transferring the pattern to the body to be processed by dry etching while using the silicon-containing resist underlayer film having the transferred pattern as a mask.

When the inventive polyvalent ammonium salt compound is contained in a composition for forming a silicon-containing resist underlayer film, an obtained pattern can be formed on a substrate as a fine pattern excellent in LWR and CDU by optimizing the combination with the hard mask or the organic film as described above. Furthermore, the silicon-containing resist underlayer film that remains after pattern formation can be removed easily by etching and so forth, and therefore, residues, which may cause defects, can be suppressed, and it is also possible to prevent substrate damage due to excessive etching conditions.

In a positive patterning process, the photoresist film (resist upper layer film) is formed and heat-treated, then exposed, and usually, subjected to alkaline development using an alkaline developer to obtain a positive resist pattern. Furthermore, post-exposure baking (PEB) is preferably performed after the exposure.

As the alkaline developer, an aqueous solution of tetramethylammonium hydroxide (TMAH) or the like can be used.

It is preferable that, in the patterning process, the circuit pattern is formed in the resist upper layer film by a photolithography with a wavelength of 10 nm or more and 300 nm or less, a direct drawing by electron beam, a nanoimprinting, or a combination thereof.

As the body to be processed, it is preferable to use a semiconductor device substrate or the semiconductor device substrate coated with any of a metal film, a metal carbide film, a metal oxide film, a metal nitride film, a metal oxycarbide film, and a metal oxynitride film.

As the metal, it is preferable to use silicon, gallium, titanium, tungsten, hafnium, zirconium, chromium, germanium, copper, silver, gold, indium, arsenic, palladium, tantalum, iridium, aluminum, iron, molybdenum, cobalt, or an alloy thereof.

### EXAMPLES

Hereinafter, the present invention will be specifically described with reference to Synthesis Examples, Comparative Synthesis Examples, Examples, and Comparative Examples, but the present invention is not limited to these Examples. Note that, in the following examples, "%" means "mass%", and the molecular weight Mw is the weight-average molecular weight in terms of polystyrene, measured by GPC using tetrahydrofuran as an eluent.

Me represents a methyl group, Et represents an ethyl group, Bu represents a butyl group, and Ph represents a phenyl group.

### [Synthesis Example 1-1]

To a mixture containing 120 g of methanol, 0.1 g of 10% nitric acid, and 60 g of deionized water, a mixture containing 30.6 g of a compound (101), 38.1 g of a compound (102), and 5.9 g of a compound (110) was added (molar ratio: 40/50/10), and hydrolysis condensation was performed for 12 hours while maintaining at 40°C. After completion of the reaction, 600 g of propylene glycol monoethyl ether (PGEE) was added thereto. The water used in the hydrolysis condensation and by-produced alcohol were distilled off under reduced pressure to obtain 440 g of a solution of a polysiloxane compound 1 in PGEE (compound concentration: 10%). The molecular weight of the polysiloxane compound 1 was measured in terms of polystyrene, and was Mw=2,900.

[Synthesis Example 1-2] to [Synthesis Example 1-18] were carried out under the same conditions as in [Synthesis Example 1-1] by using the monomers shown in Table 1 to obtain the target products as polysiloxane compounds 2 to 18.

**[Table 1]**

| | Charged amount (mol%) of each monomer | | | | | | | | | | | | | Mw |
|---|---|---|---|---|---|---|---|---|---|---|---|---|---|---|
| Synthesis Example | 100 | 101 | 102 | 110 | 111 | 112 | 113 | 114 | 115 | 116 | 117 | 118 | 119 | |
| 1 | - | 40 | 50 | 10 | - | - | - | - | - | - | - | - | - | 2900 |
| 2 | 5 | 45 | 50 | - | - | - | - | - | - | - | - | - | - | 2300 |
| 3 | 5 | 40 | 50 | 2 | 3 | - | - | - | - | - | - | - | - | 2900 |
| 4 | - | 45 | 50 | - | - | 3 | - | - | 2 | - | - | - | - | 2600 |
| 5 | 5 | 40 | 50 | - | - | - | 2 | - | 3 | - | - | - | - | 2300 |
| 6 | 5 | 40 | 50 | - | - | - | - | 5 | - | - | - | - | - | 2500 |
| 7 | 5 | 40 | 50 | - | - | - | 2 | - | - | 3 | - | - | - | 2600 |
| 8 | 5 | 40 | 50 | - | - | - | - | - | - | 2 | 3 | - | - | 2300 |
| 9 | 5 | 40 | 50 | - | - | 2 | - | - | - | - | - | 3 | - | 2300 |
| 10 | 5 | 40 | 50 | - | - | - | - | - | - | - | - | 5 | - | 2300 |
| 11 | 5 | 40 | 50 | - | - | - | - | - | - | - | - | - | 5 | 2500 |
| 12 | 5 | 40 | 50 | - | - | - | - | - | - | - | - | 2 | 3 | 2100 |
| 13 | 20 | 10 | 70 | - | - | - | - | - | - | - | - | - | - | 2800 |
| 14 | 10 | 10 | 70 | 10 | - | - | - | - | - | - | - | - | - | 2500 |
| 15 | 10 | 10 | 70 | 4 | 6 | - | - | - | - | - | - | - | - | 2700 |
| 16 | 10 | 10 | 70 | - | - | 6 | - | - | 4 | - | - | - | - | 2500 |
| 17 | 10 | 10 | 70 | - | - | - | 4 | - | 6 | - | - | - | - | 2700 |
| 18 | 10 | 10 | 70 | - | - | - | - | 10 | - | - | - | - | - | 2700 |

PhSi(OCH₃)₃··· compound (100)

CH₃Si(OCH₃)₃··· compound (101)

Si(OCH₃)₄··· compound (102)

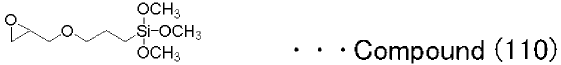

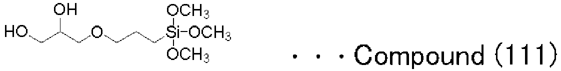

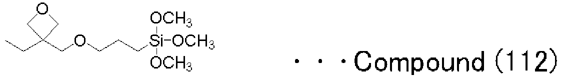

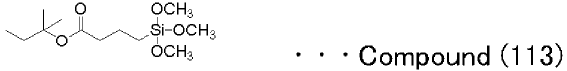

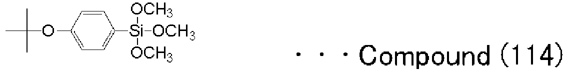

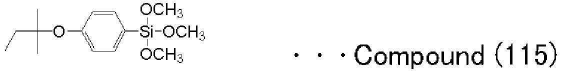

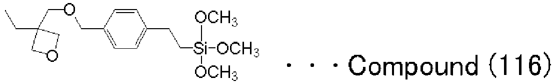

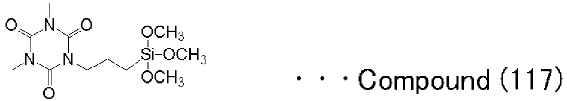

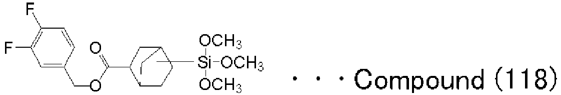

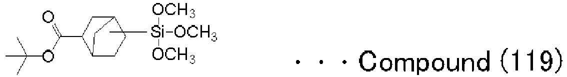

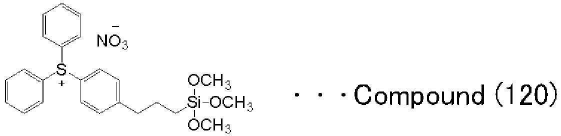

### [Synthesis Example of Crosslinking Catalyst]

### [Synthesis Example 2-1] Synthesis of Compound A'

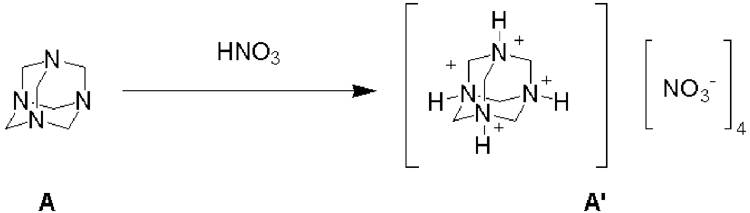

25 g of a compound A was dissolved in 40 g of water. This solution was added dropwise over 30 minutes to 650 g of a 7% aqueous solution of nitric acid cooled to 0°C. After aging at room temperature for 24 hours, the solution was dried under reduced pressure at 50°C to obtain 57 g of the target compound A' as a white solid.

### [Synthesis Example 2-2] Synthesis of Compound A"

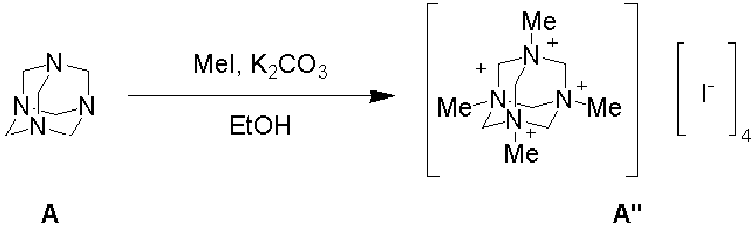

10 g of the compound A and 40.4 g of potassium carbonate were dissolved in 200 mL of ethanol, and 41 g of methyl iodide was added dropwise thereto at room temperature over 30 minutes. After aging at room temperature for 24 hours, the obtained reaction solution was filtered and dried under reduced pressure to obtain 8.7 g of the target compound A'' as a white solid.

[Synthesis Example 2-3] to [Synthesis Example 2-7] were carried out under the same conditions as in Synthesis Example 2-1 by using the starting materials shown in Table 2 to obtain the target products.

Meanwhile, [Synthesis Example 2-8] to [Synthesis Example 2-13] were carried out under the same conditions as in Synthesis Example 2-2 by using the starting materials and alkylating agents shown in Table 3 to obtain the target products.

**[Table 2]**

| Synthesis Example | Starting material for reaction | Target product |
|---|---|---|
| 2-3 | Compound (B) | Compound (B') |
| 2-4 | Compound (C) | Compound (C') |
| 2-5 | Compound (D) | Compound (D') |
| 2-6 | Compound (E) | Compound (E') |
| 2-7 | Compound (F) | Compound (F') |

**[Table 3]**

| Synthesis Example | Starting material for reaction and alkylating agent | Target product |
|---|---|---|
| 2-8 | Compound (A), nBuBr | Compound (A‴) |
| 2-9 | Compound (B), MeI | Compound (B") |
| 2-10 | Compound (C), MeI | Compound (C") |
| 2-11 | Compound (D), MeI | Compound (D") |
| 2-12 | Compound (E), MeI | Compound (E") |
| 2-13 | Compound (F), MeI | Compound (F") |

### [Starting Material for Reaction]

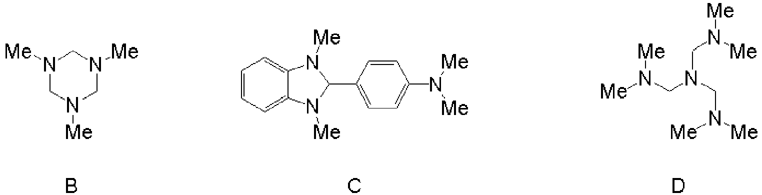

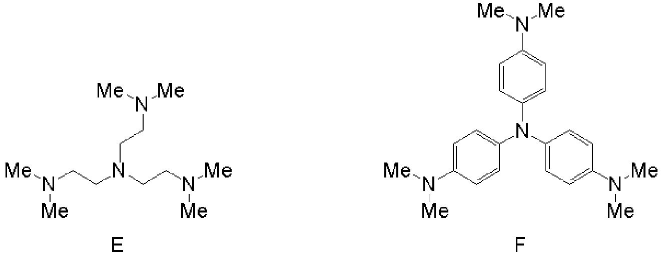

### [Target Product]

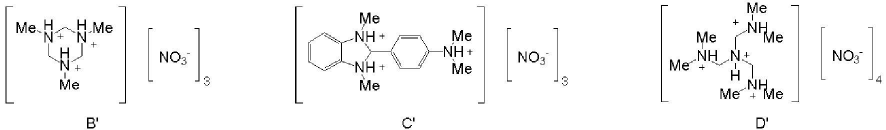

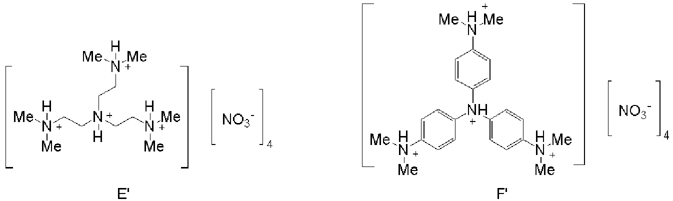

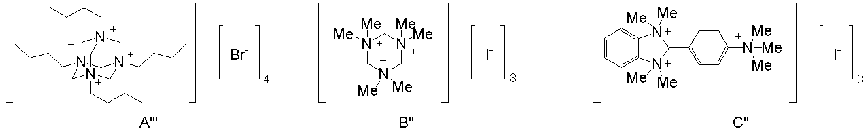

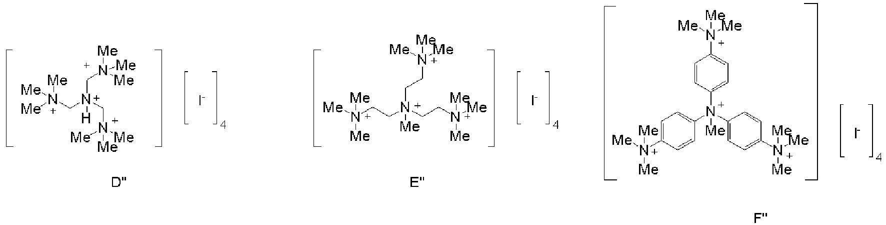

### [Comparative Synthesis Example 1-1] Synthesis of Sulfonium-Salt-Containing Polysiloxane Compound R

To a mixture of 120 g of methanol, 0.1 g of 10% nitric acid, and 60 g of deionized water, a mixture of 61.3 g of the compound (101) and 24.4 g of the compound (120) was added and maintained at 40°C for 12 hours to perform hydrolysis condensation. After completion of the reaction, 300 g of propylene glycol monoethyl ether (PGEE) was added thereto, and the water used for the hydrolysis condensation and by-produced alcohol were distilled off under reduced pressure to obtain 250 g of a solution of a sulfonium-salt-containing polysiloxane compound R in PGEE (compound concentration: 20%). The molecular weight of the polysiloxane compound R was measured in terms of polystyrene, and was Mw=2,000.

### [Examples 1-1 to 1-58 and Comparative Examples 1-1 to 1-4]

One of the polysiloxane compounds 1 to 18 obtained in the Synthesis Examples, a polyvalent ammonium salt compound represented by the general formula (A-1) or a comparative crosslinking catalyst as a crosslinking catalyst, an acid, a photo-acid generator (PAG-1 to -7 shown in Table 7), a solvent, and water were mixed at proportions shown in Tables 4 to 6. Each mixture was filtered through a 0.1-µm filter made of fluorinated resin. Thus, composition solutions for forming a silicon-containing underlayer film were prepared and referred to as Sol. 1 to 62.

**[Table 4]**

| No. | Polysiloxane (parts by mass) | Crosslinking catalyst (parts by mass) | Photo-acid generator (parts by mass) | Acid (parts by mass) | Solvent (parts by mass) | Water (parts by mass) |
|---|---|---|---|---|---|---|
| Sol. 1 | Compound 1 (1) | XL-1 (0.03) | None | Maleic acid (0.01) | PGEE (100) | Water (10) |
| Sol. 2 | Compound 1 (1) | XL-2 (0.03) | None | Maleic acid (0.01) | PGEE (100) | Water (10) |
| Sol. 3 | Compound 2 (1) | XL-3 (0.03) | None | Maleic acid (0.01) | PGEE (100) | Water (10) |
| Sol. 4 | Compound 2 (1) | XL-4 (0.03) | None | Maleic acid (0.01) | PGEE (100) | Water (10) |
| Sol. 5 | Compound 3 (1) | XL-5 (0.03) | None | Maleic acid (0.01) | PGEE (100) | Water (10) |
| Sol. 6 | Compound 3 (1) | XL-6 (0.03) | None | Maleic acid (0.01) | PGEE (100) | Water (10) |
| Sol. 7 | Compound 4 (1) | XL-7 (0.03) | None | Maleic acid (0.01) | PGEE (100) | Water (10) |
| Sol. 8 | Compound 4 (1) | XL-8 (0.03) | None | Maleic acid (0.01) | PGEE (100) | Water (10) |
| Sol. 9 | Compound 5 (1) | XL-9 (0.03) | None | Maleic acid (0.01) | PGEE (100) | Water (10) |
| Sol. 10 | Compound 5 (1) | XL-10 (0.03) | None | Maleic acid (0.01) | PGEE (100) | Water (10) |
| Sol. 11 | Compound 6 (1) | XL-11 (0.03) | None | Maleic acid (0.01) | PGEE (100) | Water (10) |
| Sol. 12 | Compound 6 (1) | XL-12 (0.03) | None | Maleic acid (0.01) | PGEE (100) | Water (10) |
| Sol. 13 | Compound 7 (1) | XL-13 (0.03) | None | Maleic acid (0.01) | PGEE (100) | Water (10) |
| Sol. 14 | Compound 7 (1) | XL-1 (0.03) | None | Maleic acid (0.01) | PGEE (100) | Water (10) |
| Sol. 15 | Compound 8 (1) | XL-2 (0.03) | None | Maleic acid (0.01) | PGEE (100) | Water (10) |
| Sol. 16 | Compound 8 (1) | XL-3 (0.03) | None | Maleic acid (0.01) | PGEE (100) | Water (10) |
| Sol. 17 | Compound 9 (1) | XL-4 (0.03) | None | Maleic acid (0.01) | PGEE (100) | Water (10) |
| Sol. 18 | Compound 9 (1) | XL-5 (0.03) | None | Maleic acid (0.01) | PGEE (100) | Water (10) |

**[Table 5]**

| No. | Polysiloxane (parts by mass) | Crosslinking catalyst (parts by mass) | Photo-acid generator (parts by mass) | Acid (parts by mass) | Solvent (parts by mass) | Water (parts by mass) |
|---|---|---|---|---|---|---|
| Sol. 19 | Compound 10 (1) | XL-6 (0.03) | None | Maleic acid (0.01) | PGEE (100) | Water (10) |
| Sol. 20 | Compound 11 (1) | XL-7 (0.03) | None | Maleic acid (0.01) | PGEE (100) | Water (10) |
| Sol. 21 | Compound 12 (1) | XL-8 (0.03) | None | Maleic acid (0.01) | PGEE (100) | Water (10) |
| Sol. 22 | Compound 13 (1) | XL-9 (0.03) | None | Maleic acid (0.01) | PGEE (100) | Water (10) |
| Sol. 23 | Compound 14 (1) | XL-10 (0.03) | None | Maleic acid (0.01) | PGEE (100) | Water (10) |
| Sol. 24 | Compound 15 (1) | XL-10 (0.03) | None | Maleic acid (0.01) | PGEE (100) | Water (10) |
| Sol. 25 | Compound 16 (1) | XL-11 (0.03) | None | Maleic acid (0.01) | PGEE (100) | Water (10) |
| Sol. 26 | Compound 17 (1) | XL-12 (0.03) | None | Maleic acid (0.01) | PGEE (100) | Water (10) |
| Sol. 27 | Compound 18 (1) | XL-13 (0.03) | None | Maleic acid (0.01) | PGEE (100) | Water (10) |
| Sol. 28 | Compound 1 (1) | XL-1 (0.03) | PAG-1 (0.01) | Maleic acid (0.01) | PGEE (100) | Water (10) |
| Sol. 29 | Compound 2 (1) | XL-2 (0.03) | PAG-2 (0.01) | Maleic acid (0.01) | PGEE (100) | Water (10) |
| Sol. 30 | Compound 3 (1) | XL-3 (0.03) | PAG-2 (0.01) | Maleic acid (0.01) | PGEE (100) | Water (10) |
| Sol. 31 | Compound 4 (1) | XL-4 (0.03) | PAG-3 (0.01) | Maleic acid (0.01) | PGEE (100) | Water (10) |
| Sol. 32 | Compound 5 (1) | XL-5 (0.03) | PAG-3 (0.01) | Maleic acid (0.01) | PGEE (100) | Water (10) |
| Sol. 33 | Compound 6 (1) | XL-6 (0.03) | PAG-4 (0.01) | Maleic acid (0.01) | PGEE (100) | Water (10) |
| Sol. 34 | Compound 7 (1) | XL-7 (0.03) | PAG-4 (0.01) | Maleic acid (0.01) | PGEE (100) | Water (10) |
| Sol. 35 | Compound 8 (1) | XL-8 (0.03) | PAG-5 (0.01) | Maleic acid (0.01) | PGEE (100) | Water (10) |
| Sol. 36 | Compound 9 (1) | XL-9 (0.03) | PAG-5 (0.01) | Maleic acid (0.01) | PGEE (100) | Water (10) |
| Sol. 37 | Compound 10 (1) | XL-10 (0.03) | PAG-6 (0.01) | Maleic acid (0.01) | PGEE (100) | Water (10) |
| Sol. 38 | Compound 11 (1) | XL-11 (0.03) | PAG-6 (0.01) | Maleic acid (0.01) | PGEE (100) | Water (10) |
| Sol. 39 | Compound 12 (1) | XL-12 (0.03) | PAG-7 (0.01) | Maleic acid (0.01) | PGEE (100) | Water (10) |
| Sol. 40 | Compound 13 (1) | XL-13 (0.03) | PAG-7 (0.01) | Maleic acid (0.01) | PGEE (100) | Water (10) |
| Sol. 41 | Compound 14 (1) | XL-1 (0.03) | PAG-7 (0.01) | Maleic acid (0.01) | PGEE (90) | Water (10) |
| | | | | | GBL (10) | |
| Sol. 42 | Compound 15 (1) | XL-2 (0.03) | PAG-3 (0.01) | Maleic acid (0.01) | PGEE (90) | Water (10) |
| | | | | | DAA (10) | |
| Sol. 43 | Compound 16 (1) | XL-3 (0.03) | None | Maleic acid (0.01) | PGEE (90) | Water (10) |
| | | | | | PGME (10) | |
| Sol. 44 | Compound 17 (1) | XL-4 (0.03) | PAG-2 (0.01) | Maleic acid (0.01) | PGEE (90) | Water (10) |
| | | | | | GBL (10) | |
| Sol. 45 | Compound 18 (1) | XL-5 (0.03) | None | Maleic acid (0.01) | PGEE (90) | Water (10) |
| | | | | | DAA (10) | |

**[Table 6]**

| No. | Polysiloxane (parts by mass) | Crosslinking catalyst (parts by mass) | Photo-acid generator (parts by mass) | Acid (parts by mass) | Solvent (parts by mass) | Water (parts by mass) |
|---|---|---|---|---|---|---|
| Sol. 46 | Compound 1 (1) | XL-6 (0.03) | None | Maleic acid (0.01) | PGEE (90) | Water |
| | | | | | PGME (10) | (10) |
| Sol. 47 | Compound 2 (1) | XL-7 (0.03) | PAG-6 (0.01) | Maleic acid (0.01) | PGEE (90) | Water |
| | | | | | GBL (10) | (10) |
| Sol. 48 | Compound 3 (1) | XL-8 (0.03) | None | Maleic acid (0.01) | PGEE (90) | Water |
| | | | | | DAA (10) | (10) |
| Sol. 49 | Compound 4 (1) | XL-9 (0.03) | PAG-3 (0.01) | Maleic acid (0.01) | PGEE (90) | Water |
| | | | | | PGME (10) | (10) |
| Sol. 50 | Compound 5 (1) | XL-10 (0.03) | None | Maleic acid (0.01) | PGEE (90) | Water |
| | | | | | PGME (10) | (10) |
| Sol. 51 | Compound 6 (1) | XL-11 (0.03) | PAG-4 (0.01) | Maleic acid (0.01) | PGEE (90) | Water |
| | | | | | GBL (10) | (10) |
| Sol. 52 | Compound 7 (1) | XL-12 (0.03) | None | Maleic acid (0.01) | PGEE (90) | Water |
| | | | | | DAA (10) | (10) |
| Sol. 53 | Compound 5 (1) | XL-1 (0.015) | None | Maleic acid (0.01) | PGEE (100) | Water (10) |
| | | XL-2 (0.015) | | | | |
| Sol. 54 | Compound 6 (1) | XL-1 (0.015) | None | Maleic acid (0.01) | PGEE (100) | Water (10) |
| | | XL-4 (0.015) | | | | |
| Sol. 55 | Compound 7 (1) | XL-1 (0.015) | None | Maleic acid (0.01) | PGEE (100) | Water (10) |
| | | XL-10 (0.015) | | | | |
| Sol. 56 | Compound 8 (1) | XL-2 (0.015) | None | Maleic acid (0.01) | PGEE (100) | Water (10) |
| | | XL-12 (0.015) | | | | |
| Sol. 57 | Compound 9 (1) | XL-1 (0.015) | None | Maleic acid (0.01) | PGEE (100) | Water (10) |
| | | XL-14 (0.015) | | | | |
| Sol. 58 | Compound 9 (1) | XL-4 (0.015) | None | Maleic acid (0.01) | PGEE (100) | Water (10) |
| | | XL-14 (0.015) | | | | |
| Sol. 59 | Compound 1 (1) | XL-14 (0.03) | None | Maleic acid (0.01) | PGEE (100) | Water (10) |
| Sol. 60 | Compound 2 (1) | XL-15 (0.03) | None | Maleic acid (0.01) | PGEE (100) | Water (10) |
| Sol. 61 | Compound 3 (1) | XL-16 (0.03) | None | Maleic acid (0.01) | PGEE (100) | Water (10) |
| Sol. 62 | Compound 4 (1) | XL-17 (0.03) | None | Maleic acid (0.01) | PGEE (100) | Water (10) |

The crosslinking catalysts used are as follows.

XL-1··· compound (A')

XL-2··· compound (A")

XL-3··· compound (B')

XL-4··· compound (C')

XL-5··· compound (D')

XL-6··· compound (E')

XL-7··· compound (F')

XL-8··· compound (A‴)

XL-9··· compound (B")

XL-10··· compound (C")

XL-11··· compound (D")

XL-12··· compound (E")

XL-13··· compound (F")

XL-14···triphenylsulfonium nitrate
XL-15···tetramethylammonium nitrate
XL-16···tetraoctylammonium nitrate
XL-17···sulfonium-salt-containing polysiloxane compound
(R)

The solvents used are as follows.
PGEE···propylene glycol monoethyl ether
PGME···propylene glycol monomethyl ether
GBL···gamma-butyrolactone
DAA···diacetone alcohol

**[Table 7]**

| PAG | | PAG | |
|---|---|---|---|
| 1 | 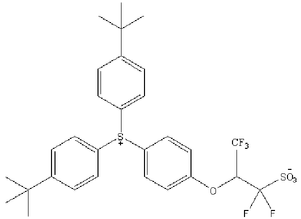 | 5 | 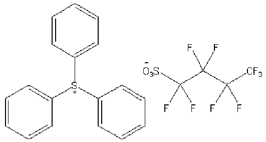 |
| 2 | 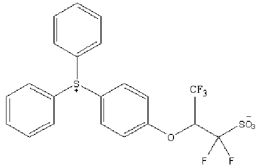 | 6 | 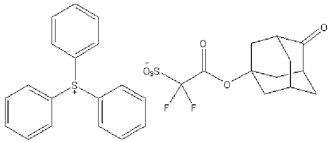 |
| 3 | 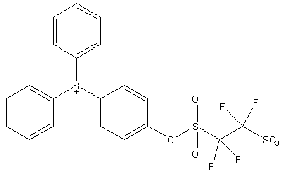 | 7 | 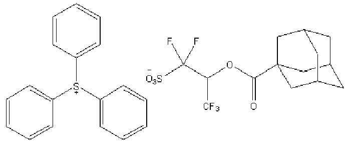 |
| 4 | 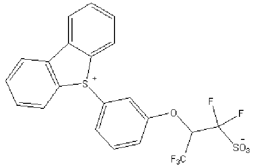 | | |

### (EUV Patterning Test)

Each of the compositions Sol. 1 to 62 for forming a silicon-containing resist underlayer film was respectively applied onto a silicon wafer by spin-coating and heated at 220°C for 60 seconds to prepare 25-nm thick silicon-containing resist underlayer films: Films 1 to 62.

Subsequently, a resist material obtained by dissolving the components shown below in the proportions of Table 8 was applied to the Films 1 to 62 by spin-coating and prebaked using a hot plate at 105°C for 60 seconds to prepare a resist upper layer film having a film thickness of 60 nm. The resist upper layer film was exposed using an EUV scanner NXE3300 (manufactured by ASML, NA: 0.33, σ: 0.9/0.6, quadrupole illumination, with a mask having a hole pattern with a pitch of 46 nm and +20% bias (on-wafer size)), followed by PEB on a hot plate at 100°C for 60 seconds and development with a 2.38 mass% aqueous solution of TMAH for 30 seconds to obtain a hole pattern with a size of 23 nm.

Using CD-SEM (CG5000) manufactured by Hitachi High-Technologies Corp., an exposure dose at which the hole dimension of 23 nm was formed was measured and determined as sensitivity. In addition, the dimensions of 50 holes in this event were measured, and dimensional variation (CDU, 3σ) was calculated. The results are shown in Tables 9 and 10.

The polymer, quencher, sensitizer, surfactant, and organic solvent used for the resist material are as follows. 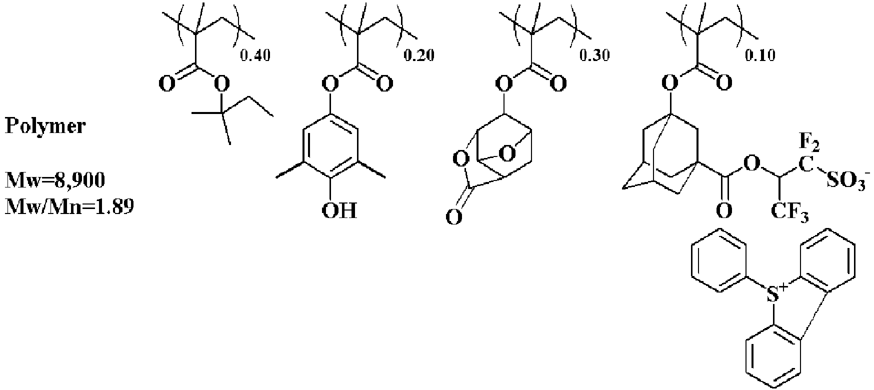 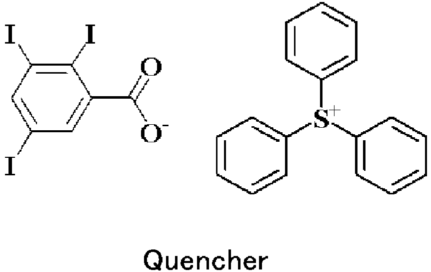 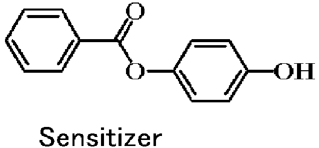
Surfactant: FC-4430 available from 3M
PGMEA: propylene glycol monomethyl ether acetate
CyHO: cyclohexanone
PGME: propylene glycol monomethyl ether

**[Table 8]**

| Component | Polymer | Quencher | Sensitizer | Surfactant | Organic solvent |
|---|---|---|---|---|---|
| Composition (parts by mass) | (100) | (4.0) | (2.1) | (0.25) | PGMEA (400) |
| | | | | | CyHO (2000) |
| | | | | | PGME (100) |

### (Etching Test of Silicon-Containing Resist Underlayer Film)

Each of compositions Sol. 1 to 62 for forming a silicon-containing resist underlayer film was respectively applied onto a silicon wafer by spin-coating and heated at 220°C for 60 seconds to prepare 25-nm thick silicon-containing resist underlayer films: Films 1 to 62.

These silicon-containing resist underlayer films were subjected to an etching test under the following etching conditions.

### Etching Test with CHF₃/CF₄-Based Gas

Apparatus: dry etching apparatus Telius SP manufactured by Tokyo Electron Limited
Etching test:

| | |
|---|---|
| Chamber pressure | 10 Pa |
| Upper/Lower RF power | 200 W/100 W |
| CHF₃ gas flow rate | 50 ml/min |
| CF₄ gas flow rate | 50 ml/min |
| N₂ gas flow rate | 100 ml/min |
| Treatment time | 20 sec |

**[Table 9]**

| | Silicon-containing resist underlayer film | Sensitivity (mJ/cm²) | CDU (nm) | Dry etching rate (nm/min) with CHF₃/CF₄-based gas |
|---|---|---|---|---|
| Example 1-1 | Film 1 | 28 | 2.2 | 50.1 |
| Example 1-2 | Film 2 | 28 | 2.4 | 50.7 |
| Example 1-3 | Film 3 | 30 | 2.2 | 48.9 |
| Example 1-4 | Film 4 | 28 | 2.2 | 51.3 |
| Example 1-5 | Film 5 | 29 | 2.4 | 48.7 |
| Example 1-6 | Film 6 | 30 | 2.3 | 50.8 |
| Example 1-7 | Film 7 | 28 | 2.2 | 51.4 |
| Example 1-8 | Film 8 | 29 | 2.4 | 50.0 |
| Example 1-9 | Film 9 | 30 | 2.2 | 48.9 |
| Example 1-10 | Film 10 | 30 | 2.2 | 48.5 |
| Example 1-11 | Film 11 | 30 | 2.3 | 50.2 |
| Example 1-12 | Film 12 | 29 | 2.2 | 49.2 |
| Example 1-13 | Film 13 | 28 | 2.4 | 48.6 |
| Example 1-14 | Film 14 | 30 | 2.2 | 48.6 |
| Example 1-15 | Film 15 | 29 | 2.2 | 50.1 |
| Example 1-16 | Film 16 | 30 | 2.2 | 49.3 |
| Example 1-17 | Film 17 | 29 | 2.4 | 48.6 |
| Example 1-18 | Film 18 | 29 | 2.2 | 50.3 |
| Example 1-19 | Film 19 | 30 | 2.3 | 51.1 |
| Example 1-20 | Film 20 | 29 | 2.2 | 48.8 |
| Example 1-21 | Film 21 | 28 | 2.2 | 49.0 |
| Example 1-22 | Film 22 | 28 | 2.2 | 49.1 |
| Example 1-23 | Film 23 | 28 | 2.4 | 49.8 |
| Example 1-24 | Film 24 | 30 | 2.2 | 48.8 |
| Example 1-25 | Film 25 | 29 | 2.2 | 49.9 |
| Example 1-26 | Film 26 | 29 | 2.4 | 50.0 |
| Example 1-27 | Film 27 | 29 | 2.2 | 50.7 |
| Example 1-28 | Film 28 | 27 | 2.3 | 48.9 |
| Example 1-29 | Film 29 | 29 | 2.3 | 49.9 |
| Example 1-30 | Film 30 | 28 | 2.3 | 51.4 |
| Example 1-31 | Film 31 | 27 | 2.3 | 49.4 |
| Example 1-32 | Film 32 | 29 | 2.4 | 50.6 |
| Example 1-33 | Film 33 | 29 | 2.2 | 51.3 |
| Example 1-34 | Film 34 | 27 | 2.2 | 51.3 |
| Example 1-35 | Film 35 | 28 | 2.2 | 50.1 |
| Example 1-36 | Film 36 | 27 | 2.4 | 49.6 |

**[Table 10]**

| | Silicon-containing resist underlayer film | Sensitivity (mJ/cm²) | CDU (nm) | Dry etching rate (nm/min) with CHF₃/CF₄-based gas |
|---|---|---|---|---|
| Example 1-37 | Film 37 | 29 | 2.4 | 49.4 |
| Example 1-38 | Film 38 | 29 | 2.2 | 51.5 |
| Example 1-39 | Film 39 | 28 | 2.2 | 49.9 |
| Example 1-40 | Film 40 | 27 | 2.4 | 50.0 |
| Example 1-41 | Film 41 | 29 | 2.2 | 50.9 |
| Example 1-42 | Film 42 | 28 | 2.4 | 50.0 |
| Example 1-43 | Film 43 | 29 | 2.2 | 49.5 |
| Example 1-44 | Film 44 | 28 | 2.4 | 49.1 |
| Example 1-45 | Film 45 | 28 | 2.4 | 50.2 |
| Example 1-46 | Film 46 | 27 | 2.4 | 49.0 |
| Example 1-47 | Film 47 | 29 | 2.4 | 50.7 |
| Example 1-48 | Film 48 | 30 | 2.2 | 51.4 |
| Example 1-49 | Film 49 | 28 | 2.2 | 51.1 |
| Example 1-50 | Film 50 | 28 | 2.3 | 51.5 |
| Example 1-51 | Film 51 | 28 | 2.4 | 51.2 |
| Example 1-52 | Film 52 | 29 | 2.4 | 51.3 |
| Example 1-53 | Film 53 | 30 | 2.2 | 50.4 |
| Example 1-54 | Film 54 | 30 | 2.2 | 48.6 |
| Example 1-55 | Film 55 | 30 | 2.2 | 48.7 |
| Example 1-56 | Film 56 | 28 | 2.4 | 49.9 |
| Example 1-57 | Film 57 | 30 | 2.3 | 50.4 |
| Example 1-58 | Film 58 | 30 | 2.2 | 50.2 |
| Comparative Example 1-1 | Film 59 | 29 | 3.1 | 47.0 |
| Comparative Example 1-2 | Film 60 | 29 | 3.1 | 47.0 |
| Comparative Example 1-3 | Film 61 | 28 | 3 | 47.1 |
| Comparative Example 1-4 | Film 62 | 29 | 3.1 | 49.4 |

As shown in Comparative Examples 1-1 to 1-4 of Tables 9 and 10, it was observed that regarding Films 59 to 62, in which a polyvalent ammonium salt compound represented by the general formula (A-1) of the present invention was not used as a crosslinking catalyst, CDU was degraded or the Films did not have a sufficient etching rate. On the other hand, as shown in Examples 1-1 to 1-58, it was found that regarding Films 1 to 58, in which a polyvalent ammonium salt compound represented by the general formula (A-1) of the present invention was used as a crosslinking catalyst, the reactivity of the silicon polymer was enhanced by the crosslinking catalyst being polyvalent, and therefore, a strong film in which diffusion to the upper layer resist was suppressed was successfully formed, and the Films had a CDU-improving effect and a sufficient etching rate.

### [Examples 2-1 to 2-15 and Comparative Examples 2-1 to 2-4]

### (ArF Patterning Test)

On a silicon wafer, a spin-on carbon film ODL-102 (carbon content: 89 mass%) available from Shin-Etsu Chemical Co., Ltd. was formed with a film thickness of 200 nm. Each of the compositions Sol. 1 to 5, 11 to 13, 28 to 30, 51 to 54, and 59 to 62 for forming a silicon-containing resist underlayer film was respectively applied to the spin-on carbon film and heated at 220°C for 60 seconds to prepare 20-nm thick silicon-containing resist underlayer films: Films 1 to 5, 11 to 13, 28 to 30, 51 to 54, and 59 to 62.

Subsequently, the ArF resist solution (PR-1) for positive development shown in Table 11 was applied to the silicon-containing resist underlayer film and baked at 110°C for 60 seconds to form a 100-nm thick photoresist film. Furthermore, the liquid immersion top coat material (TC-1) shown in Table 12 was applied to the photoresist film and baked at 90°C for 60 seconds to form a 50-nm thick top coat.

Then, the wafer was exposed to light with an ArF liquid immersion exposure apparatus (XT-1900i manufactured by ASML, NA: 1.35, σ: 0.97/0.77, 35° polarized dipole illumination), baked at 100°C for 60 seconds (PEB), and developed with a 2.38% by mass aqueous solution of tetramethylammonium hydroxide (TMAH) for 30 seconds, thereby obtaining a 40 nm 1:1 positive line-and-space pattern. Regarding the dimensions of the pattern, the LWR was observed with an electron microscope (CG5000) manufactured by Hitachi High-Technologies Corporation. The results are shown in Table 13.

**[Table 11]**

| No. | Polymer (parts by mass) | Acid generator (parts by mass) | Base (parts by mass) | Surfactant (parts by mass) | Solvent (parts by mass) |
|---|---|---|---|---|---|
| PR-1 | ArF resist polymer 1 (100) | PAG-A (7.0) | Quencher (1.0) | FC-4430 (0.2) | PGMEA (2,500) |

ArF resist polymer 1 [molecular weight (Mw)=7,800, dispersity (Mw/Mn)=1.78]:

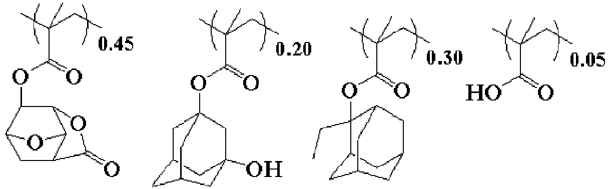

Acid generator: PAG-A

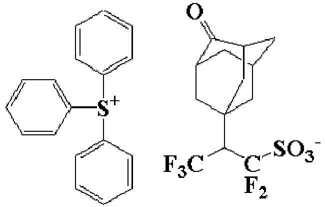

Base: Quencher

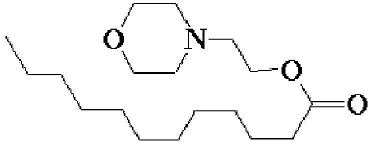

Surfactant: FC-4430 available from 3M

Top coat polymer [molecular weight (Mw)=8,800, dispersity (Mw/Mn)=1.69]

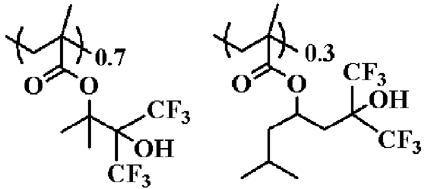

**[Table 12]**

| | Polymer (parts by mass) | Organic solvent (parts by mass) |
|---|---|---|
| TC-1 | Top coat polymer (100) | Diisoamyl ether (2700) |
| | | 2-methyl-1-butanol (270) |

**[Table 13]**

| | Silicon-containing resist underlayer film | LWR |
|---|---|---|
| Example 2-1 | Film 1 | 2.0 |
| Example 2-2 | Film 2 | 2.1 |
| Example 2-3 | Film 3 | 2.1 |
| Example 2-4 | Film 4 | 2.0 |
| Example 2-5 | Film 5 | 2.0 |
| Example 2-6 | Film 11 | 2.2 |
| Example 2-7 | Film 12 | 2.0 |
| Example 2-8 | Film 13 | 2.1 |
| Example 2-9 | Film 28 | 2.2 |
| Example 2-10 | Film 29 | 2.0 |
| Example 2-11 | Film 30 | 2.1 |
| Example 2-12 | Film 51 | 2.2 |
| Example 2-13 | Film 52 | 2.1 |
| Example 2-14 | Film 53 | 2.0 |
| Example 2-15 | Film 54 | 2.2 |
| Comparative Example 2-1 | Film 59 | 3.5 |
| Comparative Example 2-2 | Film 60 | 3.6 |
| Comparative Example 2-3 | Film 61 | 3.2 |
| Comparative Example 2-4 | Film 62 | 3.4 |

As shown in Examples 2-1 to 2-15 of Table 13, it was observed that regarding Films 1 to 5, 11 to 13, 28 to 30, and 51 to 54, in which a polyvalent ammonium salt compound represented by the general formula (A-1) of the present invention was used as a crosslinking catalyst, a strong film was formed by the advantageous effects of the crosslinking catalyst having high reactivity, and therefore, diffusion to the upper layer resist was suppressed and an LWR-improving effect was observed. On the other hand, as shown in Comparative Examples 2-1 to 2-4, regarding Films 59 to 62, in which the inventive polyvalent ammonium salt compound was not used, LWR was poor.

The present description includes the following embodiments.
[1]: A polyvalent ammonium salt compound having two or more ammonium ions per molecule, the polyvalent ammonium salt compound being represented by the following general formula (A-1), 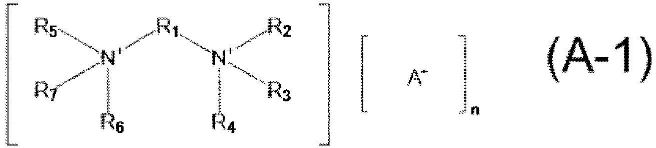 wherein R₁ represents a hydrocarbon group having 1 to 20 carbon atoms and optionally having a substituent or an aromatic group having 6 to 20 carbon atoms and optionally having a substituent, and optionally contains one or more nitrogen atoms; R₂, R₃, R₄, R₅, R₆, and R₇ each independently represent a hydrogen atom, a hydrocarbon group having 1 to 20 carbon atoms and optionally having a substituent, or an aromatic group having 6 to 20 carbon atoms and optionally having a substituent, and optionally contain one or more nitrogen atoms; any two or more selected from R₁, R₂, R₃, R₄, R₅, R₆, and R₇ are optionally bonded to each other to form a ring together with the nitrogen atom in the formula; A⁻ represents an organic or inorganic anion to be a counter ion of an ammonium cation, and excludes bistrifluoromethanesulfonylimide; and "n" represents an integer of 2 to 10.
[2]: The polyvalent ammonium salt compound of [1], wherein the A⁻ is formate, acetate, propionate, butyrate, hexanoate, benzoate, t-butylbenzoate, trichloroacetate, trifluoroacetate, 2-hydroxy-2,2-bis(trifluoromethyl)acetate, trimethylacetate, pentafluoropropionate, methanesulfonate, butanesulfonate, benzenesulfonate, toluenesulfonate, trifluoromethanesulfonate, methyl sulfate ion, chloride ion, bromide ion, iodide ion, fluoride ion, cyanide ion, nitrate ion, nitrite ion, or hydroxide ion.
[3]: The polyvalent ammonium salt compound of [1] or [2], represented by the following general formula (B-1), the following general formula (B-2), the following general formula (B-3), or the following general formula (B-4), 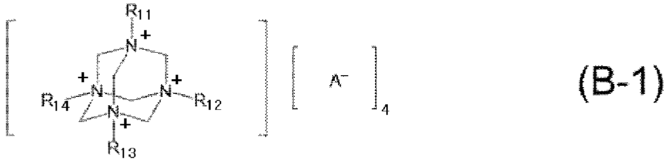 wherein R₁₁, R₁₂, R₁₃, and R₁₄ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 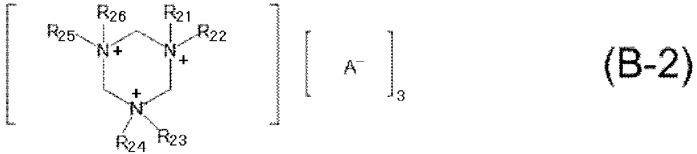 wherein R₂₁, R₂₂, R₂₃, R₂₄, R₂₅, and R₂₆ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 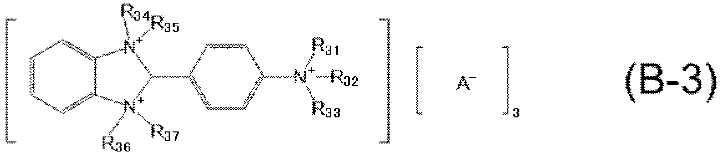 wherein R₃₁, R₃₂, R₃₃, R₃₄, R₃₅, R₃₆, and R₃₇ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 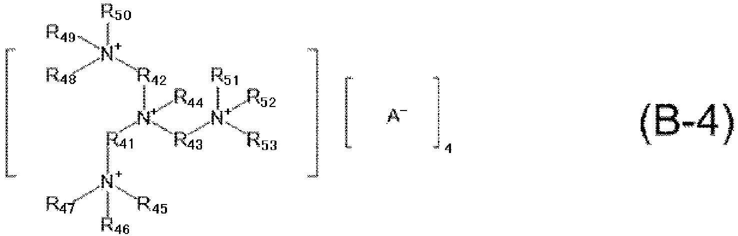 wherein R₄₁, R₄₂, and R₄₃ each independently represent a hydrocarbon group having 1 to 20 carbon atoms and optionally having a substituent or an aromatic group having 6 to 20 carbon atoms and optionally having a substituent; R₄₄, R₄₅, R₄₆, R₄₇, R₄₈, R₄₉, R₅₀, R₅₁, R₅₂, and R₅₃ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above.
[4]: A composition for forming a silicon-containing resist underlayer film, comprising: the polyvalent ammonium salt compound of any one of [1] to [3]; and a thermally crosslinkable polysiloxane.
[5]: The composition for forming a silicon-containing resist underlayer film of [4], wherein the thermally crosslinkable polysiloxane contains any one or more of a repeating unit represented by the following general formula (Sx-1), a repeating unit represented by the following general formula (Sx-2), and a partial structure represented by the following general formula (Sx-3), 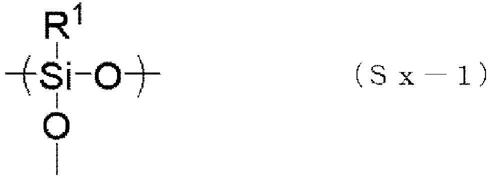 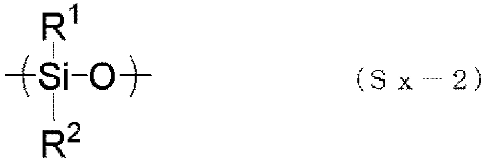 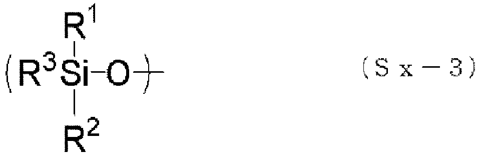 wherein R¹, R², and R³ are each identical to or different from each other and represent a monovalent organic group having 1 to 30 carbon atoms.
[6]: The composition for forming a silicon-containing resist underlayer film of [4] or [5], further comprising an acid generator.
[7]: The composition for forming a silicon-containing resist underlayer film of [6], wherein the acid generator is a photo-acid generator, which generates an acid by an action of a high-energy beam.
[8]: A patterning process for forming a pattern in a body to be processed, comprising the steps of:
   forming an organic film on a body to be processed by using a coating-type organic film material;
   forming a silicon-containing resist underlayer film on the organic film by using the composition for forming a silicon-containing resist underlayer film of any one of [4] to [7];
   forming a resist upper layer film on the silicon-containing resist underlayer film by using a resist upper layer film composition comprising a photoresist composition;
   forming a circuit pattern in the resist upper layer film;
   transferring the pattern to the silicon-containing resist underlayer film by etching while using the resist upper layer film having the formed circuit pattern as a mask;
   transferring the pattern to the organic film by etching while using the silicon-containing resist underlayer film having the transferred pattern as a mask; and
   transferring the pattern to the body to be processed by etching while using the organic film having the transferred pattern as a mask.
[9]: A patterning process for forming a pattern in a body to be processed, comprising the steps of:
   forming a hard mask on a body to be processed by a CVD method;
   forming a silicon-containing resist underlayer film on the hard mask by using the composition for forming a silicon-containing resist underlayer film of any one of [4] to [7];
   forming a resist upper layer film on the silicon-containing resist underlayer film by using a resist upper layer film composition comprising a photoresist composition;
   forming a circuit pattern in the resist upper layer film;
   transferring the pattern to the silicon-containing resist underlayer film by etching while using the resist upper layer film having the formed circuit pattern as a mask;
   transferring the pattern to the hard mask by dry etching while using the silicon-containing resist underlayer film having the transferred pattern as a mask; and
   transferring the pattern to the body to be processed by dry etching while using the hard mask having the transferred pattern as a mask.
[10]: The patterning process of [8] or [9], wherein the circuit pattern is formed in the resist upper layer film by a photolithography with a wavelength of 10 nm or more and 300 nm or less, a direct drawing by electron beam, a nanoimprinting, or a combination thereof.
[11]: The patterning process of any one of [8] to [10], wherein the body to be processed is a semiconductor device substrate or the semiconductor device substrate coated with any of a metal film, a metal carbide film, a metal oxide film, a metal nitride film, a metal oxycarbide film, and a metal oxynitride film.
[12]: The patterning process of [11], wherein the metal is silicon, gallium, titanium, tungsten, hafnium, zirconium, chromium, germanium, copper, silver, gold, indium, arsenic, palladium, tantalum, iridium, aluminum, iron, molybdenum, cobalt, or an alloy thereof.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A polyvalent ammonium salt compound having two or more ammonium ions per molecule, the polyvalent ammonium salt compound being represented by the following general formula (A-1), 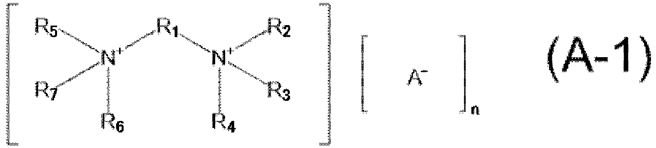 wherein R₁ represents a hydrocarbon group having 1 to 20 carbon atoms and optionally having a substituent or an aromatic group having 6 to 20 carbon atoms and optionally having a substituent, and optionally contains one or more nitrogen atoms; R₂, R₃, R₄, R₅, R₆, and R₇ each independently represent a hydrogen atom, a hydrocarbon group having 1 to 20 carbon atoms and optionally having a substituent, or an aromatic group having 6 to 20 carbon atoms and optionally having a substituent, and optionally contain one or more nitrogen atoms; any two or more selected from R₁, R₂, R₃, R₄, R₅, R₆, and R₇ are optionally bonded to each other to form a ring together with the nitrogen atom in the formula; A⁻ represents an organic or inorganic anion to be a counter ion of an ammonium cation, and excludes bistrifluoromethanesulfonylimide; and "n" represents an integer of 2 to 10.

2. The polyvalent ammonium salt compound according to claim 1, wherein the A⁻ is formate, acetate, propionate, butyrate, hexanoate, benzoate, t-butylbenzoate, trichloroacetate, trifluoroacetate, 2-hydroxy-2,2-bis(trifluoromethyl)acetate, trimethylacetate, pentafluoropropionate, methanesulfonate, butanesulfonate, benzenesulfonate, toluenesulfonate, trifluoromethanesulfonate, methyl sulfate ion, chloride ion, bromide ion, iodide ion, fluoride ion, cyanide ion, nitrate ion, nitrite ion, or hydroxide ion.

3. The polyvalent ammonium salt compound according to claim 1 or 2, represented by the following general formula (B-1), the following general formula (B-2), the following general formula (B-3), or the following general formula (B-4), 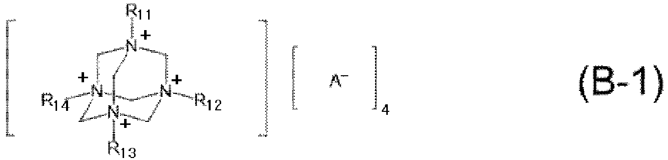 wherein R₁₁, R₁₂, R₁₃, and R₁₄ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 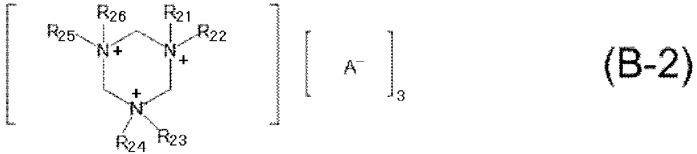 wherein R₂₁, R₂₂, R₂₃, R₂₄, R₂₅, and R₂₆ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 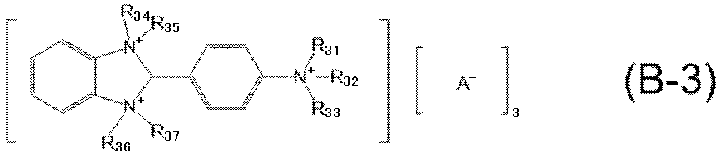 wherein R₃₁, R₃₂, R₃₃, R₃₄, R₃₅, R₃₆, and R₃₇ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above, 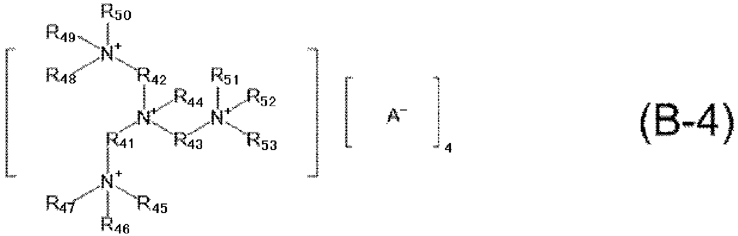 wherein R₄₁, R₄₂, and R₄₃ each independently represent a hydrocarbon group having 1 to 20 carbon atoms and optionally having a substituent or an aromatic group having 6 to 20 carbon atoms and optionally having a substituent; R₄₄, R₄₅, R₄₆, R₄₇, R₄₈, R₄₉, R₅₀, R₅₁, R₅₂, and R₅₃ each independently represent a hydrogen atom, a hydrocarbon group having 1 to 20 carbon atoms and optionally having a substituent, or an aromatic group having 6 to 20 carbon atoms and optionally having a substituent; and A⁻ is as defined above.

4. A composition for forming a silicon-containing resist underlayer film, comprising: the polyvalent ammonium salt compound according to any one of claims 1 to 3; and a thermally crosslinkable polysiloxane.

5. The composition for forming a silicon-containing resist underlayer film according to claim 4, wherein the thermally crosslinkable polysiloxane contains any one or more of a repeating unit represented by the following general formula (Sx-1), a repeating unit represented by the following general formula (Sx-2), and a partial structure represented by the following general formula (Sx-3), 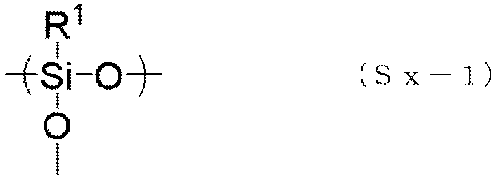 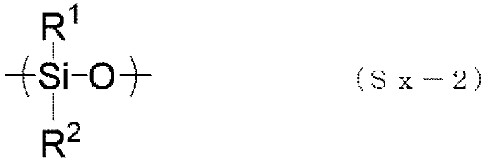 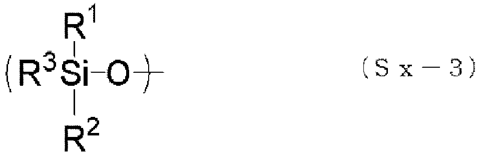 wherein R¹, R², and R³ are each identical to or different from each other and represent a monovalent organic group having 1 to 30 carbon atoms.

6. The composition for forming a silicon-containing resist underlayer film according to claim 4 or 5, further comprising an acid generator.

7. The composition for forming a silicon-containing resist underlayer film according to claim 6, wherein the acid generator is a photo-acid generator, which generates an acid by an action of a high-energy beam.

8. A patterning process for forming a pattern in a body to be processed, comprising the steps of:
forming an organic film on a body to be processed by using a coating-type organic film material;
forming a silicon-containing resist underlayer film on the organic film by using the composition for forming a silicon-containing resist underlayer film according to any one of claims 4 to 7;
forming a resist upper layer film on the silicon-containing resist underlayer film by using a resist upper layer film composition comprising a photoresist composition;
forming a circuit pattern in the resist upper layer film;
transferring the pattern to the silicon-containing resist underlayer film by etching while using the resist upper layer film having the formed circuit pattern as a mask;
transferring the pattern to the organic film by etching while using the silicon-containing resist underlayer film having the transferred pattern as a mask; and
transferring the pattern to the body to be processed by etching while using the organic film having the transferred pattern as a mask.

9. A patterning process for forming a pattern in a body to be processed, comprising the steps of:
forming a hard mask on a body to be processed by a CVD method;
forming a silicon-containing resist underlayer film on the hard mask by using the composition for forming a silicon-containing resist underlayer film according to any one of claims 4 to 7;
forming a resist upper layer film on the silicon-containing resist underlayer film by using a resist upper layer film composition comprising a photoresist composition;
forming a circuit pattern in the resist upper layer film;
transferring the pattern to the silicon-containing resist underlayer film by etching while using the resist upper layer film having the formed circuit pattern as a mask;
transferring the pattern to the hard mask by dry etching while using the silicon-containing resist underlayer film having the transferred pattern as a mask; and
transferring the pattern to the body to be processed by dry etching while using the hard mask having the transferred pattern as a mask.

10. The patterning process according to claim 8 or 9, wherein the circuit pattern is formed in the resist upper layer film by a photolithography with a wavelength of 10 nm or more and 300 nm or less, a direct drawing by electron beam, a nanoimprinting, or a combination thereof.

11. The patterning process according to any one of claims 8 to 10, wherein the body to be processed is a semiconductor device substrate or the semiconductor device substrate coated with any of a metal film, a metal carbide film, a metal oxide film, a metal nitride film, a metal oxycarbide film, and a metal oxynitride film.

12. The patterning process according to claim 11, wherein the metal is silicon, gallium, titanium, tungsten, hafnium, zirconium, chromium, germanium, copper, silver, gold, indium, arsenic, palladium, tantalum, iridium, aluminum, iron, molybdenum, cobalt, or an alloy thereof.
